(19) Europäisches Patentamt
European Patent Office
Office européen des brevets

(11) **EP 3 543 354 B1**

(12) **EUROPEAN PATENT SPECIFICATION**

(45) Date of publication and mention
of the grant of the patent:
**19.01.2022 Bulletin 2022/03**

(21) Application number: **19172284.2**

(22) Date of filing: **17.06.2014**

(51) International Patent Classification (IPC):
*C12Q 1/68* (2018.01)    *C12Q 1/6827* (2018.01)
*G16B 30/00* (2019.01)    *G16B 30/20* (2019.01)
*G16B 20/10* (2019.01)    *G16B 20/20* (2019.01)
*G16B 30/10* (2019.01)

(52) Cooperative Patent Classification (CPC):
(C-Sets available)
**C12Q 1/6827; G16B 20/10; G16B 20/20;
G16B 30/10; G16B 30/20**                (Cont.)

(54) **METHOD FOR GENERATING A MASKED REFERENCE SEQUENCE OF THE Y CHROMOSOME**

VERFAHREN ZUR BESTIMMUNG DER KOPIENZAHLVARIATIONEN BEI
GESCHLECHTSCHROMOSOMEN

PROCÉDÉ POUR DÉTERMINER LES VARIATIONS DU NOMBRE DE COPIES DANS DES
CHROMOSOMES SEXUELS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.06.2013 US 201361836057 P**

(43) Date of publication of application:
**25.09.2019 Bulletin 2019/39**

(62) Document number(s) of the earlier application(s) in
accordance with Art. 76 EPC:
**14739612.1 / 3 011 052**

(73) Proprietor: **Verinata Health, Inc.
San Diego, California 92122 (US)**

(72) Inventor: **ABDUEVA, Diana
Orinda, CA California 94563 (US)**

(74) Representative: **Cooley (UK) LLP
22 Bishopsgate
London EC2N 4BQ (GB)**

(56) References cited:
WO-A1-2012/108920    GB-A- 2 485 635
US-A1- 2013 029 852    US-A1- 2013 103 320

• A. MAGI ET AL: "Read count approach for DNA
copy number variants detection",
BIOINFORMATICS, vol. 28, no. 4, 23 December
2011 (2011-12-23), pages 470-478, XP055160553,
ISSN: 1367-4803, DOI:
10.1093/bioinformatics/btr707
• HOCHSTENBACH R ET AL: "Array analysis and
karyotyping: Workflow consequences based on
a retrospective study of 36,325 patients with
idiopathic developmental delay in the
Netherlands", EUROPEAN JOURNAL OF
MEDICAL GENETICS, ELSEVIER, NL, vol. 52, no.
4, 1 July 2009 (2009-07-01), pages 161-169,
XP026349254, ISSN: 1769-7212 [retrieved on
2009-04-09]

(52) Cooperative Patent Classification (CPC): (Cont.)

C-Sets
**C12Q 1/6827, C12Q 2537/16, C12Q 2537/165**

**Description**

**FIELD OF THE INVENTION**

The present invention relates to computer-implemented methods for generating a masked reference sequence of the Y chromosome for evaluating copy number of the Y chromosome. The present invention also relates to computer-implemented methods for evaluation of copy number of the Y chromosome in a test sample. Furthermore, the invention relates to systems for the evaluation of copy number of a genetic sequence of interest in a test sample comprising fetal and maternal cell-free nucleic acids obtained from a pregnant female. In addition, the invention relates to computer program products able to cause the implementation of the computer-implemented methods of the invention.

**BACKGROUND**

One of the critical endeavors in human medical research is the discovery of genetic abnormalities that produce adverse health consequences. In many cases, specific genes and/or critical diagnostic markers have been identified in portions of the genome that are present at abnormal copy numbers. For example, in prenatal diagnosis, extra or missing copies of whole chromosomes are frequently occurring genetic lesions. In cancer, deletion or multiplication of copies of whole chromosomes or chromosomal segments, and higher level amplifications of specific regions of the genome, are common occurrences.

Most information about copy number variation (CNV) has been provided by cytogenetic resolution that has permitted recognition of structural abnormalities. Conventional procedures for genetic screening and biological dosimetry have utilized invasive procedures, e.g., amniocentesis, cordocentesis, or chorionic villus sampling (CVS), to obtain cells for the analysis of karyotypes. Recognizing the need for more rapid testing methods that do not require cell culture, fluorescence *in situ* hybridization (FISH), quantitative fluorescence PCR (QF-PCR) and array-Comparative Genomic Hybridization (array-CGH) have been developed as molecular-cytogenetic methods for the analysis of copy number variations.

The advent of technologies that allow for sequencing entire genomes in relatively short time, and the discovery of circulating cell-free DNA (cfDNA) have provided the opportunity to compare genetic material originating from one chromosome to be compared to that of another without the risks associated with invasive sampling methods, which provides a tool to diagnose various kinds of copy number variations of genetic sequences of interest.

Diagnosis of copy number variations of the Y chromosome involves heightened technical challenges compared to autosomes, because coverage of the Y chromosome is lower than that of autosomes, and repeated sequences on the Y chromosome complicate mapping of reads to their correct location. There are about 10 Mb of unique Y sequences accessible by current NGS technologies, but gender detection remains to be a challenging task in fetal diagnostic world where the amount of fetal cfDNA in a maternal sample is at least an order of magnitude lower than that of maternal DNA, emphasizing the problem of nonspecific mapping. Additionally, some current sequencing protocols utilize ultra-short reads such as 25mer reads and tags, presenting yet another alignment challenge since 25mer tags are shorter than typical size of most ubiquitous repeatable elements. Some embodiments disclosed herein describe a strategy for filtering out (or masking) non-discriminant sequence reads on chromosome Y using representative training set of female samples. In some embodiments, this filtering strategy is also applicable to filtering autosomes for evaluation of copy number variation of sequences on the autosomes.

Limitations of existing methods in noninvasive prenatal diagnostics, which include insufficient sensitivity stemming from the limited levels of cfDNA, and the sequencing bias of the technology stemming from the inherent nature of genomic information, underlie the continuing need for noninvasive methods that would provide any or all of the specificity, sensitivity, and applicability, to reliably diagnose copy number changes in a variety of clinical settings. Embodiments disclosed herein fulfill some of the above needs and in particular offers an advantage in providing a reliable method that is applicable to the practice of noninvasive prenatal diagnostics.

US2013/103320 relates to methods, processes, and apparatuses for non-invasive assessment of genetic variations.

Magi *et al*. discloses the comparison of algorithms designed to detect the boundaries of CNVs and an investigation of the ability of read count data to predict DNA copy number (Magi et al., Read count approach for DNA copy number variants detection, Bioinformatics, 2012 Feb 15;28(4):470-8).

**SUMMARY**

In an aspect of the invention, there is provided a method for generating a masked reference sequence of the Y chromosome for evaluating copy number of the Y chromosome, according to claim 1.

In another aspect of the invention, there is provided a method for evaluation of copy number of the Y chromosome in a test sample, according to claim 2.

In a further aspect of the invention, there is provided a system for evaluation of copy number of a genetic sequence of interest in a test sample comprising fetal and maternal cell-free nucleic acids obtained from a pregnant female, according to claim 13.

In another aspect of the invention, there is provided a computer program product according to claim 14.

In some embodiments, methods are provided for determining copy number of the Y chromosome, including, but not limited to, methods for gender determination or Y chromosome aneuploidy of fetus using maternal samples comprising maternal and fetal cell free DNA.

In one embodiment, the method comprises: (a) providing, on the computer system, a training set comprising genomic reads measured from nucleic acid samples of a first plurality of female individuals; (b) aligning, by the computer system, at least about 100,000 genomic reads per individual of the training set to a reference sequence of the Y-chromosome, thereby providing training sequence tags comprising aligned genomic reads and their locations on the reference sequence of the Y chromosome; (c) dividing, by the computer system, the reference sequence of the Y chromosome into a plurality of bins; (d) determining, by the computer system, counts of training sequence tags located in each bin; (e) masking, by the computer system, bins that exceed a masking threshold, the masking threshold being based on the counts of training sequence tags in each bin, thereby providing a masked reference sequence of the Y chromosome for evaluation of copy number of the Y chromosome in a test sample. In some embodiments, the test sample comprises fetal and maternal cell-free nucleic acids.

In some embodiments, the method for evaluation of copy number of the Y chromosome in a test sample further comprises: (f) sequencing the cell free nucleic acids from the test sample comprising fetal and maternal cell-free nucleic acids using a sequencer, thereby generating genomic reads of the test sample; and (g) aligning, by the computer system, the genomic reads of the test sample to the reference sequence, thereby providing testing sequence tags comprising aligned genomic reads and locations thereof.

In some embodiments, the method for evaluation of copy number of the Y chromosome in a test sample further comprises: (h) measuring, by the computer system, counts of the testing sequence tags on the masked reference sequence of the Y chromosome; and (i) evaluating, by the computer system, copy number of the Y chromosome in the test sample based on the counts of the testing sequence tags on the masked reference sequence of the Y chromosome.

In any one of the embodiments described above, the test sample may be a maternal sample selected from blood, plasma, serum, urine and saliva samples. In any one of the embodiments, the test sample is may be plasma sample. The nucleic acid molecules of the maternal sample are a mixture of fetal and maternal cell-free DNA molecules. Sequencing of the nucleic acids can be performed using next generation sequencing (NGS). In some embodiments, sequencing is massively parallel sequencing using sequencing-by-synthesis with reversible dye terminators. In other embodiments, sequencing is sequencing-by-ligation. In yet other embodiments, sequencing is single molecule sequencing. Optionally, an amplification step is performed prior to sequencing.

In certain embodiments embodied on a computer system, the number of sequence tags identified for each of the one or more chromosomes of interest or chromosome segments of interest is at least about 10,000, or at least about 100,000. The disclosed embodiments also provide a computer program product including a non-transitory computer readable medium on which is provided program instructions for performing the recited operations and other computational operations described herein.

In some embodiments, a method additionally includes sequencing at least a portion of said nucleic acid molecules of said maternal test sample to obtain said sequence information for said fetal and maternal nucleic acid molecules of said test sample. The sequencing may involve massively parallel sequencing on maternal and fetal nucleic acids from the maternal test sample to produce the sequence reads.

In some embodiments, the masking threshold is determined by operations performed by or on the computer system: providing two or more masking threshold candidates; masking bins that exceed the masking threshold candidates, thereby providing two or more masked reference sequences; calculating a threshold evaluation index for evaluation of copy number of the genetic sequence of interest based on each of the two or more masked reference sequences; and selecting the candidate having the highest threshold evaluation index as the masking threshold.

In some embodiments, calculating the threshold evaluation index includes evaluating copy number of the Y chromosome for nucleic acid samples of (a) female individuals different from the female individuals of the training set and (b) male individuals known to have a Y chromosome. In some embodiments, the threshold evaluation index is calculated as the difference between the means of (a) and (b), divided by the standard deviation

In some embodiments, the size of each bin is determined by operations of a computer system: dividing the reference sequence of the Y chromosome into bins of a candidate bin size; calculating a bin evaluation index based on the candidate bin size; iteratively repeating the preceding steps of this embodiment on the computer system using different candidate bin sizes, thereby yielding two or more different evaluation indices; and electing the candidate bin size yielding the highest bin evaluation index as the size of the bins.

In some embodiments, female individuals of a training set have diverse alignment profiles characterized by

different distributions of the genomic reads on the reference sequence of the Y chromosome. In some embodiments, providing a training set involves dividing a second plurality of female individuals into two or more clusters and selecting a number of individuals in each of the two or more clusters to form the first plurality of female individuals as members of the training set. In some embodiments, an equal number of individuals are selected in each of the two or more clusters. In some embodiments, the dividing the plurality of female individuals into two or more clusters involves hierarchical ordered partitioning and collapsing hybrid (HOPACH) clustering.

In some embodiments, a method further includes automatically recording, using a processor, the presence or absence of a fetal chromosomal aneuploidy as determined as described above in a patient medical record for a human subject providing the maternal test sample. The recording may include recording chromosome doses and/or a diagnosis based said chromosome doses in a computer-readable medium. In some cases, the patient medical record is maintained by a laboratory, physician's office, a hospital, a health maintenance organization, an insurance company, or a personal medical record website. A method may further include prescribing, initiating, and/or altering treatment of a human subject from whom the maternal test sample was taken. Additionally or alternatively, the method may include ordering and/or performing one or more additional tests.

In some embodiments, system and computer program products are provided to perform the methods of the invention.

Although the examples herein concern humans and the language is primarily directed to human concerns, the concepts described herein are applicable to genomes from any plant or animal.

## BRIEF DESCRIPTION OF THE DRAWINGS

Figure 1 shows sequence classes, genes, and palindromes on the human Y chromosome. (a) Schematic representation of the entire human Y chromosome, with the male-specific region (MSY) indicated. (b) A more detailed representation that focuses on the euchromatic MSY and excludes the major heterochromatic block on Yq.

Figure 2 shows an example of regions that are masked on the Y chromosome in one embodiment. The masked Y chromosome can be used as a reference sequence for evaluation of copy number of the Y chromosome.

Figure 3A-3B show block diagrams of embodiments of a method for evaluation of copy number of the Y chromosome in a test sample comprising fetal and maternal cell-free nucleic acids. The method is implemented at a computer system that includes one or more processors and system memory.

Figure 4 is a flowchart of a method 100 for determining the presence or absence of a copy number variation in a test sample comprising a mixture of nucleic acids.

Figure 5 is a block diagram of a dispersed system for processing a test sample and ultimately making a diagnosis.

Figure 6 schematically illustrates how different operations in processing test samples may be grouped to be handled by different elements of a system.

Figures 7A and 7B shows electropherograms of a cfDNA sequencing library prepared according to the abbreviated protocol described in Example 1a (Fig. 7A), and the protocol described in Example 1b (Fig. 7B).

Figure 8 illustrates a heatmap of pairwise chrY 1kb coverage correlations across 475 females, sorted by using HOPACH results.

Figure 9 shows the ChrY ratio (*i.e.* chrY count/chr4 count) in 1 Mb vs. 1kb bin sizes for female (2) and male (3).

Figure 10 shows Male/Female discrimination signal to noise ratio as a function of fraction of bins masked.

Figure 11 shows the frequency distribution of sequence tags mapped to the Y chromosome for samples including female (light gray) vs. male (dark gray) fetal cfDNAs. The left panel shows the distribution of sequence tags mapped to an unmasked Y chromosome. The right panel shows the distribution mapped to a masked Y chromosome according to methods described herein.

Figures 12A and 12B illustrate the distribution of the chromosome dose for chromosome 21 determined from sequencing cfDNA extracted from a set of 48 blood samples obtained from human subjects each pregnant with a male or a female fetus. Chromosome 21 doses for qualified, *i.e.,* normal for chromosome 21 (O), and trisomy 21 test samples are shown (Δ) for chromosomes 1-12 and X (Figure 12A), and for chromosomes 1-22 and X (Figure 12B).

Figures 13A and 13B illustrate the distribution of the chromosome dose for chromosome 18 determined from sequencing cfDNA extracted from a set of 48 blood samples obtained from human subjects each pregnant with a male or a female fetus. Chromosome 18 doses for qualified, *i.e.,* normal for chromosome 18 (O), and trisomy 18 (Δ) test samples are shown for chromosomes 1-12 and X (Figure 13A), and for chromosomes 1-22 and X (Figure 13B).

Figures 14A and 14B illustrate the distribution of the chromosome dose for chromosome 13 determined from sequencing cfDNA extracted from a set of 48 blood samples obtained from human subjects each pregnant with a male or a female fetus. Chromosome 13 doses for qualified, *i.e.,* normal for chromosome 13 (O), and trisomy 13 (Δ) test samples are shown for chromosomes 1-12 and X (Figure 14A), and for chromosomes 1-22 and X (Figure 14B).

Figures 15A and 15B illustrate the distribution of the chromosome doses for chromosome X determined from sequencing cfDNA extracted from a set of 48 test blood samples obtained from human subjects each pregnant with a male or a female fetus. Chromosome X doses for males (46,XY; (O)), females (46,XX; (Δ)); monosomy X (45,X; (+)), and complex karyotypes (Cplx (X)) samples are shown for chromosomes 1-12 and X (Figure 15A), and for chromosomes 1-22 and X (Figure 15B).

Figures 16A and 16B illustrate the distribution of the chromosome doses for chromosome Y determined from sequencing cfDNA extracted from a set of 48 test blood samples obtained from human subjects each pregnant with a male or a female fetus. Chromosome Y doses for males (46,XY; (Δ)), females (46,XX; (O)); monosomy X (45,X; (+)), and complex karyotypes (Cplx (X)) samples are shown for chromosomes 1-12 (Figure 16A), and for chromosomes 1-22 (Figure 16B).

Figure 17 shows the coefficient of variation (CV) for chromosomes 21 (■), 18 (●) and 13 (▲) that was determined from the doses shown in Figures 12A and 12B, 13A and 13B, and 14A and 14B, respectively.

Figure 18 shows the coefficient of variation (CV) for chromosomes X (a) and Y (●) that was determined from the doses shown in Figures 15A and 15B and 16A and 16B, respectively.

Figures 19A-19E illustrate the distribution of normalized chromosome doses for chromosome 21 (19A), chromosome 18 (19B), chromosome 13 (19C), chromosome X (19D) and chromosome Y (1919E) relative to the standard deviation of the mean (Y-axis) for the corresponding chromosomes in the unaffected samples.

Figures 20A and 20B show two flow diagrams of design and sampling plans for the study described in Example 7. Figure 20A shows a flow diagram of the design plan and Figure 20B shows a random sampling plan.

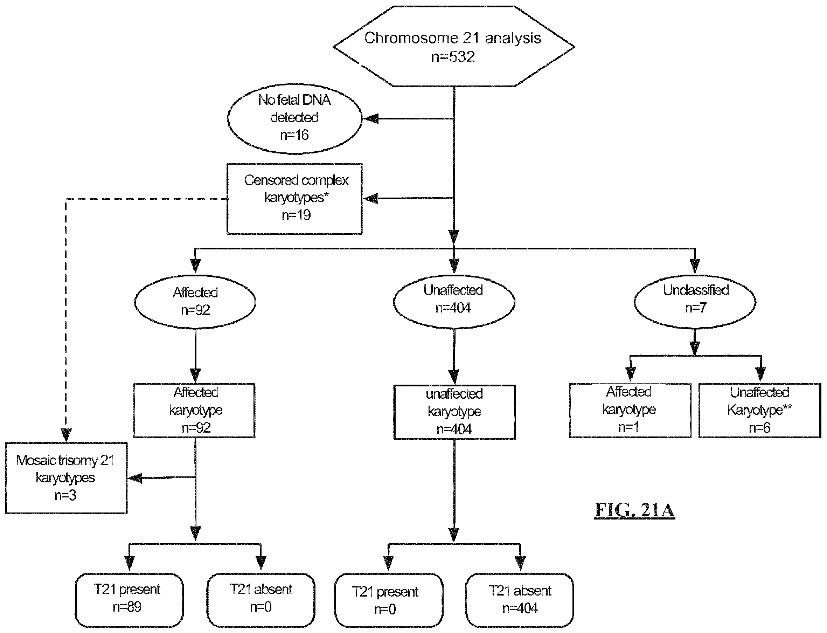

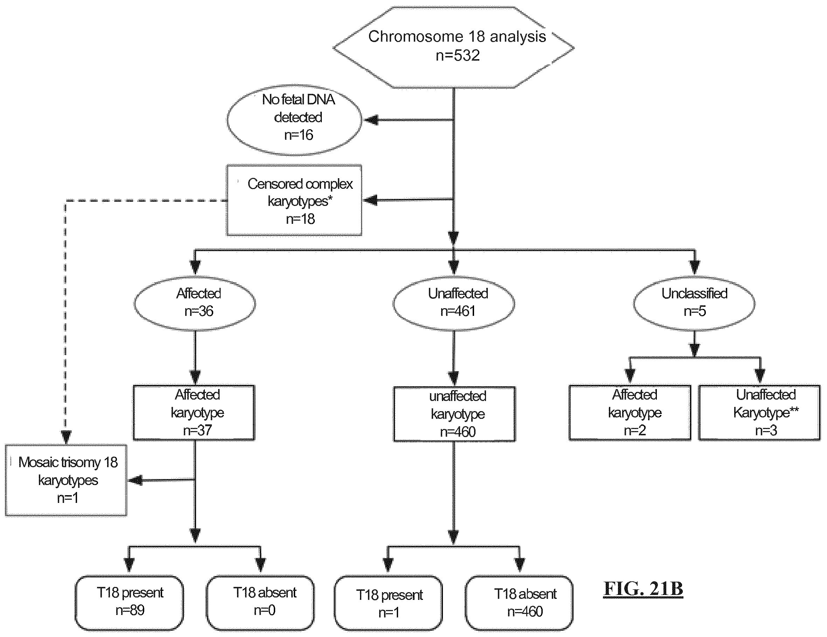

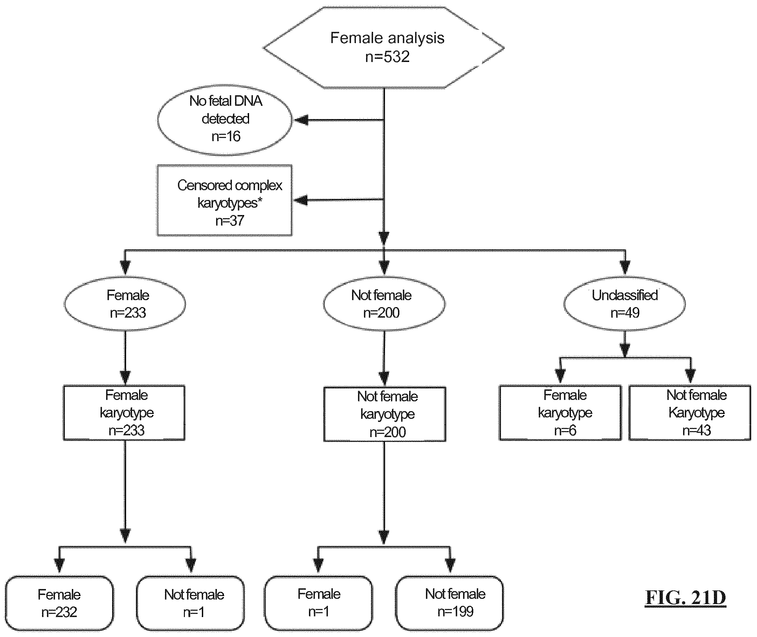

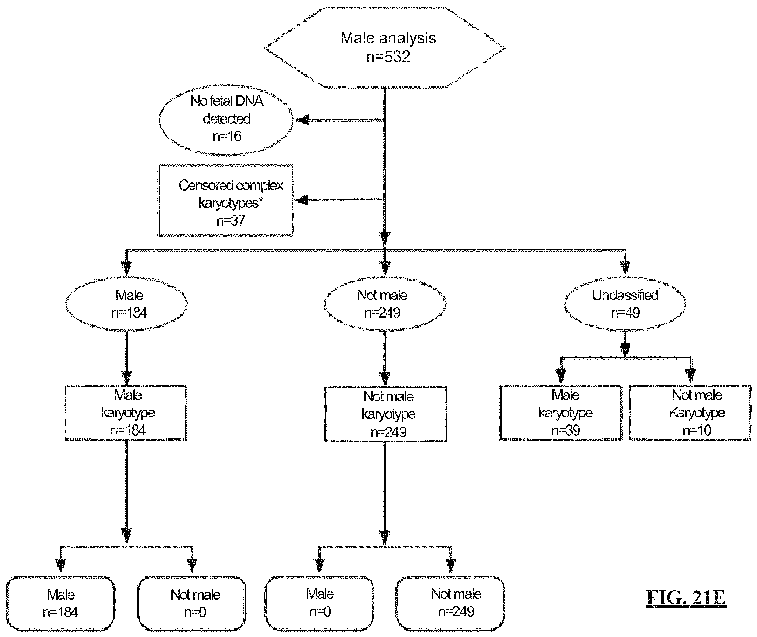

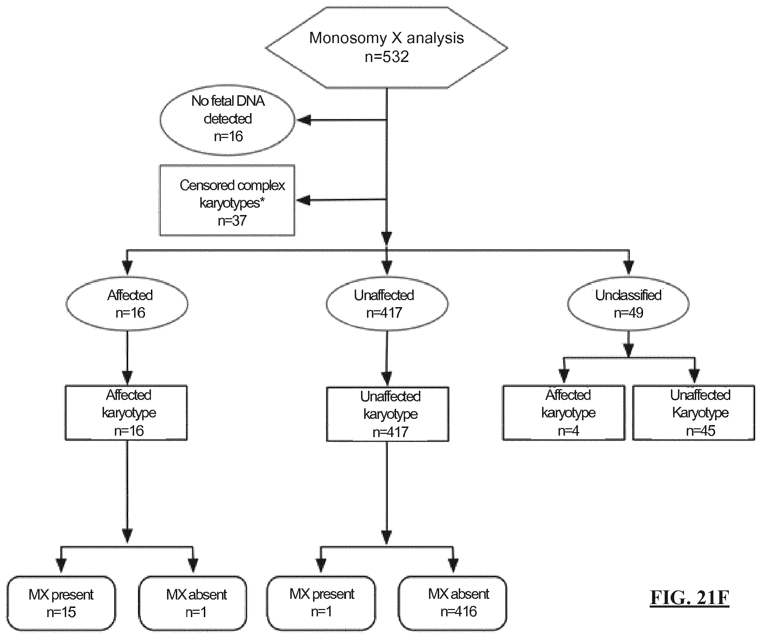

Figures 21A-21F show flow diagrams for the analyses for chromosomes 21, 18, and 13 (Figures 21A-2121C, respectively), and gender analyses for female, male, and monosomy X (Figures 21D-21F, respectively). Ovals contain results obtained from sequencing information from the laboratory, rectangles contain karyotype results, and rectangles with rounded corners show comparative results used to determine test performance (sensitivity and specificity). The dashed lines in Figure 21A and 21B denote the relationship between mosaic samples for T21 (n=3) and T18 (n=1) that were censored from the analysis of chromosome 21 and 18, respectively, but were correctly determined as described in Example 7.

Figure 22 shows normalized chromosome values (NCV) versus karyotype classifications for chromosomes 21 (●), 18 (■), and 13 (▲) for the test samples of the study described in Example 7. Circled samples denote unclassified samples with trisomy karyotype.

Figure 23 shows normalized chromosome values for chromosome X (NCV) versus karyotype classifications for gender classifications of the test samples of the study described in Example 7. Samples with female karyotypes (○), samples with male karyotypes (●), samples with 45,X (□), and samples with other karyotypes, *i.e.,* XXX, XXY, and XYY (■) are shown.

Figure 24 shows a plot of normalized chromosome values for chromosome Y versus normalized chromosome values for chromosome X for the test samples of the clinical study described in Example 7. Euploid male and female samples (○), XXX samples (●), 45,X samples (X), XYY samples (■), and XXY samples (▲) are shown. The dashed lines show the threshold values used for classifying samples as described in Example 7.

## DETAILED DESCRIPTION

The disclosed embodiments concern methods, apparatus, and systems for evaluation of copy number of the Y chromosome in a test sample comprising fetal and maternal cell-free nucleic acids. In some embodiments, sequences of interest include genomic segment sequences ranging from, *e.g.*, kilobases (kb) to megabases (Mb) to entire chromosomes that are known or are suspected to be associated with a genetic or a disease condition. In some embodiments, copy number of the Y chromosome is used to determine fetal gender. In some embodiments, CNV that can be determined according to the present method include monosomies and trisomies of sex chromosome Y (*e.g.* 47,XXY and 47,XYY), other polysomies of sex chromosomes such as tetrasomy and pentasomies (e.g. XXXXY and XYYYY), and deletions and/or duplications of segments of any one or more of the sex chromosomes. Also provided for reference are other examples of sequences of interest, which include chromosomes associated with well-known aneuploidies, e.g., trisomy XXX, trisomy 21, and segments of chromosomes that are multiplied in diseases such as cancer, e.g., partial trisomy 8 in acute myeloid leukemia.

Unless otherwise indicated, the practice of the method and system disclosed herein involves conventional techniques and apparatus commonly used in molecular biology, microbiology, protein purification, protein engineering, protein and DNA sequencing, and recombinant DNA fields, which are within the skill of the art. Such techniques and apparatus are known to those of skill in the art and are described in numerous texts and reference works (See *e.g.,* Sambrook et al., "Molecular Cloning: A Laboratory Manual," Third Edition (Cold Spring Harbor), [2001]); and Ausubel et al., "Current Protocols in Molecular Biology" [1987]).

Numeric ranges are inclusive of the numbers defining the range. It is intended that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The headings provided herein are not intended to limit the disclosure. Unless defined otherwise herein, all technical and scientific terms used herein have the same meaning as commonly understood by one of ordinary skill in the art. Various scientific dictionaries that include the terms included herein are well known and available to those in the art. Although any methods and materials similar or equivalent to those described herein find use in the practice or testing of the embodiments disclosed herein, some methods and materials are described.

The terms defined immediately below are more fully described by reference to the Specification as a whole. It is to be understood that this disclosure is not limited to the particular methodology, protocols, and reagents described, as these may vary, depending upon the context they are used by those of skill in the art.

### Definitions

As used herein, the singular terms "a," "an," and "the" include the plural reference unless the context clearly indicates otherwise.

Unless otherwise indicated, nucleic acids are written left to right in 5' to 3' orientation and amino acid sequences are written left to right in amino to carboxy orientation, respectively.

The term "assessing" when used herein in the context of analyzing a nucleic acid sample for CNV refers to characterizing the status of a chromosomal or segment aneuploidy by one of three types of calls: "normal" or "unaffected," "affected," and "no-call." Thresholds for calling normal and affected are typically set. A parameter related to aneuploidy or other copy number variation is measured in a sample and the measured value is compared to the thresholds. For duplication type aneuploidies, a call of affected is made if a chromosome or segment dose (or other measured value sequence content) is above a defined threshold set for affected samples. For such aneuploidies, a call of normal is made if the chromosome or segment dose is below a threshold set for normal samples. By contrast for deletion type aneuploidies, a call of affected is made if a chromosome or segment dose is below a defined threshold for affected samples, and a call of normal is made if the chromosome or segment dose is above a threshold set for normal samples. For example, in the presence of trisomy the "normal" call is determined by the value of a parameter, *e.g.*, a test chromosome dose that is below a user-defined threshold of reliability, and the "affected" call is determined by a parameter, *e.g.*, a test chromosome dose, that is above a user-defined threshold of reliability. A "no-call" result is determined by a parameter, *e.g.*, a test chromosome dose, that lies between the thresholds for making a "normal" or an "affected" call. The term "no-call" is used interchangeably with "unclassified".

The term "copy number variation" herein refers to variation in the number of copies of a nucleic acid sequence present in a test sample in comparison with the copy number of the nucleic acid sequence present in a reference sample. In certain embodiments, the nucleic acid sequence is 1 kb or larger. In some cases, the nucleic acid sequence is a whole chromosome or significant portion thereof. A "copy number variant" refers to the sequence of nucleic acid in which copy-number differences are found by comparison of a sequence of interest in test sample with an expected level of the sequence of interest. For example, the level of the sequence of interest in the test sample is compared to that present in a qualified sample. Copy number variants/variations include deletions, including microdeletions, insertions, including microinsertions, duplications, multiplications, inversions, translocations and complex multi-site variants. CNVs encompass chromosomal aneuploidies and partial aneuploidies.

The term "aneuploidy" herein refers to an imbalance of genetic material caused by a loss or gain of a whole chromosome, or part of a chromosome.

The terms "chromosomal aneuploidy" and "complete chromosomal aneuploidy" herein refer to an imbalance of genetic material caused by a loss or gain of a whole chromosome, and includes germline aneuploidy and mosaic aneuploidy.

The terms "partial aneuploidy" and "partial chromosomal aneuploidy" herein refer to an imbalance of genetic material caused by a loss or gain of part of a chromosome, *e.g.*, partial monosomy and partial trisomy, and encompasses imbalances resulting from translocations, deletions and insertions.

The term "plurality" refers to more than one element. For example, the term is used herein in reference to a number of nucleic acid molecules or sequence tags that is sufficient to identify significant differences in copy number variations in test samples and qualified samples using the methods disclosed herein. In some embodiments, at least about $3 \times 10^6$ sequence tags of between about 20 and 40bp are obtained for each test sample. In some embodiments, each test sample provides data for at least about $5 \times 10^6$, $8 \times 10^6$, $10 \times 10^6$, $15 \times 10^6$, $20 \times 10^6$, $30 \times 10^6$, $40 \times 10^6$, or $50 \times 10^6$ sequence tags, each sequence tag comprising between about 20 and 40bp.

The terms "polynucleotide," "nucleic acid" and "nucleic acid molecules" are used interchangeably and refer to a covalently linked sequence of nucleotides (*i.e.,* ribonucleotides for RNA and deoxyribonucleotides for DNA) in which the 3' position of the pentose of one nucleotide is joined by a phosphodiester group to the 5' position of the pentose of the next. The nucleotides include sequences of any form of nucleic acid, including, but not limited to RNA and DNA molecules such as cfDNA molecules. The term "polynucleotide" includes, without limitation, single- and double-stranded polynucleotide.

The term "portion" is used herein in reference to the amount of sequence information of fetal and maternal nucleic acid molecules in a biological sample that in sum amount to less than the sequence information of 1 human genome.

The term "test sample" herein refers to a sample, typically derived from a biological fluid, cell, tissue, organ, or organism, comprising a nucleic acid or a mixture of nucleic acids comprising at least one nucleic acid sequence that is to be screened for copy number variation. In certain embodiments the sample comprises at least one nucleic acid sequence whose copy number is suspected of having undergone variation. Such samples include, but are not limited to sputum/oral fluid, amniotic fluid, blood, a blood fraction, or fine needle biopsy samples (*e.g.*, surgical biopsy, fine needle biopsy, *etc.*), urine, peritoneal fluid, pleural fluid, and the like. Although the sample is often taken from a human subject (*e.g.*, patient), the assays can be used to copy number variations (CNVs) in samples from any mammal, including, but not limited to dogs, cats, horses, goats, sheep, cattle, pigs, *etc.* The sample may be used directly as obtained from the biological source or following a pretreatment to modify the character of the sample. For example, such pretreatment may include preparing plasma from blood, diluting viscous fluids and so forth. Methods of pretreatment may also involve, but are not limited to, filtration, precipitation, dilution, distillation, mixing, centrifugation, freezing, lyophilization, concentration, amplification, nucleic acid fragmentation, inactivation of interfering components, the addition of reagents, lysing, *etc.* If such methods of pretreatment are employed with respect to the sample, such pretreatment methods are typically such that the nucleic acid(s) of interest remain in the test sample, sometimes at a concentration proportional to that in an untreated test sample (*e.g.*, namely, a sample that is not subjected to any such pretreatment method(s)). Such "treated" or "processed" samples are still considered to be biological "test" samples with respect to the methods described herein.

The term "qualified sample" herein refers to a sample comprising a mixture of nucleic acids that are present in a known copy number to which the nucleic acids in a test sample are to be compared, and it is a sample that is normal, *i.e.,* not aneuploid, for the sequence of interest. In certain embodiments, qualified samples are used for identifying one or more normalizing chromosomes or segments for a chromosome under consideration. For example, qualified samples may be used for identifying a normalizing chromosome for chromosome 21. In such case, the qualified sample is a sample that is not a trisomy 21 sample. Qualified samples may also be employed in determining thresholds for calling affected samples.

The term "training set" herein refers to a set of samples that can comprise affected and/or unaffected samples and are used to develop a model for analyzing test samples. In some embodiments, the training set includes unaffected samples. In these embodiments, thresholds for determining CNV are established using training sets of samples that are unaffected for the copy number variation of interest. The unaffected samples in a training set may be used as the qualified samples to identify normalizing sequences, *e.g.*, normalizing chromosomes, and the chromosome doses of unaffected samples are used to set the thresholds for each of the sequences, *e.g.*, chromosomes, of interest. In some embodiments, the training set includes affected samples. The affected samples in a training set can be used to verify that affected test samples can be easily differentiated from unaffected samples.

"Training set" is also used herein in reference to a set of individuals of a statistical sample of a population of interest, data of which individuals are used to determine one or more quantitative values of interest generalizable to the population. The statistical sample is a subset of individuals in the population of interest. The individuals may be persons, animals, tissues, cells, other biological samples (i.e., a statistical sample may include multiple biological samples), and other individual entities providing data points for statistical analysis.

Usually, a training set is used in conjunction with a validation set. The term "validation set" is used here in reference to a set of individuals in a statistical sample, data of which individuals are used to validate or evaluate the quantitative values of interest determined using a training set. In some embodiments, for instance, a training set provides data for calculating a mask for a reference sequence; a validation set provides data to validate or evaluate the mask.

"Evaluation of copy number" is used herein in reference to the statistical evaluation of the status of a genetic sequence related to the copy number of the sequence. For example, in some embodiments, the evaluation comprises the determination of the presence or absence of a genetic sequence. In some embodiments the evaluation comprises the determination of the partial or complete aneuploidy of a genetic sequence. In other embodiments the evaluation comprises discrimination between two or more samples based on the copy number of a genetic sequence. In some embodiments, the evaluation comprises statistical analyses, e.g., normalization and comparison, based on the copy number of the genetic sequence.

The term "qualified nucleic acid" is used interchangeably with "qualified sequence," which is a sequence against which the amount of a test sequence or test nucleic acid is compared. A qualified sequence is one present in a biological sample preferably at a known representation, *i.e.,* the amount of a qualified sequence is known. Generally, a qualified sequence is the sequence present in a "qualified sample." A "qualified sequence of interest" is a qualified sequence for which the amount is known in a qualified sample, and is a sequence that is associated with a difference in sequence representation in an individual with a medical condition.

The term "sequence of interest" herein refers to a nucleic acid sequence that is associated with a difference in sequence representation in healthy versus diseased individuals. A sequence of interest can be a sequence on a chromosome that is misrepresented, *i.e.,* over- or under-represented, in a disease or genetic condition. A sequence of interest may be a portion of a chromosome, *i.e.,* chromosome segment, or a chromosome. For example, a sequence of interest can be a chromosome that is over-represented in an aneuploidy condition, or a gene encoding a tumor-suppressor that is under-represented in a cancer. Sequences of interest include sequences that are over- or under- represented in the total population, or a subpopulation of cells of a subject. A "qualified sequence of interest" is a sequence of interest in a qualified sample. A "test sequence of interest" is a sequence of interest in a test sample.

The term "normalizing sequence" herein refers to a sequence that is used to normalize the number of sequence tags mapped to a sequence of interest associated with the normalizing sequence. In some embodiments, the normalizing sequence displays a variability in the number of sequence tags that are mapped to it among samples and sequencing runs that approximates the variability of the sequence of interest for which it is used as a normalizing parameter. The normalizing sequence can differentiate an affected sample from one or more unaffected samples. In some implementations, the normalizing sequence best or effectively differentiates, when compared to other potential normalizing sequences such as other chromosomes, an affected sample from one or more unaffected samples. A "normalizing chromosome" or "normalizing chromosome sequence" is an example of a "normalizing sequence." A "normalizing chromosome sequence" can be composed of a single chromosome or of a group of chromosomes. A "normalizing segment" is another example of a "normalizing sequence." A "normalizing segment sequence" can be composed of a single segment of a chromosome or it can be composed of two or more segments of the same or of different chromosomes. In certain embodiments, a normalizing sequence is intended to normalize for variability such as process-related, interchromosomal (intra-run), and inter-sequencing (inter-run) variability.

The term "differentiability" herein refers to a characteristic of a normalizing chromosome that enables one to distinguish one or more unaffected, *i.e.,* normal, samples from one or more affected, *i.e.,* aneuploid, samples. A normalizing chromosome displaying the greatest "differentiability" is a chromosome or group of chromosomes that provides the greatest statistical difference between the distribution of chromosome doses for a chromosome of interest in a set of qualified samples and the chromosome dose for the same chromosome of interest in the corresponding chromosome in the one or more affected samples.

The term "variability" herein refers to another characteristic of a normalizing chromosome that enables one to distinguish one or more unaffected, *i.e.,* normal, samples from one or more affected, *i.e.,* aneuploid, samples. The variability of a normalizing chromosome, which is measured in a set of qualified samples, refers to the variability in the number of sequence tags that are mapped to it that approximates the variability in the number of sequence tags that are mapped to a chromosome of interest for which it serves as a normalizing parameter.

The term "sequence dose" herein refers to a parameter that relates the number of sequence tags identified for a sequence of interest and the number of sequence tags identified for the normalizing sequence. In some cases, the sequence dose is the ratio of the number of sequence tags identified for a sequence of interest to the number of sequence tags identified for the normalizing sequence. In some cases, the sequence dose refers to a parameter that relates the sequence tag density of a sequence of interest to the tag density of a normalizing sequence. A "test sequence dose" is a parameter that relates the sequence tag density of a sequence of interest, e.g., chromosome 21, to that of a normalizing sequence, e.g., chromosome 9, determined in a test sample. Similarly, a "qualified sequence dose" is a parameter that relates the sequence tag density of a sequence of interest to that of a normalizing sequence determined in a qualified sample.

The term "sequence tag density" herein refers to the number of sequence reads that are mapped to a reference genome sequence, e.g., the sequence tag density for chromosome 21 is the number of sequence reads generated by the sequencing method that are mapped to chromosome 21 of the reference genome. The term "sequence tag density ratio" herein refers to the ratio of the number of sequence tags that are mapped to a chromosome of the reference genome, e.g., chromosome 21, to the length of the reference genome chromosome.

The term "Next Generation Sequencing (NGS)" herein refers to sequencing methods that allow for massively parallel sequencing of clonally amplified molecules and of single nucleic acid molecules. Non-limiting examples of NGS include sequencing-by-synthesis using reversible dye terminators, and sequencing-by-ligation.

The term "parameter" herein refers to a numerical value that characterizes a physical property. Frequently, a parameter numerically characterizes a quantitative data set and/or a numerical relationship between quantitative data sets. For example, a ratio (or function of a ratio) between the number of sequence tags mapped to a chromosome and the length of the chromosome to which the tags are mapped, is a parameter.

The terms "threshold value" and "qualified threshold value" herein refer to any number that is used as a cutoff to characterize a sample such as a test sample containing a nucleic acid from an organism suspected of having a medical condition. The threshold may be compared to a parameter value to determine whether a sample giving rise to such parameter value suggests that the organism has the medical condition. In certain embodiments, a qualified threshold value is calculated using a qualifying data set and serves as a limit of diagnosis of a copy number variation, e.g., an aneuploidy, in an organism. If a threshold is exceeded by results obtained from methods disclosed herein, a subject can be diagnosed with a copy number variation, e.g., trisomy 21. Appropriate threshold values for the methods described herein can be identified by analyzing normalizing values (*e.g.* chromosome doses, NCVs or NSVs) calculated for a training set of samples. Threshold values can be identified using qualified (*i.e.,* unaffected) samples in a training set which comprises both qualified (*i.e.,* unaffected) samples and affected samples. The samples in the training set known to have chromosomal aneuploidies (*i.e.,* the affected samples) can be used to confirm that the chosen thresholds are useful in differentiating affected from unaffected samples in a test set (see the Examples herein). The choice of a threshold is dependent on the level of confidence that the user wishes to have to make the classification. In some embodiments, the training set used to identify appropriate threshold values comprises at least 10, at least 20, at least 30, at least 40, at least 50, at least 60, at least 70, at least 80, at least 90, at least 100, at least 200, at least 300, at least 400, at least 500, at least 600, at least 700, at least 800, at least 900, at least 1000, at least 2000 , at least 3000 , at least 4000, or more qualified samples. It may advantageous to use larger sets of qualified samples to improve the diagnostic utility of the threshold values.

The term "masking threshold" is used herein to refer to a quantity against which a value based on the number of sequence tags in a sequence bin is compared, wherein a bin having a value exceeding the masking threshold is masked. In some embodiments, the masking threshold can be a percentile rank, an absolute count, or other suitable values. A masking threshold value is different from the threshold value as a cutoff to characterize a sample containing a nucleic acid from an organism suspected of having a medical condition mentioned above.

The term "normalizing value" herein refers to a numerical value that relates the number of sequence tags identified for the sequence (*e.g.* chromosome or chromosome segment) of interest to the number of sequence tags identified for the normalizing sequence (*e.g.* normalizing chromosome or normalizing chromosome segment). For example, a "normalizing value" can be a chromosome dose as described elsewhere herein, or it can be an NCV (Normalized Chromosome Value) as described elsewhere herein, or it can be an NSV (Normalized Segment Value) as described elsewhere herein.

The term "read" refers to a sequence read from a portion of a nucleic acid sample. Typically, though not necessarily, a read represents a short sequence of contiguous base pairs in the sample. The read may be represented symbolically by the base pair sequence (in ATCG) of the sample portion. It may be stored in a memory device and processed as appropriate to determine whether it matches a reference sequence or meets other criteria. A read may be obtained directly from a sequencing apparatus or indirectly from stored sequence information concerning the sample. In some cases, a read is a DNA sequence of sufficient length (*e.g.,* at least about 30 bp) that can be used to identify a larger sequence or region, *e.g.,* that can be aligned and specifically assigned to a chromosome or genomic region or gene. The term "genomic read" is used in reference to a read of any segments in the entire genome of an individual.

The term "sequence tag" is herein used interchangeably with the term "mapped sequence tag" to refer to a sequence read that has been specifically assigned, *i.e.,* mapped, to a larger sequence, *e.g.,* a reference genome, by alignment. Mapped sequence tags are uniquely mapped to a reference genome, *i.e.,* they are assigned to a single location to the reference genome. Unless otherwise specified, tags that map to the same sequence on a reference sequence are counted once. Tags may be provided as data structures or other assemblages of data. In certain embodiments, a tag contains a read sequence and associated information for that read such as the location of the sequence in the genome, *e.g.,* the position on a chromosome. In certain embodiments, the location is specified for a positive strand orientation. A tag may be defined to provide a limit amount of mismatch in aligning to a reference genome. In some embodiments, tags that can be mapped to more than one location on a reference genome, *i.e.,* tags that do not map uniquely, may not be included in the analysis.

As used herein, the terms "aligned," "alignment," or "aligning" refer to the process of comparing a read or tag to a reference sequence and thereby determining whether the reference sequence contains the read sequence. If the reference sequence contains the read, the read may be mapped to the reference sequence or, in certain embodiments, to a particular location in the reference sequence. In some cases, alignment simply tells whether or not a read is a member of a particular reference sequence (*i.e.,* whether the read is present or absent in the reference sequence). For example, the alignment of a read to the reference sequence for human chromosome 13 will tell whether the read is present in the reference sequence for chromosome 13. A tool that provides this information may be called a set membership tester. In some cases, an alignment additionally indicates a location in the reference sequence where the read or tag maps to. For example, if the reference sequence is the whole human genome sequence, an alignment may indicate that a read is present on chromosome 13, and may further indicate that the read is on a particular strand and/or site of chromosome 13.

Aligned reads or tags are one or more sequences that are identified as a match in terms of the order of their nucleic acid molecules to a known sequence from a reference genome. Alignment can be done manually, although it is typically implemented by a computer algorithm, as it would be impossible to align reads in a reasonable time period for implementing the methods disclosed herein. One example of an algorithm from aligning sequences is the Efficient Local Alignment of Nucleotide Data (ELAND) computer program distributed as part of the Illumina Genomics Analysis pipeline. Alternatively, a Bloom filter or similar set membership tester may be employed to align reads to reference genomes. See US Patent Application No. 61/552,374 filed October 27, 2011 which is publicly available as the priority document of WO2013/062856A1. The matching of a sequence read in aligning can be a 100% sequence match or less than 100% (non-perfect match). The term "alignment profile" is used in reference to the distribution of sequence tags aligned to locations which may be identified as base pair bins in a reference sequence of interest.

The term "mapping" used herein refers to specifically assigning a sequence read to a larger sequence, *e.g.,* a reference genome, by alignment.

As used herein, the term "reference genome" or "reference sequence" refers to any particular known genome sequence, whether partial or complete, of any organism or virus which may be used to reference identified sequences from a subject. For example, a reference genome used for human subjects as well as many other organisms is found at the National Center for Biotechnology Information at ncbi.nlm.nih.gov. A "genome" refers to the complete genetic information of an organism or virus, expressed in nucleic acid sequences.

In various embodiments, the reference sequence is significantly larger than the reads that are aligned to it. For example, it may be at least about 100 times larger, or at least about 1000 times larger, or at least about 10,000 times larger, or at least about $10^5$ times larger, or at least about $10^6$ times larger, or at least about $10^7$ times larger.

In one example, the reference sequence is that of a full length human genome. Such sequences may be referred to as genomic reference sequences. In another example, the reference sequence is limited to a specific human chromosome such as chromosome 13. In embodiments of the invention, the reference genome comprises a reference sequence of the Y-chromosome. In some embodiments, a reference Y chromosome is the Y chromosome sequence from human genome version hg19. Such sequences may be referred to as chromosome reference sequences. Other examples of reference sequences include genomes of other species, as well as chromosomes, sub-chromosomal regions (such as strands), *etc.,* of any species.

In various embodiments, the reference sequence is a consensus sequence or other combination derived from multiple individuals. However, in certain applications, the reference sequence may be taken from a particular individual.

The term "clinically-relevant sequence" herein refers to a nucleic acid sequence that is known or is suspected to be associated or implicated with a genetic or disease condition. Determining the absence or presence of a clinically-relevant sequence can be useful in determining a diagnosis or confirming a diagnosis of a medical condition, or providing a prognosis for the development of a disease.

The term "derived" when used in the context of a nucleic acid or a mixture of nucleic acids, herein refers to the means whereby the nucleic acid(s) are obtained from the source from which they originate. For example, in one embodiment, a mixture of nucleic acids that is derived from two different genomes means that the nucleic acids, *e.g.,* cfDNA, were naturally released by cells through naturally occurring processes such as necrosis or apoptosis. In another embodiment, a mixture of nucleic acids that is derived from two different genomes means that the nucleic acids were extracted from two different types of cells from a subject.

The term "based on" when used in the context of obtaining a specific quantitative value, herein refers to using another quantity as input to calculate the specific quantitative value as an output.

The term "patient sample" herein refers to a biological sample obtained from a patient, *i.e.,* a recipient of medical attention, care or treatment. The patient sample can be any of the samples described herein. In certain embodiments, the patient sample is obtained by non-invasive procedures, *e.g.,* peripheral blood sample or a stool sample. The methods described herein need not be limited to humans. Thus, various veterinary applications are contemplated in which case the patient sample may be a sample from a non-human mammal (*e.g.,* a feline, a porcine, an equine, a bovine, and the like).

The term "mixed sample" herein refers to a sample containing a mixture of nucleic acids, which are derived from different genomes.

The term "maternal sample" herein refers to a biological sample obtained from a pregnant subject, *e.g.,* a woman.

The term "biological fluid" herein refers to a liquid taken from a biological source and includes, for example, blood, serum, plasma, sputum, lavage fluid, cerebrospinal fluid, urine, semen, sweat, tears, saliva, and the like. As used herein, the terms "blood," "plasma" and "serum" expressly encompass fractions or processed portions thereof. Similarly, where a sample is taken from a biopsy, swab, smear, *etc.,* the "sample" expressly encompasses a processed fraction or portion derived from the biopsy, swab, smear, *etc.*

The terms "maternal nucleic acids" and "fetal nucleic acids" herein refer to the nucleic acids of a pregnant female subject and the nucleic acids of the fetus being carried by the pregnant female, respectively.

As used herein, the term "corresponding to" sometimes refers to a nucleic acid sequence, *e.g.,* a gene or a chromosome, that is present in the genome of different subjects, and which does not necessarily have the same sequence in all genomes, but serves to provide the identity rather than the genetic information of a sequence of interest, *e.g.,* a gene or chromosome.

As used herein, the term "substantially cell free" used in connection with a desired sample encompasses preparations of the desired sample from which cell components normally associated with the sample are removed. For example, a plasma sample is rendered substantially cell free by removing blood cells, *e.g.,* red cells, which are normally associated with it. In some embodiments, substantially cell free samples are processed to remove cells that would otherwise contribute to the desired genetic material that is to be tested for a CNV.

As used herein, the term "fetal fraction" refers to the fraction of fetal nucleic acids present in a sample comprising fetal and maternal nucleic acid. Fetal fraction is often used to characterize the cfDNA in a mother's blood.

As used herein the term "chromosome" refers to the heredity-bearing gene carrier of a living cell, which is derived from chromatin strands comprising DNA and protein components (especially histones). The conventional internationally recognized individual human genome chromosome numbering system is employed herein.

As used herein, the term "polynucleotide length" refers to the absolute number of nucleic acid molecules (nucleotides) in a sequence or in a region of a reference genome. The term "chromosome length" refers to the known length of the chromosome given in base pairs, e.g., provided in the NCBI36/hg18 assembly of the human chromosome found at genome.ucsc.edu/cgi-bin/hgTracks?hgsid=167155613&chromInfoPage= on the World Wide Web.

The term "subject" herein refers to a human subject as well as a non-human subject such as a mammal, an invertebrate, a vertebrate, a fungus, a yeast, a bacterium, and a virus. Although the examples herein concern humans and the language is primarily directed to human concerns, the concepts disclosed herein are applicable to genomes from any plant or animal, and are useful in the fields of veterinary medicine, animal sciences, research laboratories and such.

The term "condition" herein refers to "medical condition" as a broad term that includes all diseases and disorders, but can include [injuries] and normal health situations, such as pregnancy, that might affect a person's health, benefit from medical assistance, or have implications for medical treatments.

The term "complete" when used in reference to a chromosomal aneuploidy herein refers to a gain or loss of an entire chromosome.

The term "partial" when used in reference to a chromosomal aneuploidy herein refers to a gain or loss of a portion, *i.e.,* segment, of a chromosome.

The term "mosaic" herein refers to denote the presence of two populations of cells with different karyotypes in one individual who has developed from a single fertilized egg. Mosaicism may result from a mutation during development which is propagated to only a subset of the adult cells.

The term "non-mosaic" herein refers to an organism, *e.g.,* a human fetus, composed of cells of one karyotype.

The term "using a chromosome" when used in reference to determining a chromosome dose, herein refers to using the sequence information obtained for a chromosome, *i.e.,* the number of sequence tags obtained for a chromosome.

The term "sensitivity" as used herein is equal to the number of true positives divided by the sum of true positives

12

and false negatives.

The term "specificity" as used herein is equal to the number of true negatives divided by the sum of true negatives and false positives.

The term "enrich" herein refers to the process of amplifying polymorphic target nucleic acids contained in a portion of a maternal sample, and combining the amplified product with the remainder of the maternal sample from which the portion was removed. For example, the remainder of the maternal sample can be the original maternal sample.

The term "original maternal sample" herein refers to a non-enriched biological sample obtained from a pregnant subject, *e.g.,* a woman, who serves as the source from which a portion is removed to amplify polymorphic target nucleic acids. The "original sample" can be any sample obtained from a pregnant subject, and the processed fractions thereof, *e.g.,* a purified cfDNA sample extracted from a maternal plasma sample.

The term "primer," as used herein refers to an isolated oligonucleotide that is capable of acting as a point of initiation of synthesis when placed under conditions inductive to synthesis of an extension product (*e.g.,* the conditions include nucleotides, an inducing agent such as DNA polymerase, and a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer, use of the method, and the parameters used for primer design.

The phrase "cause to be administered" refers to the actions taken by a medical professional (*e.g.*, a physician), or a person controlling or directing medical care of a subject, that control and/or permit the administration of the agent(s)/compound(s) at issue to the subject. Causing to be administered can involve diagnosis and/or determination of an appropriate therapeutic or prophylactic regimen, and/or prescribing particular agent(s)/compounds for a subject. Such prescribing can include, for example, drafting a prescription form, annotating a medical record, and the like. Similarly, "cause to be performed," *e.g.,* for a diagnostic procedure refers to the actions taken by a medical professional (*e.g.*, a physician), or a person controlling or directing medical care of a subject, that control and/or permit the performance of one or more diagnostic protocols to or on the subject.

### Introduction

Methods, apparatus, and systems are disclosed herein for determining copy number and copy number variations (CNV) of different sequences of interest in a test sample that comprises a mixture of nucleic acids derived from two different genomes, and which are known or are suspected to differ in the amount of one or more sequence of interest. Copy number variations determined by the methods and apparatus disclosed herein include gains or losses of entire chromosomes, alterations involving very large chromosomal segments that are microscopically visible, and an abundance of sub-microscopic copy number variation of DNA segments ranging from single nucleotide, to kilobases (kb), to megabases (Mb) in size

Methods are disclosed herein that are applicable to determining CNV of any fetal aneuploidy, and CNVs known or suspected to be associated with a variety of medical conditions. In human subjects, CNV that can be determined according to the present method include trisomies and monosomies of any one or more of chromosomes 1-22, X and Y, other chromosomal polysomies, and deletions and/or duplications of segments of any one or more of the chromosomes, which can be detected by sequencing only once the nucleic acids of a test sample. Any aneuploidy can be determined from sequencing information that is obtained by sequencing only once the nucleic acids of a test sample. The methods of the claimed invention are applicable to evaluation of copy number of the Y chromosome.

CNV in the human genome significantly influence human diversity and predisposition to disease (Redon et al., Nature 23:444-454 [2006], Shaikh et al. Genome Res 19:1682-1690 [2009]). CNVs have been known to contribute to genetic disease through different mechanisms, resulting in either imbalance of gene dosage or gene disruption in most cases. In addition to their direct correlation with genetic disorders, CNVs are known to mediate phenotypic changes that can be deleterious. Recently, several studies have reported an increased burden of rare or de novo CNVs in complex disorders such as Autism, ADHD, and schizophrenia as compared to normal controls, highlighting the potential pathogenicity of rare or unique CNVs (Sebat *et al.*, 316:445 - 449 [2007]; Walsh et al., Science 320:539 - 543 [2008]). CNV arise from genomic rearrangements, primarily owing to deletion, duplication, insertion, and unbalanced translocation events.

The methods and apparatus described herein may employ next generation sequencing technology (NGS), which is massively parallel sequencing. In certain embodiments, clonally amplified DNA templates or single DNA molecules are sequenced in a massively parallel fashion within a flow cell (*e.g.* as described in Volkerding et al. Clin Chem 55:641-658 [2009]; Metzker M Nature Rev 11:31-46 [2010]). In addition to high-throughput sequence information, NGS provides quantitative information, in that each sequence read is a countable "sequence tag" representing an individual clonal DNA template or a single DNA molecule. The sequencing technologies of NGS include pyrosequencing, sequencing-by-synthesis with reversible dye terminators, sequencing by oligonucleotide probe ligation and ion semiconductor sequencing. DNA from individual samples can be sequenced individually (*i.e.,* singleplex sequencing) or DNA from multiple samples can be pooled and sequenced as indexed genomic molecules (*i.e.,* multiplex sequencing) on a single sequencing run, to generate up to several hundred million reads of DNA sequences. Examples of sequencing technologies that can be used to obtain the sequence information according to the present method are described herein after.

Various CNV analyses using DNA samples involve aligning or mapping sequence reads from a sequencer to a reference sequence. A reference sequence may be the sequence of whole genome, the sequence of a chromosome, the sequence of a sub chromosomal region, etc. Due to the characteristics of the reference sequence, diagnosis of CNV of the Y chromosome involves heightened technical challenges compared to autosomes, because coverage of the Y chromosome is lower than that of autosomes, and repeated sequences on the Y chromosome complicate mapping of reads to their correct location. There are about 10 Mb of unique Y sequence accessible by current NGS technologies, but gender detection remains to be a challenging task in fetal diagnostic world where the amount of fetal cfDNA in a maternal sample is at least an order of magnitude lower than that of maternal DNA, emphasizing the problem of nonspecific mapping.

Additionally, some current sequencing protocols utilize ultra-short reads such as 25mer reads and tags. Ultra-short sequencing utilized in processes of sequencing protocols generate short read lengths that presented technical challenges for sequence alignment since nearly half of the human genome is covered by repeats, many of which have been known about for decades. From a computational perspective, repeats create ambiguities in alignment, which, in turn, can produce biases and errors even at the whole chromosome counting level. A case-study of 15 most common chromosome Y (chrY) 25mers in samples from pregnant women with female fetuses showed that they all fall within 1 edit distance away from most abundant repetitive sequences in human genome. This illustrates a problem that is inherent in the process of aligning reads to a reference genome: the source DNA is virtually never identical to the reference and systematic alignment of reads to incorrect positions on chromosome Y inevitably leads to false gender inferences. The human genome has millions of copies of repeats in the range of 200-500bp, which is longer than the reads that are produced by NGS technology, especially currently utilized ultra-short read sequencing, hence a need for targeted post-filtering of unique and non-redundant reads on chromosome Y.

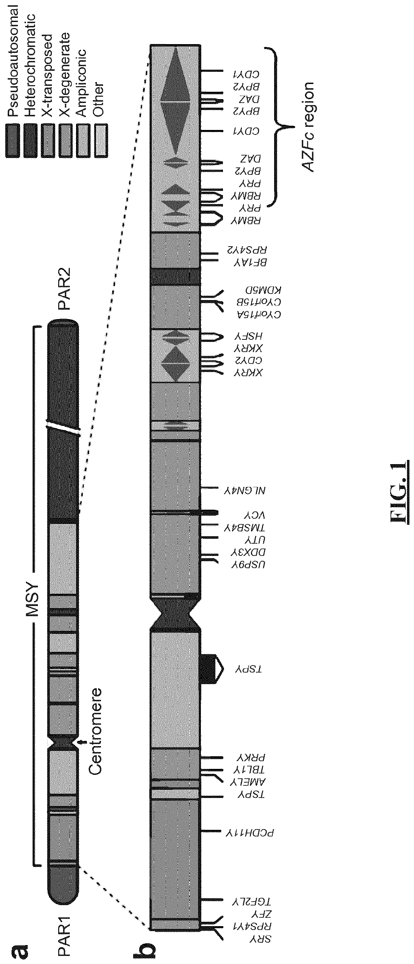

The human Y chromosome is heterogeneous, consisting heterochromatic, pseudoautosomal, X-transposed, X-degenerate, and ampliconic, see Figure 1. Specifically,

1. A significant fraction of the male-specific region of the Y chromosome comprises several discrete blocks of heterochromatic sequence, including a single ~40 Mb mass of heterochromatin on the long arm.
2. Pseudoautosomal regions (PAR) are located at the extreme termini of the Y and X chromosomes and constitute a small fraction of the total Y-chromosome sequence.
3. The X-transposed regions, which originated from an X-to-Y transposition event that span 3.4 Mb.
4. The X-degenerate sequences are a deteriorated version of the X chromosome. They are sparsely populated with 16 single-copy genes.
5. Ampliconic sequences are composed entirely of long stretches of duplicated sequence.

Accurately mapping reads to a reference sequence is one of the most critical tasks for next-generation sequencing, which remains to be one of the most challenging areas in commercial NGS system application, especially in gender calling that relies on accurate mapping of chromosome Y reads. Duke 25mer mapability track (available within UCSC's Genome Browser) reflects the uniqueness of all 25-base sequences and suggests that only 11Mb of chrY is completely unique. That said, limiting chrY mapped read count to unique sequences does not protect chrY total count from gender-indiscriminant hits that represent majority of male and all of the female coverage estate. Some conventional filtering methods address non-uniqueness of mapped reads: sequence read to sequence tag conversion involves removing all reads that map to multiple genomic positions; and tags to site conversion is a process of removing duplicated 25-mers mapping to the same genomic position. However, more efficient filtering methods are desirable to achieve better diagnostic results.

A study of many of the common chrY tags present in a cohort of de-identified commercial female samples suggests that the gender-indiscriminant tags represent sequencing errors occurring within highly duplicated genomic regions. For example, one specific 25mer gives 10,000+ hits across the genome and zero hits on chromosome Y, yet a similar 25mer with a single mismatch produces zero hits across the genome excluding Y and a single hit on chromosome Y. Hence, gender-indiscriminant tags represent a cohort of 25mers within short edit distances from 25mers with most frequent genomic duplications/repeats.

Embodiments of the invention describe a strategy for filtering out (or masking) non-discriminant sequence reads on chromosome Y using a representative training set of female samples. In some embodiments, this filtering strategy is also applicable to filtering autosomes for evaluation of copy number variation of sequences on the autosomes.

In some embodiments, the reference sequence contains masked or excluded regions that are not considered when determining how many reads are mapped to the reference sequence. Such regions may have sequences that are identical or nearly identical to sequences in other locations. Therefore any of such mapping could be problematic. A read mapped to the Y chromosome could actually originate at another location in the genome, e.g., in the X chromosome. In such cases, a false positive could occur. In some embodiments, the reads identically mapped to the reference sequence are excluded during read-to-tag conversion before sequence tags are counted to determine the mask. In such embodiments, reads nearly identically mapped to the Y chromosome still present the problem stated above. Some embodiments disclosed herein concern techniques for determining regions to be excluded or masked on the Y chromosome. In some embodiments, the techniques for masking a reference sequence are applicable to chromosomes other than the Y chromosome.

In some implementations, excluded regions on the reference sequence remain available for mapping. In such cases, reads are first aligned to excluded regions to yield sequence tags, but then sequence tags falling on the masked regions are not considered in subsequent calculation and classification. In alternative implementations, the excluded regions are simply removed from the reference sequence so that no read can map to an excluded region. However, this latter approach may lead to stray hits appearing elsewhere on the genome. For instance, some of a male fetus's reads from the Y chromosome of the fetus will be mapped to non-Y reference chromosomes. Such stray hits need to be addressed accordingly in this approach.

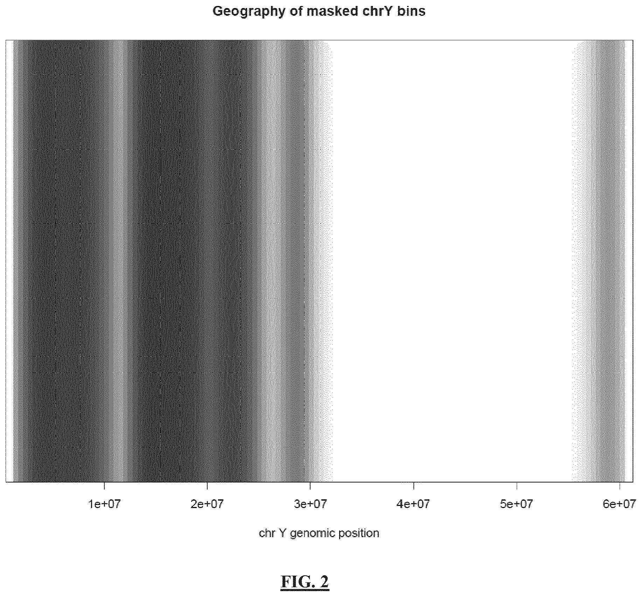

The empirical methods of filtering chromosome Y disclosed herein do not rely on a pre-defined/pre-calculated notion of gender non-discriminant regions. However, there is a fairly pronounced "masking" structure that is conserved between different versions of assays and reflects underlying repeat structure of chromosome Y. Figure 2 shows an example of segments of Y chromosome that are masked in one embodiment. The masked segments correspond to dark bands indexed by Y chromosome base pair numbers shown on the Y axis of the plot. In some embodiments, the masked Y chromosome can be pre-calculated and used as a reference sequence for evaluation of copy number of the Y chromosome. As can be seen, a majority of the mask bins fall below position 2 e7. In some embodiments, at least about 80% of the mask bins fall below position 3 e7. In some embodiments, at least about 90% of the mask bins fall below position 3 e7 and most or all of the remainder of the bins fall in region between positions 5.5 e7 and 6.2 e7.

**Masking Reference Sequence**

Embodiments of the invention employ a strategy for filtering out (or masking) non-discriminant sequence reads on chromosome Y using a representative training set of female samples. In some embodiments, the filtering strategy is also applicable to filtering autosomes for evaluation of copy number variation of sequences on the autosomes. In some embodiments, the reference Y chromosome is the Y chromosome sequence from human genome version hg19. Using the masked reference sequences generated by the methods described herein, one can reliably determine gender and/or determine various genetic conditions related to copy number and CNV with improved sensitivity, selectivity, and/or efficiency relative to conventional methods.

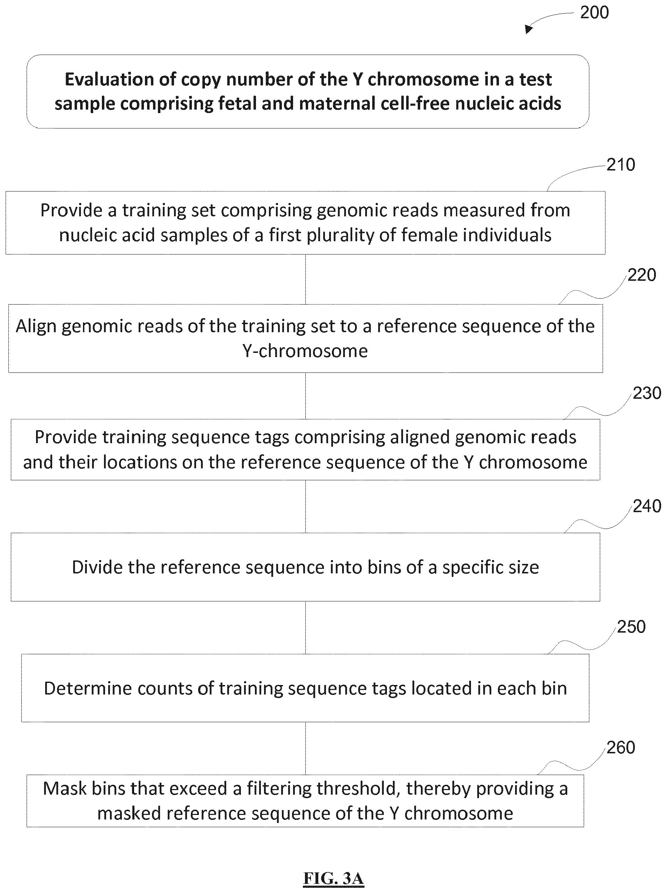

In some embodiments, a process is provided for chromosome Y filtering of uniquely mapped non-redundant reads (e.g., 25mers) based on their empirical frequency of occurrence in a representative cohort of clinical female samples. Figure 3A-3B show block diagrams of embodiments of a method for evaluation of copy number of the Y chromosome in a test sample comprising fetal and maternal cell-free nucleic acids. In some embodiments, the method is implemented at a computer system that includes one or more processors and system memory.

Figure 3A shows a block diagram of embodiments of the method of block 200. According to these embodiments, the method first provides a training set comprising genomic reads measured from nucleic acid samples of a first plurality of female individuals, block 210. In some embodiments described hereinafter, a training set selected by a method that maximizes the representativeness of the training set relative to the population to be tested. In some embodiments, the genomic reads comprise ultra-short sequences (e.g., 25 bp sequences). In some embodiments, the evaluation of copy number of the Y-chromosome is used to determine the gender of the fetus.

In some embodiments, the method further involves aligning genomic reads of the training set to a reference sequence of the Y-chromosome, block 220. Typically, genomic reads of sequences from the genome of the samples of the training set are aligned to a reference genome including the complete or nearly complete Y-chromosome. The alignment provides training sequence tags comprising aligned genomic reads and their locations on the reference sequence of the Y chromosome, see block 230.

Furthermore, the method involves dividing the reference sequence into bins of a specific size, see block 240. This division may be performed prior to aligning genomic reads. The method then determine the counts of training sequence tags located in each bin, see block 250. The method further involves masking bins that exceed a masking threshold, thereby providing a masked reference sequence of the Y chromosome, see block 260. In some embodiments, the method also involves determining the masking threshold. The masked reference sequence of the Y chromosome can be used to analyze copy number of the Y chromosome in test samples as described further below.

## Selecting a training set

Typically, a random sample set of female samples is used for training purposes for copy number evaluation of the Y chromosome. In an ideal scenario, a training set is a large set of genomic reads from females having similar Y chromosome alignment profiles as the test samples. So a goal of training set selection may be to make it as representative as possible, maintaining one or more of the following properties. (1) Training set is significantly smaller in size compared to the original dataset. (2) It captures the most of information from the original dataset compared to any subset of the same size. (3) It has low redundancy among the representatives it contains. (4) Adequate data must remain to substantiate validation results.

The female population has significant heterogeneity in "alignment profiles" for the Y chromosome. An alignment profile in this context is the distribution within the Y chromosome of sequence tags from female samples. Some female samples have reads that align to particular regions of the Y chromosome, while other female samples do not. An effective mask of the Y chromosome should be applicable across a wide range of female genotypes. To this end, the locations of the mask on the Y chromosome are selected by purposefully considering disparate alignment profiles identified from a number of female samples.

Some embodiments provide a method for selecting a training set to generate a mask for the Y chromosome that reduces the incidence of false positives (male gender identification) across many different types of female samples in the population. A female sample can be characterized by the distribution of reads from a sample mapping to a reference Y chromosome. Each female sample will have its own distribution, which can be referred to as an alignment profile in the Y chromosome.

To provide an effective masked reference sequence of the Y chromosome, female samples for a training set are selected to cover a wide range of alignment profiles represented in the population at large.

Various techniques can be employed for selecting samples to be used in the training set. One technique that can be used requires clustering of samples and selecting samples from each cluster. Other techniques may be applied to select a training set that is representative of the population to be tested, therefore providing adequate information to derive a useful mask of the reference sequence. Other methods for training set selection that may be implemented include, but are not limited to, intentional samples diversification with respect to vendors, reagents, instruments, operators and specific clinical sample parameters, e.g. cfDNA yield, etc.

In some embodiments, the training set selection technique divides female samples into clusters based upon similarities in alignment profile. The clustering technique is implemented to provide a reasonable number of clusters (e.g., about 10 to 30). In one embodiment, female DNA samples are separated into 20 clusters. Thereafter, a number of samples are selected from each cluster to populate the training set. In certain embodiments, the samples are randomly selected from each cluster.

In certain embodiments, the same number of samples is selected from each cluster (e.g., 15 samples are selected from each cluster). If a cluster has less than the required number of samples for selection, all members of the cluster are selected. In other embodiments, the number of members selected from each cluster is determined by the relative size of the clusters. For example, a cluster having a relatively large number of members would contribute a relatively large number of members to the training set. Conversely, a cluster having a relatively small number of samples would contribute a relatively small number of members to the training set. In some implementations, the contribution of each cluster is a fraction of its number of samples.

In some embodiments, clustering of training samples is performed by a hybrid clustering method, Hierarchical Ordered Partitioning and Collapsing Hybrid (HOPACH), which is a hierarchical tree of clusters. See, M. van der Laan and K. Pollard. A new algorithm for hybrid hierarchical clustering with visualization and the bootstrap. Journal of Statistical Planning and Inference, 117:275-303, 2003. HOPACH methodology combines the strengths of both partitioning and agglomerative clustering methods and allows a researcher to review clusters at increasing levels of detail. Further details of an embodiment are illustrated in example 2.

## Defining a mask for the Y chromosome

In some embodiments involving CNV analysis of the Y chromosome, the mask of the Y chromosome is comprised of a plurality of mask segments. Each segment comprises one or more bins, the segment having a length and a starting point. In some embodiments, the starting point may be defined as an offset from a defined location on the Y chromosome sequence. In the process of determining the mask segments, one may assume a particular bin size. In one example the length is 1 Mb and in another example the length is 1 kb. In principle, the bin size can extend down to the length of a single read, e.g., about 20 to 50 base pairs in length. In some embodiments, it is shown that methods using 1-kb bin size perform better than 1-Mb bin size.

In some embodiments, the size of the bins can be adjusted by a discrimination analysis or other technique. In some embodiments, an arbitrarily small bin size down to the size of a sequencer read would be appropriate. On the other hand, sequencing protocols and computational efficiencies may require a larger size. In some embodiments, bin size selection is driven by the most frequent size of the repeat seen in human genome. In some implementations, bins in the range of 500-1000 bp work well for initial binning that can later be coupled with bin merging to produce a final set of masking segments. Treangen TJ, Salzberg SL. Repetitive DNA and next-generation sequencing: computational challenges and solutions. Nat Rev Genet. 2011 Nov 29;13(1):36-46. doi: 10.1038/nrg3117. However, other technical restriction may possibly contribute to increase of bin size, e.g. an upper limit on total count of masking segments, etc.

In some embodiments, the sequence of each member of the training set is used to generate all possible reads. Each of those reads is checked for a match or alignment with a reference Y chromosome. In some embodiments, alignment allows up to two base mismatches in the read. In some embodiments, an alignment algorithm provides a match not only when a read exactly matches a portion of a reference chromosome, but also when a one or two base variation of the read matches a portion of the reference chromosome. The clustering of samples and calculation of sequence tags are not limited to alignment requiring exact match or allowing mismatches.

Each female sample in the training set is analyzed to produce the alignment profile of sequence tags based on how the reads from the female sample align to the reference Y chromosome. The reference Y chromosome is divided into bins of, typically, equal size. The alignment profile provides the number of sequence tags in each bin of the reference Y chromosome. Each of the bins of the reference Y chromosome is sorted by counts of reads for the members of the training set; *i.e.,* the most overrepresented bins are the top candidates for masking.

In some embodiments, all bins having at least one count are considered for masking. In some embodiments, the number of such bins that are actually removed, or more precisely the fraction of such bins actually removed, can be selected empirically. The topmost bin - the bin having the greatest number of counts from the training set - is the first bin to be removed. The bin with the second largest number of counts is the second to be be removed, and so on. Thus, even when the threshold fraction for masking is very low, typically the top-ranked bins will nevertheless be removed. If the threshold is set at 50%, one half of the bins will be masked. Those are the bins having count values at the 50th percentile and higher. In some embodiments, the masking threshold is set at 90th percentile or higher.

In the embodiment above, the threshold number of bins to be masked is determined empirically using a discrimination metric such as a male/female or aneuploidy discrimination metric. In some embodiments, the signal-to-noise ratio may be used as such metric as described above. Other discrimination metrics known in the art may also be employed.

### Determining copy number of the Y chromosome

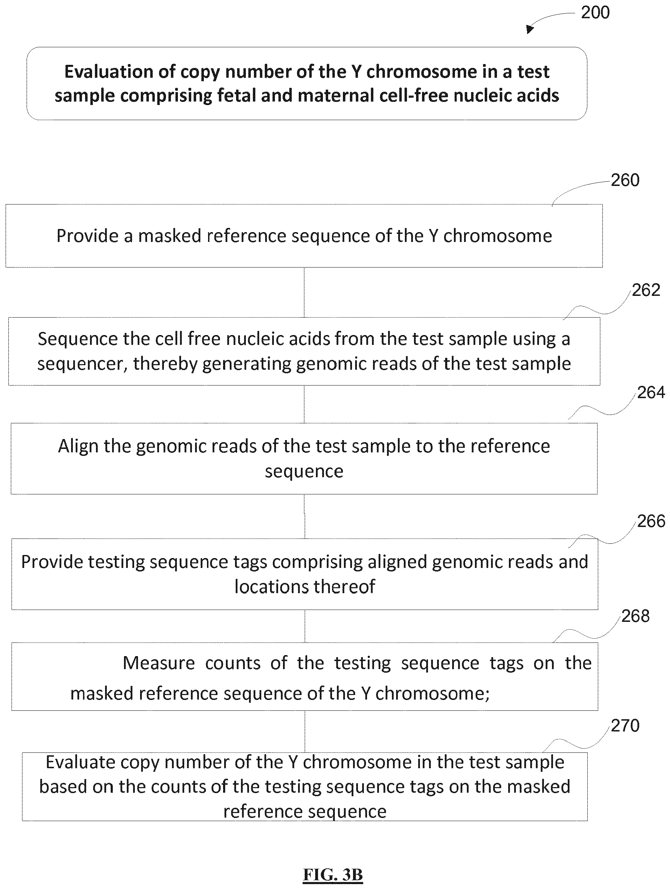

In some embodiments, chromosome Y filtering techniques described above are used to determine the copy number of the Y chromosome. Figure 3B shows a block diagram of embodiments of the method for evaluation of copy number of the Y chromosome, block 200. The method provides a masked reference sequence of the Y chromosome determined according to various embodiments described above, see block 260. The method further involves sequencing cell free nucleic acids from a test sample using a sequencer, thereby generating genomic reads of the test sample, block 262. The sample and sample processing methods are described with further details hereinafter. The samples may be sequenced by methods described hereinafter. The method further involves aligning the genomic reads of the test sample to a reference sequence 264, providing testing sequence tags comprising aligned genomic reads and locations on the reference sequence 266. Typically, the test sample reads are aligned to the unmasked reference sequence, although it is also possible to align the reads to the masked reference sequence. In some embodiments, aligning to unmasked reference sequence may yield better results. This may be especially true when the alignment allows for certain degree of mismatch.

In some embodiments, the method further involves measuring counts of the testing sequence tags on the masked reference sequence of the Y chromosome, block 268. The method can then evaluate copy number of the Y chromosome in the test sample based on the counts of the testing sequence tags on the masked reference sequence. See block 270.

### Masking chromosomes other than the Y chromosome

In some embodiments, chromosome Y filtering techniques described above may be extended to other chromosomes for evaluation of CNV or other purpose. In such embodiments, a filtering method first involves selecting a training set for whole genome filtering to represent distinct clusters of normal samples without known aberrant genetic condition or aneuploidy of interest. The training set is selected by, for instance, maximizing cluster representation as in the above-described approaches for chromosome Y. For validation, known affected samples with confirmed aneuploidies are used along with a set of normal samples not in the training set.

In some embodiments, the method involves determining the total count of non-duplicated sequence tags for every non-overlapping genomic bin of pre-defined size (not limited to, *e.g.,* chrY) across all samples in the training set. In some embodiments, the method involves standardization by subtracting from the bin sequence tag counts the expected count that can be approximated by median coverage across bins (the median calculated, *e.g.*, whole genome-wide, autosome-wide, or within-chromosome). Alternatively, mean or other values representative of the training set may be used instead of median.

The value of the deviation from the median/mean is then compared to a masking threshold. Bins that exceed the threshold are masked from the reference sequence. These bins contain relatively large fluctuation of sequence tag counts, which occurs within the non-aberrant training set. Therefore, the sequence tag counts in these bins tend to be noisy when used to derive a discrimination metric for discriminating unaffected vs. affected cohorts. By masking or filtering out these bins from the reference sequence, discrimination between the two cohorts is improved in some embodiments. In some embodiments, only the positive deviation from the median is considered for masking, removing bins that have over representation of sequence tags due to mis-alignment of reads from non-reference sequences.

Then in a SNR calculation, the method considers discrimination between affected validation cohort vs. independent un-affected cohort and finds an optimal masking threshold value via consensus across all chromosomes of interest (e.g., chromosome 13, 18, and/or 21), the optimal masking threshold value being the value that yields the highest SNR of a discrimination metric for differentiating the affected vs. unaffected cohorts.

Finally, the method provides a mask including bins having sequence tag counts exceeding the optimal masking threshold value. The mask is applied to a reference sequence that is used for evaluation of CNV.

In some embodiments, the process may be characterized by the following sequence of operations:

1. receive a training set of reads for each of a plurality of samples unaffected by a CNV in a genomic region of interest.
2. align the reads to a reference genome (or other large genomic reference sequence).
3. determine the number of tags in each of a plurality of equally sized bins in the reference genome.
4. standardize the tag counts in the bins of the samples by subtracting a median (or mean) tag count calculated across much or all of the reference sequence. Standardization may be conducted for each member of the training set. Standardizing is an optional step.
5. rank bins based on their standardized counts. Disregard bins having negative standardized counts. The bins with the larger values will be masked first.
6. evaluate different thresholds in the fraction of ranked bins to mask for the thresholds' ability to discriminate affected and unaffected samples. The mask may be defined for the chromosome or chromosomes of interest for testing (or for another region of the genome).
7. determine a threshold based on discrimination power and define a mask by including all high ranked bins above the threshold.

This strategy may target bins that are over-represented due to cross-talk with repetitive portions of the genome yielding stray hits that increase coverage compared to the baseline. In alternative embodiments, the absolute value of the standardized bins is used in the filtering strategy.

### Determination of CNV

#### *Methods for determination of CNV*

Using the masked reference sequences generated by the methods described above, one can determine various genetic conditions related to copy number and CNV of Y chromosome and other chromosomes with improved sensitivity, selectivity, and/or efficiency relative to conventional methods.

For example, in some embodiments, the masked reference sequences are used for determining the presence or absence of any two or more different complete fetal chromosomal aneuploidies in a maternal test sample comprising fetal and maternal nucleic acid molecules. Exemplary methods provided below align reads to reference sequences (including reference genomes). The alignment can be performed on an unmasked or masked reference sequence, thereby yielding sequence tags mapped to the reference sequence. In subsequent calculations, only sequence tags falling on unmasked segments of the reference sequence are taken into account to determine copy number variation.

In some embodiments, the method for determining the presence or absence of any two or more different complete fetal chromosomal aneuploidies in a maternal test sample comprises (a) obtaining sequence information for the fetal and maternal nucleic acids in the maternal test sample; (b) using the sequence information and the masked reference sequence obtained as described above to identify a number of sequence tags for each of the any two or more chromosomes of interest selected from chromosomes 1-22, X and Y and to identify a number of sequence tags for a normalizing chromosome sequence for each of the any two or more chromosomes of interest; (c) using the number of sequence tags identified for each of the any two or more chromosomes of interest and the number of sequence tags identified for each normalizing chromosome to calculate a single chromosome dose for each of the any two or more chromosomes of interest; and (d) comparing each of the single chromosome doses for each of the any two or more chromosomes of interest to a threshold value for each of the two or more chromosomes of interest, and thereby determining the presence or absence of any two or more complete different fetal chromosomal aneuploidies in the maternal test sample.

In some embodiments, step (a) described above can comprise sequencing at least a portion of the nucleic acid molecules of a test sample to obtain said sequence information for the fetal and maternal nucleic acid molecules of the test sample. In some embodiments, step (c) comprises calculating a single chromosome dose for each of the chromosomes of interest as the ratio of the number of sequence tags identified for each of the chromosomes of interest and the number of sequence tags identified for the normalizing chromosome sequence for each of the chromosomes of interest. In some other embodiments, chromosome dose is based on sequence tag density ratio, instead of number of sequence tags. A sequence tag density ratio is the number of sequence tag standardized by sequence length. In such embodiments, the chromosome dose is calculated as the ratio of the sequence tag density ratio for each of the chromosomes of interest and the sequence tag density ratio for the normalizing chromosome sequence for each of the chromosomes of interest.

In any one of the embodiments above, the different complete chromosomal aneuploidies are selected from complete chromosomal trisomies, complete chromosomal monosomies and complete chromosomal polysomies. The different complete chromosomal aneuploidies are selected from complete aneuploidies of any one of chromosome 1-22, X, and Y. For example, the said different complete fetal chromosomal aneuploidies are selected from trisomy 2, trisomy 8, trisomy 9, trisomy 20, trisomy 21, trisomy 13, trisomy 16, trisomy 18, trisomy 22, , 47,XXX, 47,XYY, and monosomy X.

In any one of the embodiments above, steps (a)-(d) are repeated for test samples from different maternal subjects, and the method comprises determining the presence or absence of any two or more different complete fetal chromosomal aneuploidies in each of the test samples.

In any one of the embodiments above, the method can further comprise calculating a normalized chromosome value (NCV), wherein the NCV relates the chromosome dose to the mean of the corresponding chromosome dose in a set of qualified samples as:

$$NCV_{ij} = \frac{x_{ij} - \hat{\mu}_j}{\hat{\sigma}_j}$$

where $\hat{u}_j$ and $\hat{\sigma}_j$ are the estimated mean and standard deviation, respectively, for the $j$-th chromosome dose in a set of qualified samples, and $x_{ij}$ is the observed $j$-th chromosome dose for test sample i.

In another embodiment, a method is provided for determining the presence or absence of different partial fetal chromosomal aneuploidies in a maternal test sample comprising fetal and maternal nucleic acids. The method involves procedures analogous to the method for detecting complete aneuploidy as outlined above. However, instead of analyzing a complete chromosome, a segment of a chromosome is analyzed. See U.S. Patent Application Publication No. 20130029852.

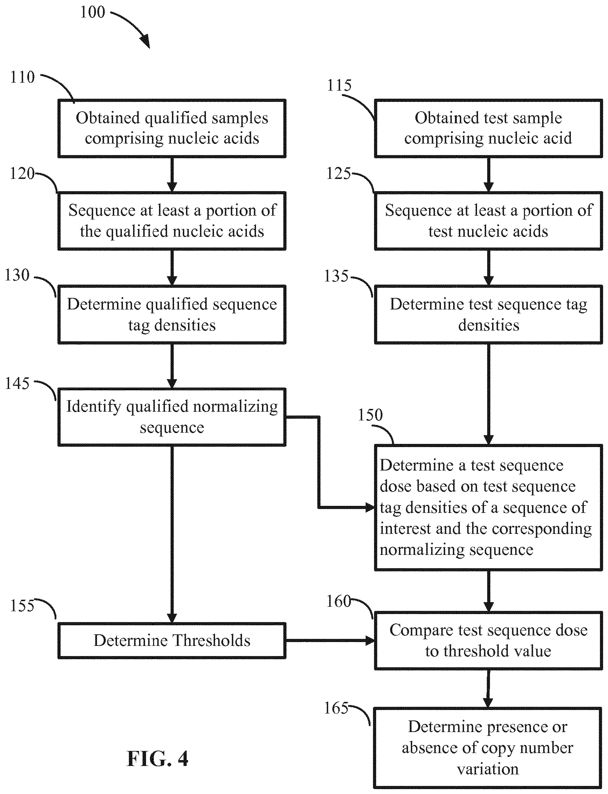

Figure 4 shows a method for determining the presence of copy number variation in accordance with some embodiments. From an over-view perspective, the method makes use of normalizing sequences of qualified samples in determination of CNV of test samples. Normalizing sequences provide a mechanism to normalize measurements for intra-run and inter-run variabilities. Normalizing sequences are identified using sequence information from a set of qualified samples obtained from subjects known to comprise cells having a normal copy number for any one sequence of interest, *e.g.,* a chromosome or segment thereof. Determination of normalizing sequences is outlined in steps **110, 120, 130, 140,** and **145** of the embodiment of the method depicted in Figure 4. In some embodiments, the normalizing sequences are used to calculate sequence dose for test sequences. See step **150.** In some embodiments, normalizing sequences are also used to calculate a threshold against which the sequence dose of the test sequences is compared. See step **150.** The sequence information obtained from the normalizing sequence and the test sequence is used for determining statistically meaningful identification of chromosomal aneuploidies in test samples (step **165**)

Turning to the details of the method for determining the presence of copy number variation according to some embodiments, Figure 4 provides a flow diagram 100 of an embodiment for determining a CNV of a sequence of interest, *e.g.,* a chromosome or segment thereof, in a biological sample. In some embodiments, a biological sample is obtained from a subject and comprises a mixture of nucleic acids contributed by different genomes. The different genomes can be contributed to the sample by two individuals, *e.g.,* the different genomes are contributed by the fetus and the mother carrying the fetus. Alternatively, the genomes are contributed to the sample by aneuploid cancerous cells and normal euploid cells from the same subject, *e.g.,* a plasma sample from a cancer patient.

Apart from analyzing a patient's test sample, one or more normalizing chromosomes or one or more normalizing chromosome segments are selected for each possible chromosome of interest. The normalizing chromosomes or segments are identified asynchronously from the normal testing of patient samples, which may take place in a clinical setting. In other words, the normalizing chromosomes or segments are identified prior to testing patient samples. The associations between normalizing chromosomes or segments and chromosomes or segments of interest are stored for use during testing. As explained below, such association is typically maintained over periods of time that span testing of many samples. The following discussion concerns embodiments for selecting normalizing chromosomes or chromosome segments for individual chromosomes or segments of interest.

A set of qualified samples is obtained to identify qualified normalizing sequences and to provide variance values for use in determining statistically meaningful identification of CNV in test samples. In step **110,** a plurality of biological qualified samples are obtained from a plurality of subjects known to comprise cells having a normal copy number for any one sequence of interest. In one embodiment, the qualified samples are obtained from mothers pregnant with a fetus that has been confirmed using cytogenetic means to have a normal copy number of chromosomes. The biological qualified samples may be a biological fluid, *e.g.,* plasma, or any suitable sample as described below. In some embodiments, a qualified sample contains a mixture of nucleic acid molecules, *e.g.,* cfDNA molecules. In some embodiments, the qualified sample is a maternal plasma sample that contains a mixture of fetal and maternal cfDNA molecules. Sequence information for normalizing chromosomes and/or segments thereof is obtained by sequencing at least a portion of the nucleic acids, *e.g.,* fetal and maternal nucleic acids, using any known sequencing method. Preferably, any one of the Next Generation Sequencing (NGS) methods described elsewhere herein is used to sequence the fetal and maternal nucleic acids as single or clonally amplified molecules. In various embodiments, the qualified samples are processed as disclosed below prior to and during sequencing. They may be processed using apparatus, systems, and kits as disclosed herein.

In step **120,** at least a portion of each of all the qualified nucleic acids contained in the qualified samples are sequenced to generate millions of sequence reads, *e.g.,* 36bp reads, which are aligned to a reference genome, *e.g.,* hg18. In some embodiments, the sequence reads comprise about 20bp, about 25bp, about 30bp, about 35bp, about 40bp, about 45bp, about 50bp, about 55bp, about 60bp, about 65bp, about 70bp, about 75bp, about 80bp, about 85bp, about90bp, about 95bp, about 100bp, about 110bp, about 120bp, about 130, about 140bp, about 150bp, about 200bp, about 250bp, about 300bp, about 350bp, about 400bp, about 450bp, or about 500bp. It is expected that technological advances will enable single-end reads of greater than 500bp enabling for reads of greater than about 1000bp when paired end reads are generated. In one embodiment, the mapped sequence reads comprise 36bp. In another embodiment, the mapped sequence reads comprise 25bp.

Sequence reads are aligned to a reference genome, and the reads that are uniquely mapped to the reference genome are known as sequence tags. Sequence tags falling on unmasked segments of a masked reference sequence are counted for analysis of CNV.

In one embodiment, at least about $3 \times 10^6$ qualified sequence tags, at least about $5 \times 10^6$ qualified sequence tags, at least about $8 \times 10^6$ qualified sequence tags, at least about $10 \times 10^6$ qualified sequence tags, at least about $15 \times 10^6$ qualified sequence tags, at least about $20 \times 10^6$ qualified sequence tags, at least about $30 \times 10^6$ qualified sequence tags, at least about $40 \times 10^6$ qualified sequence tags, or at least about $50 \times 10^6$ qualified sequence tags comprising between 20 and 40bp reads are obtained from reads that map uniquely to a reference genome.

In step **130,** all the tags obtained from sequencing the nucleic acids in the qualified samples are counted to determine a qualified sequence tag density. In one embodiment the sequence tag density is determined as the number of qualified sequence tags mapped to the sequence of interest on the reference genome. In another embodiment, the qualified sequence tag density is determined as the number of qualified sequence tags mapped to a sequence of interest normalized to the length of the qualified sequence of interest to which they are mapped. Sequence tag densities that are determined as a ratio of the tag density relative to the length of the sequence of interest are herein referred to as tag density ratios. Normalization to the length of the sequence of interest is not required, and may be included as a step to reduce the number of digits in a number to simplify it for human interpretation. As all qualified sequence tags are mapped and counted in each of the qualified samples, the sequence tag density for a sequence of interest, *e.g.,* a clinically-relevant sequence, in the qualified samples is determined, as are the sequence tag densities for additional sequences from which normalizing sequences are identified subsequently.

In some embodiments, the sequence of interest is a chromosome that is associated with a complete chromosomal aneuploidy, e.g., chromosome 21, and the qualified normalizing sequence is a complete chromosome that is not associated with a chromosomal aneuploidy and whose variation in sequence tag density approximates that of the sequence (*i.e.,* chromosome) of interest, *e.g.,* chromosome 21. The selected normalizing chromosome(s) may be the one or group that best approximates the variation in sequence tag density of the sequence of interest. Any one or more of chromosomes 1-22, X, and Y can be a sequence of interest, and one or more chromosomes can be identified as the normalizing sequence for each of the any one chromosomes 1-22, X and Y in the qualified samples. The normalizing chromosome can be an individual chromosome or it can be a group of chromosomes as described elsewhere herein.

In another embodiment, the sequence of interest is a segment of a chromosome associated with a partial aneuploidy, e.g., a chromosomal deletion or insertion, or unbalanced chromosomal translocation, and the normalizing sequence is a chromosomal segment (or group of segments) that is not associated with the partial aneuploidy and whose variation in sequence tag density approximates that of the chromosome segment associated with the partial aneuploidy.

The selected normalizing chromosome segment(s) may be the one or more that best approximates the variation in sequence tag density of the sequence of interest. Any one or more segments of any one or more chromosomes 1-22, X, and Y can be a sequence of interest.

In other embodiments, the sequence of interest is a segment of a chromosome associated with a partial aneuploidy and the normalizing sequence is a whole chromosome or chromosomes. In still other embodiments, the sequence of interest is a whole chromosome associated with an aneuploidy and the normalizing sequence is a chromosomal segment or segments that are not associated with the aneuploidy.

Whether a single sequence or a group of sequences are identified in the qualified samples as the normalizing sequence(s) for any one or more sequences of interest, the qualified normalizing sequence may be chosen to have a variation in sequence tag density that best or effectively approximates that of the sequence of interest as determined in the qualified samples. For example, a qualified normalizing sequence is a sequence that produces the smallest variability across the qualified samples when used to normalize the sequence of interest, *i.e.,* the variability of the normalizing sequence is closest to that of the sequence of interest determined in qualified samples. Stated another way, the qualified normalizing sequence is the sequence selected to produce the least variation in sequence dose (for the sequence of interest) across the qualified samples. Thus, the process selects a sequence that when used as a normalizing chromosome is expected to produce the smallest variability in run-to-run chromosome dose for the sequence of interest.

The normalizing sequence identified in the qualified samples for any one or more sequences of interest remains the normalizing sequence of choice for determining the presence or absence of aneuploidy in test samples over days, weeks, months, and possibly years, provided that procedures needed to generate sequencing libraries, and sequencing the samples are essentially unaltered over time. As described above, normalizing sequences for determining the presence of aneuploidies are chosen for (possibly among other reasons as well) the variability in the number of sequence tags that are mapped to it among samples, *e.g.,* different samples, and sequencing runs, *e.g.,* sequencing runs that occur on the same day and/or different days, that best approximates the variability of the sequence of interest for which it is used as a normalizing parameter. Substantial alterations in these procedures will affect the number of tags that are mapped to all sequences, which in turn will determine which one or group of sequences will have a variability across samples in the same and/or in different sequencing runs, on the same day or on different days that most closely approximates that of the sequence(s) of interest, which would require that the set of normalizing sequences be re-determined. Substantial alterations in procedures include changes in the laboratory protocol used for preparing the sequencing library, which includes changes related to preparing samples for multiplex sequencing instead of singleplex sequencing, and changes in sequencing platforms, which include changes in the chemistry used for sequencing.

In some embodiments, the normalizing sequence chosen to normalize a particular sequence of interest is a sequence that best distinguishes one or more qualified, samples from one or more affected samples, which implies that the normalizing sequence is a sequence that has the greatest differentiability, *i.e.,* the differentiability of the normalizing sequence is such that it provides optimal differentiation to a sequence of interest in an affected test sample to easily distinguish the affected test sample from other unaffected samples. In other embodiments, the normalizing sequence is a sequence that has a combination of the smallest variability and the greatest differentiability.

The level of differentiability can be determined as a statistical difference between the sequence doses, *e.g.,* chromosome doses or segment doses, in a population of qualified samples and the chromosome dose(s) in one or more test samples as described below and shown in the Examples. For example, differentiability can be represented numerically as a t-test value, which represents the statistical difference between the chromosome doses in a population of qualified samples and the chromosome dose(s) in one or more test samples. Similarly, differentiability can be based on segment doses instead of chromosome doses. Alternatively, differentiability can be represented numerically as a Normalized Chromosome Value (NCV), which is a z-score for chromosome doses as long as the distribution for the NCV is normal. Similarly, in the case where chromosome segments are the sequences of interest, differentiability of segment doses can be represented numerically as a Normalized Segment Value (NSV), which is a z-score for chromosome segment doses as long as the distribution for the NSV is normal. In determining the z-score, the mean and standard deviation of chromosome or segment doses in a set of qualified samples can be used. Alternatively, the mean and standard deviation of chromosome or segment doses in a training set comprising qualified samples and affected samples can be used. In other embodiments, the normalizing sequence is a sequence that has the smallest variability and the greatest differentiability or an optimal combination of small variability and large differentiability.

The method identifies sequences that inherently have similar characteristics and that are prone to similar variations among samples and sequencing runs, and which are useful for determining sequence doses in test samples.

### *Determination of sequence doses*

In some embodiments, chromosome or segment doses for one or more chromosomes or segments of interest are determined in all qualified samples as described in step **140** shown in Figure 4, and a normalizing chromosome or segment sequence is identified in step **145**. Note, although step **145** is shown as downstream of step **140,** some normalizing sequences are provided before sequence doses are calculated. Then one or more normalizing sequences are identified according to various criteria as further described below, see step **145.** In some embodiments, e.g., the identified normalizing sequence results in the smallest variability in sequence dose for the sequence of interest across all qualified samples.

In step **140,** based on the calculated qualified tag densities, a qualified sequence dose, *i.e.,* a chromosome dose or a segment dose, for a sequence of interest is determined as the ratio of the sequence tag density for the sequence of interest and the qualified sequence tag density for additional sequences from which normalizing sequences are identified subsequently in step **145.** The identified normalizing sequences are used subsequently to determine sequence doses in test samples.

In one embodiment, the sequence dose in the qualified samples is a chromosome dose that is calculated as the ratio of the number of sequence tags for a chromosome of interest and the number of sequence tags for a normalizing chromosome sequence in a qualified sample. The normalizing chromosome sequence can be a single chromosome, a group of chromosomes, a segment of one chromosome, or a group of segments from different chromosomes. Accordingly, a chromosome dose for a chromosome of interest is determined in a qualified sample as the ratio of the number of tags for a chromosome of interest and the number of tags for (i) a normalizing chromosome sequence composed of a single chromosome, (ii) a normalizing chromosome sequence composed of two or more chromosomes, (iii) a normalizing segment sequence composed of a single segment of a chromosome, (iv) a normalizing segment sequence composed of two or more segments form one chromosome, or (v) a normalizing segment sequence composed of two or more segments of two or more chromosomes. Examples for determining a chromosome dose for chromosome of interest 21 according to (i)-(v) are as follows: chromosome doses for chromosome of interest, *e.g.,* chromosome 21, are determined as a ratio of the sequence tag density of chromosome 21 and one of the following sequence tag densities: (i) each of all the remaining chromosomes, *i.e.,* chromosomes 1-20, chromosome 22, chromosome X, and chromosome Y; (ii) all possible combinations of two or more remaining chromosomes; (iii) a segment of another chromosome, *e.g.,* chromosome 9; (iv) two segments of one other chromosome, *e.g.,* two segments of chromosome 9; (v) two segments of two different chromosomes, *e.g.,* a segment of chromosome 9 and a segment of chromosome 14.

In another embodiment, the sequence dose in the qualified samples is a segment dose as opposed to a chromosome dose, which segment dose is calculated as the ratio of the number of sequence tags for a segment of interest, that is not a whole chromosome, and the number of sequence tags for a normalizing segment sequence in a qualified sample. The normalizing segment sequence can be any of the normalizing chromosome or segment sequences discussed above.

### *Identification of normalizing sequences*

In step **145,** a normalizing sequence is identified for a sequence of interest. In some embodiments, *e.g.,* the normalizing sequence is the sequence based on the calculated sequence doses, *e.g.,* that results in the smallest variability in sequence dose for the sequence of interest across all qualified samples. The method identifies sequences that inherently have similar characteristics and are prone to similar variations among samples and sequencing runs, and which are useful for determining sequence doses in test samples.

Normalizing sequences for one or more sequences of interest can be identified in a set of qualified samples, and the sequences that are identified in the qualified samples are used subsequently to calculate sequence doses for one or more sequences of interest in each of the test samples (step **150**) to determine the presence or absence of aneuploidy in each of the test samples. The normalizing sequence identified for chromosomes or segments of interest may differ when different sequencing platforms are used and/or when differences exist in the purification of the nucleic acid that is to be sequenced and/or preparation of the sequencing library. The use of normalizing sequences according to the methods described herein provides specific and sensitive measure of a variation in copy number of a chromosome or segment thereof irrespective of sample preparation and/or sequencing platform that is used.

In some embodiments, more than one normalizing sequence is identified, *i.e.,* different normalizing sequences can be determined for one sequence of interest, and multiple sequence doses can be determined for one sequence of interest. For example, the variation, *e.g.,* coefficient of variation (CV= standard deviation/mean), in chromosome dose for chromosome of interest 21 is least when the sequence tag density of chromosome 14 is used. However, two, three, four, five, six, seven, eight or more normalizing sequences can be identified for use in determining a sequence dose for a sequence of interest in a test sample. As an example, a second dose for chromosome 21 in any one test sample can be determined using chromosome 7, chromosome 9, chromosome 11 or chromosome 12 as the normalizing chromosome sequence as these chromosomes all have CV close to that for chromosome 14 (see Example **4**, Table **2**).

In some embodiments, when a single chromosome is chosen as the normalizing chromosome sequence for a chromosome of interest, the normalizing chromosome sequence will be a chromosome that results in chromosome doses for the chromosome of interest that has the smallest variability across all samples tested, *e.g.,* qualified samples. In some instances, the best normalizing chromosome may not have the least variation, but may have a distribution of qualified doses that best distinguishes a test sample or samples from the qualified samples, *i.e.,* the best normalizing chromosome may not have the lowest variation, but may have the greatest differentiability.

### *Determination of aneuploidies in test samples*

Based on the identification of the normalizing sequence(s) in qualified samples, a sequence dose is determined for a sequence of interest in a test sample comprising a mixture of nucleic acids derived from genomes that differ in one or more sequences of interest.

In step **115,** a test sample is obtained from a subject suspected or known to carry a clinically-relevant CNV of a sequence of interest. The test sample may be a biological fluid, *e.g.,* plasma, or any suitable sample as described below. As explained, the sample may be obtained using a non-invasive procedure such as a simple blood draw. In some embodiments, a test sample contains a mixture of nucleic acid molecules, *e.g.,* cfDNA molecules. In some embodiments, the test sample is a maternal plasma sample that contains a mixture of fetal and maternal cfDNA molecules.

In step **125,** at least a portion of the test nucleic acids in the test sample is sequenced as described for the qualified samples to generate millions of sequence reads, *e.g.,* 36bp reads. As in step **120,** the reads generated from sequencing the nucleic acids in the test sample are uniquely mapped or aligned to a reference genome to produce tags. As described in step **120,** at least about $3 \times 10^6$ qualified sequence tags, at least about $5 \times 10^6$ qualified sequence tags, at least about $8 \times 10^6$ qualified sequence tags, at least about $10 \times 10^6$ qualified sequence tags, at least about $15 \times 10^6$ qualified sequence tags, at least about $20 \times 10^6$ qualified sequence tags, at least about $30 \times 10^6$ qualified sequence tags, at least about $40 \times 10^6$ qualified sequence tags, or at least about $50 \times 10^6$ qualified sequence tags comprising between 20 and 40bp reads are obtained from reads that map uniquely to a reference genome. In certain embodiments, the reads produced by sequencing apparatus are provided in an electronic format. Alignment is accomplished using computational apparatus as discussed below. Individual reads are compared against the reference genome, which is often vast (millions of base pairs) to identify sites where the reads uniquely correspond with the reference genome. In some embodiments, the alignment procedure permits limited mismatch between reads and the reference genome. In some cases, 1, 2, or 3 base pairs in a read are permitted to mismatch corresponding base pairs in a reference genome, and yet a mapping is still made.

In step **135,** all or most of the tags obtained from sequencing the nucleic acids in the test samples are counted to determine a test sequence tag density using a computational apparatus as described below. In some embodiments, each read is aligned to a particular region of the reference genome (a chromosome or segment in most cases), and the read is converted to a tag by appending site information to the read. As this process unfolds, the computational apparatus may keep a running count of the number of tags/reads mapping to each region of the reference genome (chromosome or segment in most cases). The counts are stored for each chromosome or segment of interest and each corresponding normalizing chromosome or segment.

In certain embodiments, the reference genome has one or more excluded regions that are part of a true biological genome but are not included in the reference genome. Reads potentially aligning to these excluded regions are not counted. Examples of excluded regions include regions of long repeated sequences, regions of similarity between X and Y chromosomes, *etc.* Using a masked reference sequence obtained by masking techniques described above, only tags on unmasked segments of the reference sequence are taken into account for analysis of CNV.

In some embodiments, the method determines whether to count a tag more than once when multiple reads align to the same site on a reference genome or sequence. There may be occasions when two tags have the same sequence and therefore align to an identical site on a reference sequence. The method employed to count tags may under certain circumstances exclude from the count identical tags deriving from the same sequenced sample. If a disproportionate number of tags are identical in a given sample, it suggests that there is a strong bias or other defect in the procedure. Therefore, in accordance with certain embodiments, the counting method does not count tags from a given sample that are identical to tags from the sample that were previously counted.

Various criteria may be set for choosing when to disregard an identical tag from a single sample. In certain embodiments, a defined percentage of the tags that are counted must be unique. If more tags than this threshold are not unique, they are disregarded. For example, if the defined percentage requires that at least 50% are unique, identical tags are not counted until the percentage of unique tags exceeds 50% for the sample. In other embodiments, the threshold number of unique tags is at least about 60%. In other embodiments, the threshold percentage of unique tags is at least about 75%, or at least about 90%, or at least about 95%, or at least about 98%, or at least about 99%. A threshold may be set at 90% for chromosome 21. If 30M tags are aligned to chromosome 21, then at least 27M of them must be unique. If 3M counted tags are not unique and the 30 million and first tag is not unique, it is not counted. The choice of the particular threshold or other criterion used to determine when not to count further identical tags can be selected using appropriate statistical analysis. One factor influencing this threshold or other criterion is the relative amount of sequenced sample to the size of the genome to which tags can be aligned. Other factors include the size of the reads and similar considerations.

In one embodiment, the number of test sequence tags mapped to a sequence of interest is normalized to the known length of a sequence of interest to which they are mapped to provide a test sequence tag density ratio. As described for the qualified samples, normalization to the known length of a sequence of interest is not required, and may be included as a step to reduce the number of digits in a number to simplify it for human interpretation. As all the mapped test sequence tags are counted in the test sample, the sequence tag density for a sequence of interest, *e.g.,* a clinically-relevant sequence, in the test samples is determined, as are the sequence tag densities for additional sequences that correspond to at least one normalizing sequence identified in the qualified samples.

In step **150,** based on the identity of at least one normalizing sequence in the qualified samples, a test sequence dose is determined for a sequence of interest in the test sample. In various embodiments, the test sequence dose is computationally determined using the sequence tag densities of the sequence of interest and the corresponding normalizing sequence as described herein. The computational apparatus responsible for this undertaking will electronically access the association between the sequence of interest and its associated normalizing sequence, which may be stored in a database, table, graph, or be included as code in program instructions.

As described elsewhere herein, the at least one normalizing sequence can be a single sequence or a group of sequences. The sequence dose for a sequence of interest in a test sample is a ratio of the sequence tag density determined for the sequence of interest in the test sample and the sequence tag density of at least one normalizing sequence determined in the test sample, wherein the normalizing sequence in the test sample corresponds to the normalizing sequence identified in the qualified samples for the particular sequence of interest. For example, if the normalizing sequence identified for chromosome 21 in the qualified samples is determined to be a chromosome, *e.g.,* chromosome 14, then the test sequence dose for chromosome 21 (sequence of interest) is determined as the ratio of the sequence tag density for chromosome 21 in and the sequence tag density for chromosome 14 each determined in the test sample. Similarly, chromosome doses for chromosomes 13, 18, X, Y, and other chromosomes associated with chromosomal aneuploidies are determined. A normalizing sequence for a chromosome of interest can be one or a group of chromosomes, or one or a group of chromosome segments. As described previously, a sequence of interest can be part of a chromosome, e.g., a chromosome segment. Accordingly, the dose for a chromosome segment can be determined as the ratio of the sequence tag density determined for the segment in the test sample and the sequence tag density for the normalizing chromosome segment in the test sample, wherein the normalizing segment in the test sample corresponds to the normalizing segment (single or a group of segments) identified in the qualified samples for the particular segment of interest. Chromosome segments can range from kilobases (kb) to megabases (Mb) in size (*e.g.,* about 1kb to 10 kb, or about 10 kb to 100 kb, or about 100kb to 1 Mb).

In step **155,** threshold values are derived from standard deviation values established for qualified sequence doses determined in a plurality of qualified samples and sequence doses determined for samples known to be aneuploid for a sequence of interest. Note that this operation is typically performed asynchronously with analysis of patient test samples. It may be performed, for example, concurrently with the selection of normalizing sequences from qualified samples. Accurate classification depends on the differences between probability distributions for the different classes, *i.e.,* type of aneuploidy. In some examples, thresholds are chosen from empirical distribution for each type of aneuploidy, *e.g.,* trisomy 21. Possible threshold values that were established for classifying trisomy 13, trisomy 18, trisomy 21, and monosomy X aneuploidies as described in the Examples, which describe the use of the method for determining chromosomal aneuploidies by sequencing cfDNA extracted from a maternal sample comprising a mixture of fetal and maternal nucleic acids. The threshold value that is determined to distinguish samples affected for an aneuploidy of a chromosome can be the same or can be different from the threshold for a different aneuploidy. As is shown in the Examples, the threshold value for each chromosome of interest is determined from the variability in the dose of the chromosome of interest across samples and sequencing runs. The less variable the chromosome dose for any chromosome of interest, the narrower the spread in the dose for the chromosome of interest across all the unaffected samples, which are used to set the threshold for determining different aneuploidies.

Returning to the process flow associated with classifying a patient test sample, in step **160,** the copy number variation of the sequence of interest is determined in the test sample by comparing the test sequence dose for the sequence of interest to at least one threshold value established from the qualified sequence doses. This operation may be performed by the same computational apparatus employed to measure sequence tag densities and/or calculate segment doses.

In step **165,** the calculated dose for a test sequence of interest is compared to that set as the threshold values that are chosen according to a user-defined "threshold of reliability" to classify the sample as a "normal" an "affected" or a "no call." The "no call" samples are samples for which a definitive diagnosis cannot be made with reliability. Each type of affected sample (*e.g.*, trisomy 21, partial trisomy 21, monosomy X) has its own thresholds, one for calling normal (unaffected) samples and another for calling affected samples (although in some cases the two thresholds coincide). As described elsewhere herein, under some circumstances a no-call can be converted to a call (affected or normal) if fetal fraction of nucleic acid in the test sample is sufficiently high. The classification of the test sequence may be reported by the computational apparatus employed in other operations of this process flow. In some cases, the classification is reported in an electronic format and may be displayed, emailed, texted, *etc.* to interest persons.

Certain embodiments provide a method for providing prenatal diagnosis of a fetal chromosomal aneuploidy in a biological sample comprising fetal and maternal nucleic acid molecules. The diagnosis is made based on obtaining sequence information from at least a portion of the mixture of the fetal and maternal nucleic acid molecules derived from a biological test sample, *e.g.,* a maternal plasma sample, computing from the sequencing data a normalizing chromosome dose for one or more chromosomes of interest, and/or a normalizing segment dose for one or more segments of interest, and determining a statistically significant difference between the chromosome dose for the chromosome of interest and/or the segment dose for the segment of interest, respectively, in the test sample and a threshold value established in a plurality of qualified (normal) samples, and providing the prenatal diagnosis based on the statistical difference. As described in step **165** of the method, a diagnosis of normal or affected is made. A "no call" is provided in the event that the diagnosis for normal or affected cannot be made with confidence.

## Samples and Sample Processing

### *Samples*

Samples that are used for determining a CNV, e.g., chromosomal aneuploidies, partial aneuploidies, and the like, can include samples taken from any cell, tissue, or organ in which copy number variations for one or more sequences of interest are to be determined. Desirably, the samples contain nucleic acids that are that are present in cells and/or nucleic acids that are "cell-free" (*e.g.,* cfDNA).

In some embodiments it is advantageous to obtain cell-free nucleic acids, *e.g.,* cell-free DNA (cfDNA). Cell-free nucleic acids, including cell-free DNA, can be obtained by various methods known in the art from biological samples including but not limited to plasma, serum, and urine (*see, e.g.,* Fan et al., Proc Natl Acad Sci 105:16266-16271 [2008]; Koide et al., Prenatal Diagnosis 25:604-607 [2005]; Chen et $\alpha$/., Nature Med. 2: 1033-1035 [1996]; Lo et al., Lancet 350: 485-487 [1997]; Botezatu et al., Clin Chem. 46: 1078-1084, 2000; and Su et al., J Mol. Diagn. 6: 101-107 [2004]). To separate cell-free DNA from cells in a sample, various methods including, but not limited to fractionation, centrifugation (*e.g.*, density gradient centrifugation), DNA-specific precipitation, or high-throughput cell sorting and/or other separation methods can be used. Commercially available kits for manual and automated separation of cfDNA are available (Roche Diagnostics, Indianapolis, IN, Qiagen, Valencia, CA, Macherey-Nagel, Duren, DE). Biological samples comprising cfDNA have been used in assays to determine the presence or absence of chromosomal abnormalities, *e.g.*, trisomy 21, by sequencing assays that can detect chromosomal aneuploidies and/or various polymorphisms.

In various embodiments the cfDNA present in the sample can be enriched specifically or non-specifically prior to use (*e.g.,* prior to preparing a sequencing library). Non-specific enrichment of sample DNA refers to the whole genome amplification of the genomic DNA fragments of the sample that can be used to increase the level of the sample DNA prior to preparing a cfDNA sequencing library. Non-specific enrichment can be the selective enrichment of one of the two genomes present in a sample that comprises more than one genome. For example, non-specific enrichment can be selective of the fetal genome in a maternal sample, which can be obtained by known methods to increase the relative proportion of fetal to maternal DNA in a sample. Alternatively, non-specific enrichment can be the non-selective amplification of both genomes present in the sample. For example, non-specific amplification can be of fetal and maternal DNA in a sample comprising a mixture of DNA from the fetal and maternal genomes. Methods for whole genome amplification are known in the art. Degenerate oligonucleotide-primed PCR (DOP), primer extension PCR technique (PEP) and multiple displacement amplification (MDA) are examples of whole genome amplification methods. In some embodiments, the sample comprising the mixture of cfDNA from different genomes is un-enriched for cfDNA of the genomes present in the mixture. In other embodiments, the sample comprising the mixture of cfDNA from different genomes is non-specifically enriched for any one of the genomes present in the sample.

The sample comprising the nucleic acid(s) to which the methods described herein are applied typically comprises a biological sample ("test sample"), *e.g.,* as described above. In some embodiments, the nucleic acid(s) to be screened for one or more CNVs is purified or isolated by any of a number of well-known methods.

Accordingly, in certain embodiments the sample comprises or consists of a purified or isolated polynucleotide, or it can comprise samples such as a tissue sample, a biological fluid sample, a cell sample, and the like. Suitable biological fluid samples include, but are not limited to blood, plasma, serum, sweat, tears, sputum, urine, ear flow, lymph, saliva, cerebrospinal fluid, ravages, bone marrow suspension, vaginal flow, trans-cervical lavage, brain fluid, ascites, milk, secretions of the respiratory, intestinal and genitourinary tracts, amniotic fluid, milk, and leukophoresis samples. In some embodiments, the sample is a sample that is easily obtainable by non-invasive procedures, *e.g.*, blood, plasma, serum, sweat, tears, sputum, urine, ear flow, saliva or feces. In certain embodiments the sample is a peripheral blood sample, or the plasma and/or serum fractions of a peripheral blood sample. In other embodiments, the biological sample is a swab or smear, a biopsy specimen, or a cell culture. In another embodiment, the sample is a mixture of two or more biological samples, *e.g.,* a biological sample can comprise two or more of a biological fluid sample, a tissue sample, and a cell culture sample. As used herein, the terms "blood," "plasma" and "serum" expressly encompass fractions or processed portions thereof. Similarly, where a sample is taken from a biopsy, swab, smear, *etc.,* the "sample" expressly encompasses a processed fraction or portion derived from the biopsy, swab, smear, *etc.*

In certain embodiments, samples can be obtained from sources, including, but not limited to, samples from different individuals, samples from different developmental stages of the same or different individuals, samples from different diseased individuals (*e.g.*, individuals with cancer or suspected of having a genetic disorder), normal individuals, samples obtained at different stages of a disease in an individual, samples obtained from an individual subjected to different treatments for a disease, samples from individuals subjected to different environmental factors, samples from individuals with predisposition to a pathology, samples individuals with exposure to an infectious disease agent (*e.g.,* HIV), and the like.

In one illustrative, but non-limiting embodiment, the sample is a maternal sample that is obtained from a pregnant female, for example a pregnant woman. In this instance, the sample can be analyzed using the methods described herein to provide a prenatal diagnosis of potential chromosomal abnormalities in the fetus. The maternal sample can be a tissue sample, a biological fluid sample, or a cell sample. A biological fluid includes, as non-limiting examples, blood, plasma, serum, sweat, tears, sputum, urine, ear flow, lymph, saliva, cerebrospinal fluid, ravages, bone marrow suspension, vaginal flow, transcervical lavage, brain fluid, ascites, milk, secretions of the respiratory, intestinal and genitourinary tracts, and leukophoresis samples.

In another illustrative, but non-limiting embodiment, the maternal sample is a mixture of two or more biological samples, *e.g.*, the biological sample can comprise two or more of a biological fluid sample, a tissue sample, and a cell culture sample. In some embodiments, the sample is a sample that is easily obtainable by non-invasive procedures, *e.g.*, blood, plasma, serum, sweat, tears, sputum, urine, milk, ear flow, saliva and feces. In some embodiments, the biological sample is a peripheral blood sample, and/or the plasma and serum fractions thereof. In other embodiments, the biological sample is a swab or smear, a biopsy specimen, or a sample of a cell culture. As disclosed above, the terms "blood," "plasma" and "serum" expressly encompass fractions or processed portions thereof. Similarly, where a sample is taken from a biopsy, swab, smear, *etc.,* the "sample" expressly encompasses a processed fraction or portion derived from the biopsy, swab, smear, *etc.*

In certain embodiments samples can also be obtained from *in vitro* cultured tissues, cells, or other polynucleotide-containing sources. The cultured samples can be taken from sources including, but not limited to, cultures (*e.g.*, tissue or cells) maintained in different media and conditions (*e.g.*, pH, pressure, or temperature), cultures (*e.g.,* tissue or cells) maintained for different periods of length, cultures (*e.g.,* tissue or cells) treated with different factors or reagents (*e.g.,* a drug candidate, or a modulator), or cultures of different types of tissue and/or cells.

Methods of isolating nucleic acids from biological sources are well known and will differ depending upon the nature of the source. One of skill in the art can readily isolate nucleic acid(s) from a source as needed for the method described herein. In some instances, it can be advantageous to fragment the nucleic acid molecules in the nucleic acid sample. Fragmentation can be random, or it can be specific, as achieved, for example, using restriction endonuclease digestion. Methods for random fragmentation are well known in the art, and include, for example, limited DNAse digestion, alkali treatment and physical shearing. In one embodiment, sample nucleic acids are obtained from as cfDNA, which is not subjected to fragmentation.

In other illustrative embodiments, the sample nucleic acid(s) are obtained as genomic DNA, which is subjected to fragmentation into fragments of approximately 300 or more, approximately 400 or more, or approximately 500 or more base pairs, and to which NGS methods can be readily applied.

### *Sequencing Library Preparation*

In one embodiment, the methods described herein can utilize next generation sequencing technologies (NGS), that allow multiple samples to be sequenced individually as genomic molecules (*i.e.,* singleplex sequencing) or as pooled samples comprising indexed genomic molecules (*e.g.*, multiplex sequencing) on a single sequencing run. These methods can generate up to several hundred million reads of DNA sequences. In various embodiments the sequences of genomic nucleic acids, and/or of indexed genomic nucleic acids can be determined using, for example, the Next Generation Sequencing Technologies (NGS) described herein. In various embodiments analysis of the massive amount of sequence data obtained using NGS can be performed using one or more processors as described herein.

In various embodiments the use of such sequencing technologies does not involve the preparation of sequencing libraries.

However, in certain embodiments the sequencing methods contemplated herein involve the preparation of sequencing libraries. In one illustrative approach, sequencing library preparation involves the production of a random collection of adapter-modified DNA fragments (*e.g.*, polynucleotides) that are ready to be sequenced. Sequencing libraries of polynucleotides can be prepared from DNA or RNA, including equivalents, analogs of either DNA or cDNA, for example, DNA or cDNA that is complementary or copy DNA produced from an RNA template, by the action of reverse transcriptase.

The polynucleotides may originate in double-stranded form (*e.g.*, dsDNA such as genomic DNA fragments, cDNA, PCR amplification products, and the like) or, in certain embodiments, the polynucleotides may originated in single-stranded form (*e.g.,* ssDNA, RNA, *etc.*) and have been converted to dsDNA form. By way of illustration, in certain embodiments, single stranded mRNA molecules may be copied into double-stranded cDNAs suitable for use in preparing a sequencing library. The precise sequence of the primary polynucleotide molecules is generally not material to the method of library preparation, and may be known or unknown. In one embodiment, the polynucleotide molecules are DNA molecules. More particularly, in certain embodiments, the polynucleotide molecules represent the entire genetic complement of an organism or substantially the entire genetic complement of an organism, and are genomic DNA molecules (*e.g.*, cellular DNA, cell free DNA (cfDNA), *etc.*)*,* that typically include both intron sequence and exon sequence (coding sequence), as well as non-coding regulatory sequences such as promoter and enhancer sequences. In certain embodiments, the primary polynucleotide molecules comprise human genomic DNA molecules, *e.g.*, cfDNA molecules present in peripheral blood of a pregnant subject.

Preparation of sequencing libraries for some NGS sequencing platforms is facilitated by the use of polynucleotides comprising a specific range of fragment sizes. Preparation of such libraries typically involves the fragmentation of large polynucleotides (*e.g.* cellular genomic DNA) to obtain polynucleotides in the desired size range.

Fragmentation can be achieved by any of a number of methods known to those of skill in the art. For example, fragmentation can be achieved by mechanical means including, but not limited to nebulization, sonication and hydroshear. However mechanical fragmentation typically cleaves the DNA backbone at C-O, P-O and C-C bonds resulting in a heterogeneous mix of blunt and 3'- and 5'-overhanging ends with broken C-O, P-O and/ C-C bonds (*see, e.g.,* Alnemri and Liwack, J Biol. Chem 265:17323-17333 [1990]; Richards and Boyer, J Mol Biol 11:327-240 [1965]) which may need to be repaired as they may lack the requisite 5'-phosphate for the subsequent enzymatic reactions, *e.g.*, ligation of sequencing adaptors, that are required for preparing DNA for sequencing.

In contrast, cfDNA, typically exists as fragments of less than about 300 base pairs and consequently, fragmentation is not typically necessary for generating a sequencing library using cfDNA samples.

Typically, whether polynucleotides are forcibly fragmented (*e.g.*, fragmented *in vitro*), or naturally exist as fragments, they are converted to blunt-ended DNA having 5'-phosphates and 3'-hydroxyl. Standard protocols, *e.g.*, protocols for sequencing using, for example, the Illumina platform as described elsewhere herein, instruct users to end-repair sample DNA, to purify the end-repaired products prior to dA-tailing, and to purify the dA-tailing products prior to the adaptor-ligating steps of the library preparation.

Various embodiments of methods of sequence library preparation described herein obviate the need to perform one or more of the steps typically mandated by standard protocols to obtain a modified DNA product that can be sequenced by NGS. An abbreviated method (ABB method), a 1-step method, and a 2-step method are examples of methods for preparation of a sequencing library, which can be found in patent application 13/555,037 filed on July 20, 2012 (published as US2013-0029852 A1).

### *Marker Nucleic Acids for tracking and verifying sample integrity*

In various embodiments verification of the integrity of the samples and sample tracking can be accomplished by sequencing mixtures of sample genomic nucleic acids, *e.g.*, cfDNA, and accompanying marker nucleic acids that have been introduced into the samples, *e.g.*, prior to processing.

Marker nucleic acids can be combined with the test sample (*e.g.*, biological source sample) and subjected to processes that include, for example, one or more of the steps of fractionating the biological source sample, *e.g.*, obtaining an essentially cell-free plasma fraction from a whole blood sample, purifying nucleic acids from a fractionated, *e.g.,* plasma, or unfractionated biological source sample, *e.g.,* a tissue sample, and sequencing. In some embodiments, sequencing comprises preparing a sequencing library. The sequence or combination of sequences of the marker molecules that are combined with a source sample is chosen to be unique to the source sample. In some embodiments, the unique marker molecules in a sample all have the same sequence. In other embodiments, the unique marker molecules in a sample are a plurality of sequences, *e.g.*, a combination of two, three, four, five, six, seven, eight, nine, ten, fifteen, twenty, or more different sequences.

In one embodiment, the integrity of a sample can be verified using a plurality of marker nucleic acid molecules having identical sequences. Alternatively, the identity of a sample can be verified using a plurality of marker nucleic acid molecules that have at least two, at least three, at least four, at least five, at least six, at least seven, at least eight, at least nine, at least ten, at least 11, at least 12, at least 13, at least 14, at least 15, at least 16, at least 17m, at least 18, at least 19, at least 20, at least 25, at least 30, at least 35, at least 40, at least 50, or more different sequences. Verification of the integrity of the plurality of biological samples, *i.e.,* two or more biological samples, requires that each of the two or more samples be marked with marker nucleic acids that have sequences that are unique to each of the plurality of test sample that is being marked. For example, a first sample can be marked with a marker nucleic acid having sequence A, and a second sample can be marked with a marker nucleic acid having sequence B. Alternatively, a first sample can be marked with marker nucleic acid molecules all having sequence A, and a second sample can be marked with a mixture of sequences B and C, wherein sequences A, B and C are marker molecules having different sequences.

The marker nucleic acid(s) can be added to the sample at any stage of sample preparation that occurs prior to library preparation (if libraries are to be prepared) and sequencing. In one embodiment, marker molecules can be combined with an unprocessed source sample. For example, the marker nucleic acid can be provided in a collection tube that is used to collect a blood sample. Alternatively, the marker nucleic acids can be added to the blood sample following the blood draw. In one embodiment, the marker nucleic acid is added to the vessel that is used to collect a biological fluid sample, *e.g.,* the marker nucleic acid(s) are added to a blood collection tube that is used to collect a blood sample. In another embodiment, the marker nucleic acid(s) are added to a fraction of the biological fluid sample. For example, the marker nucleic acid is added to the plasma and/or serum fraction of a blood sample, *e.g.,* a maternal plasma sample. In yet another embodiment, the marker molecules are added to a purified sample, *e.g.,* a sample of nucleic acids that have been purified from a biological sample. For example, the marker nucleic acid is added to a sample of purified maternal and fetal cfDNA. Similarly, the marker nucleic acids can be added to a biopsy specimen prior to processing the specimen. In some embodiments, the marker nucleic acids can be combined with a carrier that delivers the marker molecules into the cells of the biological sample. Cell-delivery carriers include pH-sensitive and cationic liposomes.

In various embodiments, the marker molecules have antigenomic sequences, that are sequences that are absent from the genome of the biological source sample. In an exemplary embodiment, the marker molecules that are used to verify the integrity of a human biological source sample have sequences that are absent from the human genome. In an alternative embodiment, the marker molecules have sequences that are absent from the source sample and from any one or more other known genomes. For example, the marker molecules that are used to verify the integrity of a human biological source sample have sequences that are absent from the human genome and from the mouse genome. The alternative allows for verifying the integrity of a test sample that comprises two or more genomes. For example, the integrity of a human cell-free DNA sample obtained from a subject affected by a pathogen, *e.g.,* a bacterium, can be verified using marker molecules having sequences that are absent from both the human genome and the genome of the affecting bacterium. Sequences of genomes of numerous pathogens, *e.g.*, bacteria, viruses, yeasts, fungi, protozoa *etc.,* are publicly available on the World Wide Web at ncbi.nlm.nih.gov/genomes. In another embodiment, marker molecules are nucleic acids that have sequences that are absent from any known genome. The sequences of marker molecules can be randomly generated algorithmically.

In various embodiments the marker molecules can be naturally-occurring deoxyribonucleic acids (DNA), ribonucleic acids or artificial nucleic acid analogs (nucleic acid mimics) including peptide nucleic acids (PMA), morpholino nucleic acid, locked nucleic acids, glycol nucleic acids, and threose nucleic acids, which are distinguished from naturally-occurring DNA or RNA by changes to the backbone of the molecule or DNA mimics that do not have a phosphodiester backbone. The deoxyribonucleic acids can be from naturally-occurring genomes or can be generated in a laboratory through the use of enzymes or by solid phase chemical synthesis. Chemical methods can also be used to generate the DNA mimics that are not found in nature. Derivatives of DNA are that are available in which the phosphodiester linkage has been replaced but in which the deoxyribose is retained include but are not limited to DNA mimics having backbones formed by thioformacetal or a carboxamide linkage, which have been shown to be good structural DNA mimics. Other DNA mimics include morpholino derivatives and the peptide nucleic acids (PNA), which contain an N-(2-aminoethyl)glycine-based pseudopeptide backbone (Ann Rev Biophys Biomol Struct 24:167-183 [1995]). PNA is an extremely good structural mimic of DNA (or of ribonucleic acid [RNA]), and PNA oligomers are able to form very stable duplex structures with Watson-Crick complementary DNA and RNA (or PNA) oligomers, and they can also bind to targets in duplex DNA by helix invasion (Mol Biotechnol 26:233-248 [2004]. Another good structural mimic/analog of DNA analog that can be used as a marker molecule is phosphorothioate DNA in which one of the non-bridging oxygens is replaced by a sulfur. This modification reduces the action of endo-and exonucleases2 including 5' to 3' and 3' to 5' DNA POL 1 exonuclease, nucleases S1 and P1, RNases, serum nucleases and snake venom phosphodiesterase.

The length of the marker molecules can be distinct or indistinct from that of the sample nucleic acids, *i.e.,* the length of the marker molecules can be similar to that of the sample genomic molecules, or it can be greater or smaller than that of the sample genomic molecules. The length of the marker molecules is measured by the number of nucleotide or nucleotide analog bases that constitute the marker molecule. Marker molecules having lengths that differ from those of the sample genomic molecules can be distinguished from source nucleic acids using separation methods known in the art. For example, differences in the length of the marker and sample nucleic acid molecules can be determined by electrophoretic separation, *e.g.*, capillary electrophoresis. Size differentiation can be advantageous for quantifying and assessing the quality of the marker and sample nucleic acids. Preferably, the marker nucleic acids are shorter than the genomic nucleic acids, and of sufficient length to exclude them from being mapped to the genome of the sample. For example, as a 30 base human sequence is needed to uniquely map it to a human genome. Accordingly in certain embodiments, marker molecules used in sequencing bioassays of human samples should be at least 30 bp in length.

The choice of length of the marker molecule is determined primarily by the sequencing technology that is used to verify the integrity of a source sample. The length of the sample genomic nucleic acids being sequenced can also be considered. For example, some sequencing technologies employ clonal amplification of polynucleotides, which can require that the genomic polynucleotides that are to be clonally amplified be of a minimum length. For example, sequencing using the Illumina GAII sequence analyzer includes an *in vitro* clonal amplification by bridge PCR (also known as cluster amplification) of polynucleotides that have a minimum length of 1 10bp, to which adaptors are ligated to provide a nucleic acid of at least 200 bp and less than 600 bp that can be clonally amplified and sequenced. In some embodiments, the length of the adaptor-ligated marker molecule is between about 200bp and about 600bp, between about 250bp and 550bp, between about 300bp and 500bp, or between about 350 and 450. In other embodiments, the length of the adaptor-ligated marker molecule is about 200bp. For example, when sequencing fetal cfDNA that is present in a maternal sample, the length of the marker molecule can be chosen to be similar to that of fetal cfDNA molecules. Thus, in one embodiment, the length of the marker molecule used in an assay that comprises massively parallel sequencing of cfDNA in a maternal sample to determine the presence or absence of a fetal chromosomal aneuploidy, can be about 150 bp, about 160bp, 170 bp, about 180bp, about 190bp or about 200bp; preferably, the marker molecule is about 170 pp. Other sequencing approaches, e.g., SOLiD sequencing, Polony Sequencing and 454 sequencing use emulsion PCR to clonally amplify DNA molecules for sequencing, and each technology dictates the minimum and the maximum length of the molecules that are to be amplified. The length of marker molecules to be sequenced as clonally amplified nucleic acids can be up to about 600bp. In some embodiments, the length of marker molecules to be sequenced can be greater than 600bp.

Single molecule sequencing technologies, that do not employ clonal amplification of molecules, and are capable of sequencing nucleic acids over a very broad range of template lengths, in most situations do not require that the molecules to be sequenced be of any specific length. However, the yield of sequences per unit mass is dependent on the number of 3' end hydroxyl groups, and thus having relatively short templates for sequencing is more efficient than having long templates. If starting with nucleic acids longer than 1000 nt, it is generally advisable to shear the nucleic acids to an average length of 100 to 200 nt so that more sequence information can be generated from the same mass of nucleic acids. Thus, the length of the marker molecule can range from tens of bases to thousands of bases. The length of marker molecules used for single molecule sequencing can be up to about 25bp, up to about 50bp, up to about 75bp, up to about 100bp, up to about 200bp, up to about 300bp, up to about 400bp, up to about 500bp, up to about 600bp, up to about 700bp, up to about 800 bp, up to about 900bp, up to about 1000bp, or more in length.

The length chosen for a marker molecule is also determined by the length of the genomic nucleic acid that is being sequenced. For example, cfDNA circulates in the human bloodstream as genomic fragments of cellular genomic DNA. Fetal cfDNA molecules found in the plasma of pregnant women are generally shorter than maternal cfDNA molecules (Chan et al., Clin Chem 50:8892 [2004]). Size fractionation of circulating fetal DNA has confirmed that the average length of circulating fetal DNA fragments is <300 bp, while maternal DNA has been estimated to be between about 0.5 and 1 Kb (Li et al., Clin Chem, 50: 1002-1011 [2004]). These findings are consistent with those of Fan *et al.*, who determined using NGS that fetal cfDNA is rarely >340bp (Fan et al., Clin Chem 56:1279-1286 [2010]). DNA isolated from urine with a standard silica-based method consists of two fractions, high molecular weight DNA, which originates from shed cells and low molecular weight (150-250 base pair) fraction of transrenal DNA (Tr-DNA) (Botezatu et al., Clin Chem. 46: 1078-1084, 2000; and Su et al., J Mol. Diagn. 6: 101-107, 2004). The application of newly developed technique for isolation of cell-free nucleic acids from body fluids to the isolation of transrenal nucleic acids has revealed the presence in urine of DNA and RNA fragments much shorter than 150 base pairs (U.S. Patent Application Publication No. 20080139801). In embodiments, wherein cfDNA is the genomic nucleic acid that is sequenced, marker molecules that are chosen can be up to about the length of the cfDNA. For example, the length of marker molecules used in maternal cfDNA samples to be sequenced as single nucleic acid molecules or as clonally amplified nucleic acids can be between about 100 bp and 600. In other embodiments, the sample genomic nucleic acids are fragments of larger molecules. For example, a sample genomic nucleic acid that is sequenced is fragmented cellular DNA. In embodiments, when fragmented cellular DNA is sequenced, the length of the marker molecules can be up to the length of the DNA fragments. In some embodiments, the length of the marker molecules is at least the minimum length required for mapping the sequence read uniquely to the appropriate reference genome. In other embodiments, the length of the marker molecule is the minimum length that is required to exclude the marker molecule from being mapped to the sample reference genome.

In addition, marker molecules can be used to verify samples that are not assayed by nucleic acid sequencing, and that can be verified by common biotechniques other than sequencing, e.g., real-time PCR.

### Sample controls (*e.g., in process positive controls for sequencing and/or analysis*).

In various embodiments marker sequences introduced into the samples, e.g., as described above, can function as positive controls to verity the verify the accuracy and efficacy of sequencing and subsequent processing and analysis.

Accordingly, compositions and method for providing an in-process positive control (IPC) for sequencing DNA in a sample are provided. In certain embodiments, positive controls are provided for sequencing cfDNA in a sample comprising a mixture of genomes are provided. An IPC can be used to relate baseline shifts in sequence information obtained from different sets of samples, e.g., samples that are sequenced at different times on different sequencing runs. Thus, for example, an IPC can relate the sequence information obtained for a maternal test sample to the sequence information obtained from a set of qualified samples that were sequenced at a different time.

Similarly, in the case of segment analysis, an IPC can relate the sequence information obtained from a subject for particular segment(s) to the sequence obtained from a set of qualified samples (of similar sequences) that were sequenced at a different time. In certain embodiments an IPC can relate the sequence information obtained from a subject for particular cancer-related loci to the sequence information obtained from a set of qualified samples (*e.g.*, from a known amplification/deletion, and the like).

In addition, IPCs can be used as markers to track sample(s) through the sequencing process. IPCs can also provide a qualitative positive sequence dose value, *e.g.,* NCV, for one or more aneuploidies of chromosomes of interest, *e.g.,* trisomy 21, trisomy 13, trisomy 18 to provide proper interpretation, and to ensure the dependability and accuracy of the data. In certain embodiments IPCs can be created to comprise nucleic acids from male and female genomes to provide doses for chromosomes X and Y in a maternal sample to determine whether the fetus is male.

The type and the number of in-process controls depends on the type or nature of the test needed. For example, for a test requiring the sequencing of DNA from a sample comprising a mixture of genomes to determine whether a chromosomal aneuploidy exists, the in-process control can comprise DNA obtained from a sample known comprising the same chromosomal aneuploidy that is being tested. In some embodiments, the IPC includes DNA from a sample known to comprise an aneuploidy of a chromosome of interest. For example, the IPC for a test to determine the presence or absence of a fetal trisomy, *e.g.*, trisomy 21, in a maternal sample comprises DNA obtained from an individual with trisomy 21. In some embodiments, the IPC comprises a mixture of DNA obtained from two or more individuals with different aneuploidies. For example, for a test to determine the presence or absence of trisomy 13, trisomy 18, trisomy 21, and monosomy X, the IPC comprises a combination of DNA samples obtained from pregnant women each carrying a fetus with one of the trisomies being tested. In addition to complete chromosomal aneuploidies, IPCs can be created to provide positive controls for tests to determine the presence or absence of partial aneuploidies.

An IPC that serves as the control for detecting a single aneuploidy can be created using a mixture of cellular genomic DNA obtained from a two subjects one being the contributor of the aneuploid genome. For example, an IPC that is created as a control for a test to determine a fetal trisomy, *e.g.*, trisomy 21, can be created by combining genomic DNA from a male or female subject carrying the trisomic chromosome with genomic DNA with a female subject known not to carry the trisomic chromosome. Genomic DNA can be extracted from cells of both subjects, and sheared to provide fragments of between about 100 - 400 bp, between about 150-350 bp, or between about 200-300 bp to simulate the circulating cfDNA fragments in maternal samples. The proportion of fragmented DNA from the subject carrying the aneuploidy, e.g., trisomy 21, is chosen to simulate the proportion of circulating fetal cfDNA found in maternal samples to provide an IPC comprising a mixture of fragmented DNA comprising about 5%, about 10%, about 15%, about 20%, about 25%, about 30%, of DNA from the subject carrying the aneuploidy. The IPC can comprise DNA from different subjects each carrying a different aneuploidy. For example, the IPC can comprise about 80% of the unaffected female DNA, and the remaining 20% can be DNA from three different subjects each carrying a trisomic chromosome 21, a trisomic chromosome 13, and a trisomic chromosome 18. The mixture of fragmented DNA is prepared for sequencing. Processing of the mixture of fragmented DNA can comprise preparing a sequencing library, which can be sequenced using any massively parallel methods in singleplex or multiplex fashion. Stock solutions of the genomic IPC can be stored and used in multiple diagnostic tests.

Alternatively the IPC can be created using cfDNA obtained from a mother known to carry a fetus with a known chromosomal aneuploidy. For example, cfDNA can be obtained from a pregnant woman carrying a fetus with trisomy 21. The cfDNA is extracted from the maternal sample, and cloned into a bacterial vector and grown in bacteria to provide an ongoing source of the IPC. The DNA can be extracted from the bacterial vector using restriction enzymes. Alternatively, the cloned cfDNA can be amplified by, *e.g., PCR.* The IPC DNA can be processed for sequencing in the same runs as the cfDNA from the test samples that are to be analyzed for the presence or absence of chromosomal aneuploidies.

While the creation of IPCs is described above with respect to trisomies, it will be appreciated that IPCs can be created to reflect other partial aneuploidies including for example, various segment amplification and/or deletions. Thus, for example, where various cancers are known to be associated with particular amplifications (*e.g.*, breast cancer associated with 20Q13) IPCs can be created that incorporate those known amplifications.

## Sequencing Methods

As indicated above, the prepared samples (*e.g.*, Sequencing Libraries) are sequenced as part of the procedure for identifying copy number variation(s). Any of a number of sequencing technologies can be utilized.

Some sequencing technologies are available commercially, such as the sequencing-by-hybridization platform from Affymetrix Inc. (Sunnyvale, CA) and the sequencing-by-synthesis platforms from 454 Life Sciences (Bradford, CT), Illumina/Solexa (Hayward, CA) and Helicos Biosciences (Cambridge, MA), and the sequencing-by-ligation platform from Applied Biosystems (Foster City, CA), as described below. In addition to the single molecule sequencing performed using sequencing-by-synthesis of Helicos Biosciences, other single molecule sequencing technologies include, but are not limited to, the SMRT™ technology of Pacific Biosciences, the ION TORRENT™ technology, and nanopore sequencing developed for example, by Oxford Nanopore Technologies.

While the automated Sanger method is considered as a 'first generation' technology, Sanger sequencing including the automated Sanger sequencing, can also be employed in the methods described herein. Additional suitable sequencing methods include, but are not limited to nucleic acid imaging technologies, *e.g.*, atomic force microscopy (AFM) or transmission electron microscopy (TEM). Illustrative sequencing technologies are described in greater detail below.

In one illustrative, but non-limiting, embodiment, the methods described herein comprise obtaining sequence information for the nucleic acids in a test sample, *e.g.,* cfDNA in a maternal sample, cfDNA or cellular DNA in a subject being screened for a cancer, and the like, using single molecule sequencing technology of the Helicos True Single Molecule Sequencing (tSMS) technology (*e.g.* as described in Harris T.D. et al., Science 320:106-109 [2008]). In the tSMS technique, a DNA sample is cleaved into strands of approximately 100 to 200 nucleotides, and a polyA sequence is added to the 3' end of each DNA strand. Each strand is labeled by the addition of a fluorescently labeled adenosine nucleotide. The DNA strands are then hybridized to a flow cell, which contains millions of oligo-T capture sites that are immobilized to the flow cell surface. In certain embodiments the templates can be at a density of about 100 million templates/cm$^2$. The flow cell is then loaded into an instrument, *e.g.*, HeliScope™ sequencer, and a laser illuminates the surface of the flow cell, revealing the position of each template. A CCD camera can map the position of the templates on the flow cell surface. The template fluorescent label is then cleaved and washed away. The sequencing reaction begins by introducing a DNA polymerase and a fluorescently labeled nucleotide. The oligo-T nucleic acid serves as a primer. The polymerase incorporates the labeled nucleotides to the primer in a template directed manner. The polymerase and unincorporated nucleotides are removed. The templates that have directed incorporation of the fluorescently labeled nucleotide are discerned by imaging the flow cell surface. After imaging, a cleavage step removes the fluorescent label, and the process is repeated with other fluorescently labeled nucleotides until the desired read length is achieved. Sequence information is collected with each nucleotide addition step. Whole genome sequencing by single molecule sequencing technologies excludes or typically obviates PCR-based amplification in the preparation of the sequencing libraries, and the methods allow for direct measurement of the sample, rather than measurement of copies of that sample.

In another illustrative, but non-limiting embodiment, the methods described herein comprise obtaining sequence information for the nucleic acids in the test sample, *e.g.*, cfDNA in a maternal test sample, cfDNA or cellular DNA in a subject being screened for a cancer, and the like, using the 454 sequencing (Roche) (*e.g.* as described in Margulies, M. et al. Nature 437:376-380 [2005]). 454 sequencing typically involves two steps. In the first step, DNA is sheared into fragments of approximately 300-800 base pairs, and the fragments are blunt-ended. Oligonucleotide adaptors are then ligated to the ends of the fragments. The adaptors serve as primers for amplification and sequencing of the fragments. The fragments can be attached to DNA capture beads, *e.g.,* streptavidin-coated beads using, *e.g.,* Adaptor B, which contains 5'-biotin tag. The fragments attached to the beads are PCR amplified within droplets of an oil-water emulsion. The result is multiple copies of clonally amplified DNA fragments on each bead. In the second step, the beads are captured in wells (e.g., picoliter-sized wells). Pyrosequencing is performed on each DNA fragment in parallel. Addition of one or more nucleotides generates a light signal that is recorded by a CCD camera in a sequencing instrument. The signal strength is proportional to the number of nucleotides incorporated. Pyrosequencing makes use of pyrophosphate (PPi) which is released upon nucleotide addition. PPi is converted to ATP by ATP sulfurylase in the presence of adenosine 5' phosphosulfate. Luciferase uses ATP to convert luciferin to oxyluciferin, and this reaction generates light that is measured and analyzed.

In another illustrative, but non-limiting, embodiment, the methods described herein comprises obtaining sequence information for the nucleic acids in the test sample , *e.g.*, cfDNA in a maternal test sample, cfDNA or cellular DNA in a subject being screened for a cancer, and the like, using the SOLiD™ technology (Applied Biosystems). In SOLiD™ sequencing-by-ligation, genomic DNA is sheared into fragments, and adaptors are attached to the 5' and 3' ends of the fragments to generate a fragment library. Alternatively, internal adaptors can be introduced by ligating adaptors to the 5' and 3' ends of the fragments, circularizing the fragments, digesting the circularized fragment to generate an internal adaptor, and attaching adaptors to the 5' and 3' ends of the resulting fragments to generate a mate-paired library. Next, clonal bead populations are prepared in microreactors containing beads, primers, template, and PCR components. Following PCR, the templates are denatured and beads are enriched to separate the beads with extended templates. Templates on the selected beads are subjected to a 3' modification that permits bonding to a glass slide. The sequence can be determined by sequential hybridization and ligation of partially random oligonucleotides with a central determined base (or pair of bases) that is identified by a specific fluorophore. After a color is recorded, the ligated oligonucleotide is cleaved and removed and the process is then repeated.

In another illustrative, but non-limiting, embodiment, the methods described herein comprise obtaining sequence information for the nucleic acids in the test sample, *e.g.*, cfDNA in a maternal test sample, cfDNA or cellular DNA in a subject being screened for a cancer, and the like, using the single molecule, real-time (SMRT™) sequencing technology of Pacific Biosciences. In SMRT sequencing, the continuous incorporation of dye-labeled nucleotides is imaged during DNA synthesis. Single DNA polymerase molecules are attached to the bottom surface of individual zero-mode wavelength detectors (ZMW detectors) that obtain sequence information while phospholinked nucleotides are being incorporated into the growing primer strand. A ZMW detector comprises a confinement structure that enables observation of incorporation of a single nucleotide by DNA polymerase against a background of fluorescent nucleotides that rapidly diffuse in an out of the ZMW (*e.g.,* in microseconds). It typically takes several milliseconds to incorporate a nucleotide into a growing strand. During this time, the fluorescent label is excited and produces a fluorescent signal, and the fluorescent tag is cleaved off. Measurement of the corresponding fluorescence of the dye indicates which base was incorporated. The process is repeated to provide a sequence.

In another illustrative, but non-limiting embodiment, the methods described herein comprise obtaining sequence information for the nucleic acids in the test sample, *e.g.*, cfDNA in a maternal test sample, cfDNA or cellular DNA in a subject being screened for a cancer, and the like, using nanopore sequencing (*e.g.* as described in Soni GV and Meller A. Clin Chem 53: 1996-2001 [2007]). Nanopore sequencing DNA analysis techniques are developed by a number of companies, including, for example, Oxford Nanopore Technologies (Oxford, United Kingdom), Sequenom, NABsys, and the like. Nanopore sequencing is a single-molecule sequencing technology whereby a single molecule of DNA is sequenced directly as it passes through a nanopore. A nanopore is a small hole, typically of the order of 1 nanometer in diameter. Immersion of a nanopore in a conducting fluid and application of a potential (voltage) across it results in a slight electrical current due to conduction of ions through the nanopore. The amount of current that flows is sensitive to the size and shape of the nanopore. As a DNA molecule passes through a nanopore, each nucleotide on the DNA molecule obstructs the nanopore to a different degree, changing the magnitude of the current through the nanopore in different degrees. Thus, this change in the current as the DNA molecule passes through the nanopore provides a read of the DNA sequence.

In another illustrative, but non-limiting, embodiment, the methods described herein comprises obtaining sequence information for the nucleic acids in the test sample, *e.g.*, cfDNA in a maternal test sample, cfDNA or cellular DNA in a subject being screened for a cancer, and the like, using the chemical-sensitive field effect transistor (chemFET) array (*e.g.,* as described in U.S. Patent Application Publication No. 2009/0026082). In one example of this technique, DNA molecules can be placed into reaction chambers, and the template molecules can be hybridized to a sequencing primer bound to a polymerase. Incorporation of one or more triphosphates into a new nucleic acid strand at the 3' end of the sequencing primer can be discerned as a change in current by a chemFET. An array can have multiple chemFET sensors. In another example, single nucleic acids can be attached to beads, and the nucleic acids can be amplified on the bead, and the individual beads can be transferred to individual reaction chambers on a chemFET array, with each chamber having a chemFET sensor, and the nucleic acids can be sequenced.

In another embodiment, the present method comprises obtaining sequence information for the nucleic acids in the test sample, *e.g.*, cfDNA in a maternal test sample, using the Halcyon Molecular's technology, which uses transmission electron microscopy (TEM). The method, termed Individual Molecule Placement Rapid Nano Transfer (IMPRNT), comprises utilizing single atom resolution transmission electron microscope imaging of high-molecular weight (150kb or greater) DNA selectively labeled with heavy atom markers and arranging these molecules on ultra-thin films in ultra-dense (3nm strand-to-strand) parallel arrays with consistent base-to-base spacing. The electron microscope is used to image the molecules on the films to determine the position of the heavy atom markers and to extract base sequence information from the DNA. The method is further described in PCT patent publication WO 2009/046445. The method allows for sequencing complete human genomes in less than ten minutes.

In another embodiment, the DNA sequencing technology is the Ion Torrent single molecule sequencing, which pairs semiconductor technology with a simple sequencing chemistry to directly translate chemically encoded information (A, C, G, T) into digital information (0, 1) on a semiconductor chip. In nature, when a nucleotide is incorporated into a strand of DNA by a polymerase, a hydrogen ion is released as a byproduct. Ion Torrent uses a high-density array of micro-machined wells to perform this biochemical process in a massively parallel way. Each well holds a different DNA molecule. Beneath the wells is an ion-sensitive layer and beneath that an ion sensor. When a nucleotide, for example a C, is added to a DNA template and is then incorporated into a strand of DNA, a hydrogen ion will be released. The charge from that ion will change the pH of the solution, which can be detected by Ion Torrent's ion sensor. The sequencer-essentially the world's smallest solid-state pH meter-calls the base, going directly from chemical information to digital information. The Ion personal Genome Machine (PGM™) sequencer then sequentially floods the chip with one nucleotide after another. If the next nucleotide that floods the chip is not a match. No voltage change will be recorded and no base will be called. If there are two identical bases on the DNA strand, the voltage will be double, and the chip will record two identical bases called. Direct detection allows recordation of nucleotide incorporation in seconds.

In another embodiment, the present method comprises obtaining sequence information for the nucleic acids in the test sample, *e.g.*, cfDNA in a maternal test sample, using sequencing by hybridization. Sequencing-by-hybridization comprises contacting the plurality of polynucleotide sequences with a plurality of polynucleotide probes, wherein each of the plurality of polynucleotide probes can be optionally tethered to a substrate. The substrate might be flat surface comprising an array of known nucleotide sequences. The pattern of hybridization to the array can be used to determine the polynucleotide sequences present in the sample. In other embodiments, each probe is tethered to a bead, *e.g.,* a magnetic bead or the like. Hybridization to the beads can be determined and used to identify the plurality of polynucleotide sequences within the sample.

In another embodiment, the present method comprises obtaining sequence information for the nucleic acids in the test sample , *e.g.,* cfDNA in a maternal test sample, by massively parallel sequencing of millions of DNA fragments using Illumina's sequencing-by-synthesis and reversible terminator-based sequencing chemistry *(e.g.* as described in Bentley et al., Nature 6:53-59 [2009]). Template DNA can be genomic DNA, *e.g.,* cfDNA. In some embodiments, genomic DNA from isolated cells is used as the template, and it is fragmented into lengths of several hundred base pairs. In other embodiments, cfDNA is used as the template, and fragmentation is not required as cfDNA exists as short fragments. For example fetal cfDNA circulates in the bloodstream as fragments approximately 170 base pairs (bp) in length (Fan et al., Clin Chem 56:1279-1286 [2010]), and no fragmentation of the DNA is required prior to sequencing. Illumina's sequencing technology relies on the attachment of fragmented genomic DNA to a planar, optically transparent surface on which oligonucleotide anchors are bound. Template DNA is end-repaired to generate 5'-phosphorylated blunt ends, and the polymerase activity of Klenow fragment is used to add a single A base to the 3' end of the blunt phosphorylated DNA fragments. This addition prepares the DNA fragments for ligation to oligonucleotide adapters, which have an overhang of a single T base at their 3' end to increase ligation efficiency. The adapter oligonucleotides are complementary to the flow-cell anchors. Under limiting-dilution conditions, adapter-modified, single-stranded template DNA is added to the flow cell and immobilized by hybridization to the anchors. Attached DNA fragments are extended and bridge amplified to create an ultra-high density sequencing flow cell with hundreds of millions of clusters, each containing ~1,000 copies of the same template. In one embodiment, the randomly fragmented genomic DNA, e.g., cfDNA, is amplified using PCR before it is subjected to cluster amplification. Alternatively, an amplification-free genomic library preparation is used, and the randomly fragmented genomic DNA, *e.g.,* cfDNA is enriched using the cluster amplification alone (Kozarewa et al., Nature Methods 6:291-295 [2009]). The templates are sequenced using a robust four-color DNA sequencing-by-synthesis technology that employs reversible terminators with removable fluorescent dyes. High-sensitivity fluorescence detection is achieved using laser excitation and total internal reflection optics. Short sequence reads of about 20-40 bp, *e.g.,* 36 bp, are aligned against a repeat-masked reference genome and unique mapping of the short sequence reads to the reference genome are identified using specially developed data analysis pipeline software. Non-repeat-masked reference genomes can also be used. Whether repeat-masked or non-repeat-masked reference genomes are used, only reads that map uniquely to the reference genome are counted. After completion of the first read, the templates can be regenerated *in situ* to enable a second read from the opposite end of the fragments. Thus, either single-end or paired end sequencing of the DNA fragments can be used. Partial sequencing of DNA fragments present in the sample is performed, and sequence tags comprising reads of predetermined length, *e.g.,* 36 bp, are mapped to a known reference genome are counted. In one embodiment, the reference genome sequence is the NCBI36/hg18 sequence, which is available on the world wide web at genome.ucsc.edu/cgi-bin/hgGateway?org=Human&db=hg18&hgsid=166260105). Alternatively, the reference genome sequence is the GRCh37/hg19, which is available on the world wide web at genome.ucsc.edu/cgi-bin/hgGateway. Other sources of public sequence information include GenBank, dbEST, dbSTS, EMBL (the European Molecular Biology Laboratory), and the DDBJ (the DNA Databank of Japan). A number of computer algorithms are available for aligning sequences, including without limitation BLAST (Altschul *et al.,* 1990), BLITZ (MPsrch) (Sturrock & Collins, 1993), FASTA (Person & Lipman, 1988), BOWTIE (Langmead et al., Genome Biology 10:R25.1-R25.10 [2009]), or ELAND (Illumina, Inc., San Diego, CA, USA). In one embodiment, one end of the clonally expanded copies of the plasma cfDNA molecules is sequenced and processed by bioinformatic alignment analysis for the Illumina Genome Analyzer, which uses the Efficient Large-Scale Alignment of Nucleotide Databases (ELAND) software.

In some embodiments of the methods described herein, the mapped sequence tags comprise sequence reads of about 20bp, about 25bp, about 30bp, about 35bp, about 40bp, about 45bp, about 50bp, about 55bp, about 60bp, about 65bp, about 70bp, about 75bp, about 80bp, about 85bp, about90bp, about 95bp, about 100bp, about 110bp, about 120bp, about 130, about 140bp, about 150bp, about 200bp, about 250bp, about 300bp, about 350bp, about 400bp, about 450bp, or about 500bp. It is expected that technological advances will enable single-end reads of greater than 500bp enabling for reads of greater than about 1000bp when paired end reads are generated. In one embodiment, the mapped sequence tags comprise sequence reads that are 36bp. Mapping of the sequence tags is achieved by comparing the sequence of the tag with the sequence of the reference to determine the chromosomal origin of the sequenced nucleic acid (*e.g.* cfDNA) molecule, and specific genetic sequence information is not needed. A small degree of mismatch (0-2 mismatches per sequence tag) may be allowed to account for minor polymorphisms that may exist between the reference genome and the genomes in the mixed sample.

A plurality of sequence tags are typically obtained per sample. In some embodiments, at least about $3 \times 10^6$ sequence tags, at least about $5 \times 10^6$ sequence tags, at least about $8 \times 10^6$ sequence tags, at least about $10 \times 10^6$ sequence tags, at least about $15 \times 10^6$ sequence tags, at least about $20 \times 10^6$ sequence tags, at least about $30 \times 10^6$

sequence tags, at least about $40 \times 10^6$ sequence tags, or at least about $50 \times 10^6$ sequence tags comprising between 20 and 40bp reads, *e.g.,* 36bp, are obtained from mapping the reads to the reference genome per sample. In one embodiment, all the sequence reads are mapped to all regions of the reference genome. In one embodiment, the tags that have been mapped to all regions, *e.g.,* all chromosomes, of the reference genome are counted, and the CNV, *i.e.,* the over- or under-representation of a sequence of interest, *e.g.,* a chromosome or portion thereof, in the mixed DNA sample is determined. The method does not require differentiation between the two genomes.

The accuracy required for correctly determining whether a CNV, *e.g.,* aneuploidy, is present or absent in a sample, is predicated on the variation of the number of sequence tags that map to the reference genome among samples within a sequencing run (inter-chromosomal variability), and the variation of the number of sequence tags that map to the reference genome in different sequencing runs (inter-sequencing variability). For example, the variations can be particularly pronounced for tags that map to GC-rich or GC-poor reference sequences. Other variations can result from using different protocols for the extraction and purification of the nucleic acids, the preparation of the sequencing libraries, and the use of different sequencing platforms. The present method uses sequence doses (chromosome doses, or segment doses) based on the knowledge of normalizing sequences (normalizing chromosome sequences or normalizing segment sequences), to intrinsically account for the accrued variability stemming from interchromosomal (intra-run), and inter-sequencing (inter-run) and platform-dependent variability. Chromosome doses are based on the knowledge of a normalizing chromosome sequence, which can be composed of a single chromosome, or of two or more chromosomes selected from chromosomes 1-22, X, and Y. Alternatively, normalizing chromosome sequences can be composed of a single chromosome segment, or of two or more segments of one chromosome or of two or more chromosomes. Segment doses are based on the knowledge of a normalizing segment sequence, which can be composed of a single segment of any one chromosome, or of two or more segments of any two or more of chromosomes 1-22, X, and Y.

**CNV and Prenatal Diagnoses**

Cell-free fetal DNA and RNA circulating in maternal blood can be used for the early non-invasive prenatal diagnosis (NIPD) of an increasing number of genetic conditions, both for pregnancy management and to aid reproductive decision-making. The presence of cell-free DNA circulating in the bloodstream has been known for over 50 years. More recently, presence of small amounts of circulating fetal DNA was discovered in the maternal bloodstream during pregnancy (Lo et al., Lancet 350:485-487 [1997]). Thought to originate from dying placental cells, cell-free fetal DNA (cfDNA) has been shown to consists of short fragments typically fewer than 200 bp in length Chan et al., Clin Chem 50:88-92 [2004]), which can be discerned as early as 4 weeks gestation (Illanes et al., Early Human Dev 83:563-566 [2007]), and known to be cleared from the maternal circulation within hours of delivery (Lo et al., Am J Hum Genet 64:218-224 [1999]). In addition to cfDNA, fragments of cell-free fetal RNA (cfRNA) can also be discerned in the maternal bloodstream, originating from genes that are transcribed in the fetus or placenta. The extraction and subsequent analysis of these fetal genetic elements from a maternal blood sample offers novel opportunities for NIPD.

The present method is a polymorphism-independent method that for use in NIPD and that does not require that the fetal cfDNA be distinguished from the maternal cfDNA to enable the determination of a fetal aneuploidy. In some embodiments, the aneuploidy is a complete chromosomal trisomy or monosomy, or a partial trisomy or monosomy. Partial aneuploidies are caused by loss or gain of part of a chromosome, and encompass chromosomal imbalances resulting from unbalanced translocations, unbalanced inversions, deletions and insertions. By far, the most common known aneuploidy compatible with life is trisomy 21, *i.e., Down* Syndrome (DS), which is caused by the presence of part or all of chromosome 21. Rarely, DS can be caused by an inherited or sporadic defect whereby an extra copy of all or part of chromosome 21 becomes attached to another chromosome (usually chromosome 14) to form a single aberrant chromosome. DS is associated with intellectual impairment, severe learning difficulties and excess mortality caused by long-term health problems such as heart disease. Other aneuploidies with known clinical significance include Edward syndrome (trisomy 18) and Patau Syndrome (trisomy 13), which are frequently fatal within the first few months of life. Abnormalities associated with the number of sex chromosomes are also known and include monosomy X, *e.g., Turner* syndrome (XO), and triple X syndrome (XXX) in female births and Kleinefelter syndrome (XXY) and XYY syndrome in male births, which are all associated with various phenotypes including sterility and reduction in intellectual skills. Monosomy X [45, X] is a common cause of early pregnancy loss accounting for about 7% of spontaneous abortions. Based on the liveborn frequency of 45,X (also called Turner syndrome) of 1-2/10,000, it is estimated that less than 1% of 45,X conceptions will survive to term. About 30% of Turners syndrome patients are mosaic with both a 45,X cell line and either a 46,XX cell line or one containing a rearranged X chromosome (Hook and Warburton 1983). The phenotype in a liveborn infant is relatively mild considering the high embryonic lethality and it has been hypothesized that possibly all liveborn females with Turner syndrome carry a cell line containing two sex chromosomes. Monosomy X can occur in females as 45,X or as 45,X/46XX, and in males as 45,X/46XY. Autosomal monosomies in human are generally suggested to be incompatible with life; however, there is quite a number of cytogenetic reports describing full monosomy of one chromosome 21 in live born children (Vosranova let al., Molecular Cytogen. 1:13 [2008]; Joosten et al., Prenatal Diagn.

17:271-5 [1997]. The method described herein can be used to diagnose these and other chromosomal abnormalities prenatally.

According to some embodiments the methods disclosed herein can determine the presence or absence of chromosomal trisomies of any one of chromosomes 1-22, X and Y. Examples of chromosomal trisomies that can be detected according to the present method include without limitation trisomy 21 (T21; Down Syndrome), trisomy 18 (T18; Edward's Syndrome), trisomy 16 (T16), trisomy 20 (T20), trisomy 22 (T22; Cat Eye Syndrome), trisomy 15 (T15; Prader Willi Syndrome), trisomy 13 (T13; Patau Syndrome), trisomy 8 (T8; Warkany Syndrome), trisomy 9, and the XXY (Kleinefelter Syndrome), XYY, or XXX trisomies. Complete trisomies of other autosomes existing in a non-mosaic state are lethal, but can be compatible with life when present in a mosaic state. It will be appreciated that various complete trisomies, whether existing in a mosaic or non-mosaic state, and partial trisomies can be determined in fetal cfDNA according to the teachings provided herein.

Non-limiting examples of partial trisomies that can be determined by the present method include, but are not limited to, partial trisomy 1q32-44, trisomy 9 p, trisomy 4 mosaicism, trisomy 17p, partial trisomy 4q26-qter, partial 2p trisomy, partial trisomy 1q, and/or partial trisomy 6p/monosomy 6q.

The methods disclosed herein can be also used to determine chromosomal monosomy X, chromosomal monosomy 21, and partial monosomies such as, monosomy 13, monosomy 15, monosomy 16, monosomy 21, and monosomy 22, which are known to be involved in pregnancy miscarriage. Partial monosomy of chromosomes typically involved in complete aneuploidy can also be determined by the method described herein. Non-limiting examples of deletion syndromes that can be determined according to the present method include syndromes caused by partial deletions of chromosomes. Examples of partial deletions that can be determined according to the methods described herein include without limitation partial deletions of chromosomes 1, 4, 5, 7, 11, 18, 15, 13, 17, 22 and 10, which are described in the following.

1q21.1 deletion syndrome or 1q21.1 (recurrent) microdeletion is a rare aberration of chromosome 1. Next to the deletion syndrome, there is also a 1q21.1 duplication syndrome. While there is a part of the DNA missing with the deletion syndrome on a particular spot, there are two or three copies of a similar part of the DNA on the same spot with the duplication syndrome. Literature refers to both the deletion and the duplication as the 1q21.1 copy-number variations (CNV). The 1q21.1 deletion can be associated with the TAR Syndrome (Thrombocytopenia with Absent radius).

Wolf-Hirschhorn syndrome (WHS) (OMIN #194190) is a contiguous gene deletion syndrome associated with a hemizygous deletion of chromosome 4p16.3. Wolf-Hirschhorn syndrome is a congenital malformation syndrome characterized by pre- and postnatal growth deficiency, developmental disability of variable degree, characteristic craniofacial features ('Greek warrior helmet' appearance of the nose, high forehead, prominent glabella, hypertelorism, high-arched eyebrows, protruding eyes, epicanthal folds, short philtrum, distinct mouth with downturned corners, and micrognathia), and a seizure disorder.

Partial deletion of chromosome 5, also known as 5p- or 5p minus, and named Cris du Chat syndrome (OMIN#123450), is caused by a deletion of the short arm (p arm) of chromosome 5 (5p15.3-p15.2). Infants with this condition often have a high-pitched cry that sounds like that of a cat. The disorder is characterized by intellectual disability and delayed development, small head size (microcephaly), low birth weight, and weak muscle tone (hypotonia) in infancy, distinctive facial features and possibly heart defects.

Williams-Beuren Syndrome also known as chromosome 7ql 1.23 deletion syndrome (OMIN 194050) is a contiguous gene deletion syndrome resulting in a multisystem disorder caused by hemizygous deletion of 1.5 to 1.8 Mb on chromosome 7q11.23, which contains approximately 28 genes.

Jacobsen Syndrome, also known as 11q deletion disorder, is a rare congenital disorder resulting from deletion of a terminal region of chromosome 11 that includes band 11q24.1. It can cause intellectual disabilities, a distinctive facial appearance, and a variety of physical problems including heart defects and a bleeding disorder.

Partial monosomy of chromosome 18, known as monosomy 18p is a rare chromosomal disorder in which all or part of the short arm (p) of chromosome 18 is deleted (monosomic). The disorder is typically characterized by short stature, variable degrees of mental retardation, speech delays, malformations of the skull and facial (craniofacial) region, and/or additional physical abnormalities. Associated craniofacial defects may vary greatly in range and severity from case to case.

Conditions caused by changes in the structure or number of copies of chromosome 15 include Angelman Syndrome and Prader-Willi Syndrome, which involve a loss of gene activity in the same part of chromosome 15, the 15q11-q13 region. It will be appreciated that several translocations and microdeletions can be asymptomatic in the carrier parent, yet can cause a major genetic disease in the offspring. For example, a healthy mother who carries the 15q11-q13 microdeletion can give birth to a child with Angelman syndrome, a severe neurodegenerative disorder. Thus, the methods, apparatus and systems described herein can be used to identify such a partial deletion and other deletions in the fetus.

Partial monosomy 13q is a rare chromosomal disorder that results when a piece of the long arm (q) of chromosome 13 is missing (monosomic). Infants born with partial monosomy 13q may exhibit low birth weight, malformations of the head and face (craniofacial region), skeletal abnormalities (especially of the hands and feet), and other physical abnormalities. Mental retardation is characteristic of this condition. The mortality rate during infancy is high among individuals born with this disorder. Almost all cases of partial monosomy 13q occur randomly for no apparent reason (sporadic).

Smith-Magenis syndrome (SMS - OMIM #182290) is caused by a deletion, or loss of genetic material, on one copy of chromosome 17. This well-known syndrome is associated with developmental delay, mental retardation, congenital anomalies such as heart and kidney defects, and neurobehavioral abnormalities such as severe sleep disturbances and self-injurious behavior. Smith-Magenis syndrome (SMS) is caused in most cases (90%) by a 3.7-Mb interstitial deletion in chromosome 17p11.2.

22q11.2 deletion syndrome, also known as DiGeorge syndrome, is a syndrome caused by the deletion of a small piece of chromosome 22. The deletion (22 q11.2) occurs near the middle of the chromosome on the long arm of one of the pair of chromosome. The features of this syndrome vary widely, even among members of the same family, and affect many parts of the body. Characteristic signs and symptoms may include birth defects such as congenital heart disease, defects in the palate, most commonly related to neuromuscular problems with closure (velo-pharyngeal insufficiency), learning disabilities, mild differences in facial features, and recurrent infections. Microdeletions in chromosomal region 22q11.2 are associated with a 20 to 30-fold increased risk of schizophrenia.

Deletions on the short arm of chromosome 10 are associated with a DiGeorge Syndrome like phenotype. Partial monosomy of chromosome 10p is rare but has been observed in a portion of patients showing features of the DiGeorge Syndrome.

In one embodiment, the methods, apparatus, and systems described herein is used to determine partial monosomies including but not limited to partial monosomy of chromosomes 1, 4, 5, 7, 11, 18, 15, 13, 17, 22 and 10, e.g., partial monosomy 1q21.11, partial monosomy 4p16.3, partial monosomy 5p15.3-p15.2, partial monosomy 7q11.23, partial monosomy 11q24.1, partial monosomy 18p, partial monosomy of chromosome 15 (15q11-q13), partial monosomy 13q, partial monosomy 17p11.2, partial monosomy of chromosome 22 (22q11.2), and partial monosomy 10p can also be determined using the method.

Other partial monosomies that can be determined according to the methods described herein include unbalanced translocation t(8;11)(p23.2;p15.5); 11q23 microdeletion; 17p11.2 deletion; 22q13.3 deletion; Xp22.3 microdeletion; 10p14 deletion; 20p microdeletion, [del(22)(q11.2q11.23)], 7q11.23 and 7q36 deletions; 1p36 deletion; 2p microdeletion; neurofibromatosis type 1 (17q11.2 microdeletion), Yq deletion ; 4p16.3 microdeletion; 1p36.2 microdeletion; 11q14 deletion; 19q13.2 microdeletion; Rubinstein-Taybi (16 p13.3 microdeletion); 7p21 microdeletion; Miller-Dieker syndrome (17p13.3); and 2q37 microdeletion. Partial deletions can be small deletions of part of a chromosome, or they can be microdeletions of a chromosome where the deletion of a single gene can occur.

Several duplication syndromes caused by the duplication of part of chromosome arms have been identified (see OMIN [Online Mendelian Inheritance in Man viewed online at ncbi.nlm.nih.gov/omim]). In one embodiment, the present method can be used to determine the presence or absence of duplications and/or multiplications of segments of any one of chromosomes 1-22, X and Y. Non-limiting examples of duplications syndromes that can be determined according to the present method include duplications of part of chromosomes 8, 15, 12, and 17, which are described in the following.

8p23.1 duplication syndrome is a rare genetic disorder caused by a duplication of a region from human chromosome 8. This duplication syndrome has an estimated prevalence of 1 in 64,000 births and is the reciprocal of the 8p23.1 deletion syndrome. The 8p23.1 duplication is associated with a variable phenotype including one or more of speech delay, developmental delay, mild dysmorphism, with prominent forehead and arched eyebrows, and congenital heart disease (CHD).

Chromosome 15q Duplication Syndrome (Dup15q) is a clinically identifiable syndrome which results from duplications of chromosome 15q11-13.1 Babies with Dup15q usually have hypotonia (poor muscle tone), growth retardation; they may be born with a cleft lip and/or palate or malformations of the heart, kidneys or other organs; they show some degree of cognitive delay/disability (mental retardation), speech and language delays, and sensory processing disorders.

Pallister Killian syndrome is a result of extra #12 chromosome material. There is usually a mixture of cells (mosaicism), some with extra #12 material, and some that are normal (46 chromosomes without the extra #12 material). Babies with this syndrome have many problems including severe mental retardation, poor muscle tone, "coarse" facial features, and a prominent forehead. They tend to have a very thin upper lip with a thicker lower lip and a short nose. Other health problems include seizures, poor feeding, stiff joints, cataracts in adulthood, hearing loss, and heart defects. Persons with Pallister Killian have a shortened lifespan.

Individuals with the genetic condition designated as dup(17)(p11.2p11.2) or dup 17p carry extra genetic information (known as a duplication) on the short arm of chromosome 17. Duplication of chromosome 17p11.2 underlies Potocki-Lupski syndrome (PTLS), which is a newly recognized genetic condition with only a few dozen cases reported in the medical literature. Patients who have this duplication often have low muscle tone, poor feeding, and failure to thrive during infancy, and also present with delayed development of motor and verbal milestones. Many individuals who have PTLS have difficulty with articulation and language processing. In addition, patients may have behavioral characteristics similar to those seen in persons with autism or autism-spectrum disorders. Individuals with PTLS may have heart defects and sleep apnea. A duplication of a large region in chromosome 17p12 that includes the gene PMP22 is known to cause Charcot-Marie Tooth disease.

CNV have been associated with stillbirths. However, due to inherent limitations of conventional cytogenetics, the contribution of CNV to stillbirth is thought to be underrepresented (Harris et al., Prenatal Diagn 31:932-944 [2011]). As is shown in the examples and described elsewhere herein, the present method is capable of determining the presence of partial aneuploidies, e.g., deletions and multiplications of chromosome segments, and can be used to identify and determine the presence or absence of CNV that are associated with stillbirths.

## Apparatus and systems for determining CNV

Analysis of the sequencing data and the diagnosis derived therefrom are performed using various computer executed algorithms and programs. Therefore, certain embodiments employ processes involving data stored in or transferred through one or more computer systems or other processing systems. Embodiments disclosed herein also relate to apparatus for performing these operations. This apparatus may be specially constructed for the required purposes, or it may be a general-purpose computer (or a group of computers) selectively activated or reconfigured by a computer program and/or data structure stored in the computer. In some embodiments, a group of processors performs some or all of the recited analytical operations collaboratively (*e.g.*, via a network or cloud computing) and/or in parallel. A processor or group of processors for performing the methods described herein may be of various types including microcontrollers and microprocessors such as programmable devices (*e.g.*, CPLDs and FPGAs) and non-programmable devices such as gate array ASICs or general purpose microprocessors.

In addition, certain embodiments relate to tangible and/or non-transitory computer readable media or computer program products that include program instructions and/or data (including data structures) for performing various computer-implemented operations. Examples of computer-readable media include, but are not limited to, semiconductor memory devices, magnetic media such as disk drives, magnetic tape, optical media such as CDs, magneto-optical media, and hardware devices that are specially configured to store and perform program instructions, such as read-only memory devices (ROM) and random access memory (RAM). The computer readable media may be directly controlled by an end user or the media may be indirectly controlled by the end user. Examples of directly controlled media include the media located at a user facility and/or media that are not shared with other entities. Examples of indirectly controlled media include media that is indirectly accessible to the user via an external network and/or via a service providing shared resources such as the "cloud." Examples of program instructions include both machine code, such as produced by a compiler, and files containing higher level code that may be executed by the computer using an interpreter.

In various embodiments, the data or information employed in the disclosed methods and apparatus is provided in an electronic format. Such data or information may include reads and tags derived from a nucleic acid sample, counts or densities of such tags that align with particular regions of a reference sequence (*e.g.*, that align to a chromosome or chromosome segment), reference sequences (including reference sequences providing solely or primarily polymorphisms), chromosome and segment doses, calls such as aneuploidy calls, normalized chromosome and segment values, pairs of chromosomes or segments and corresponding normalizing chromosomes or segments, counseling recommendations, diagnoses, and the like. As used herein, data or other information provided in electronic format is available for storage on a machine and transmission between machines. Conventionally, data in electronic format is provided digitally and may be stored as bits and/or bytes in various data structures, lists, databases, *etc.* The data may be embodied electronically, optically, *etc.*

One embodiment provides a computer program product for generating an output indicating the presence or absence of an aneuploidy, *e.g.*, a fetal aneuploidy or cancer, in a test sample. The computer product may contain instructions for performing any one or more of the above-described methods for determining a chromosomal anomaly. As explained, the computer product may include a non-transitory and/or tangible computer readable medium having a computer executable or compilable logic (*e.g.*, instructions) recorded thereon for enabling a processor to determine chromosome doses and, in some cases, whether a fetal aneuploidy is present or absent. In one example, the computer product comprises a computer readable medium having a computer executable or compilable logic (*e.g.,* instructions) recorded thereon for enabling a processor to diagnose a fetal aneuploidy comprising: a receiving procedure for receiving sequencing data from at least a portion of nucleic acid molecules from a maternal biological sample, wherein said sequencing data comprises a calculated chromosome and/or segment dose; computer assisted logic for analyzing a fetal aneuploidy from said received data; and an output procedure for generating an output indicating the presence, absence or kind of said fetal aneuploidy.

The sequence information from the sample under consideration may be mapped to chromosome reference sequences to identify a number of sequence tags for each of any one or more chromosomes of interest and to identify a number of sequence tags for a normalizing segment sequence for each of said any one or more chromosomes of interest. In various embodiments, the reference sequences are stored in a database such as a relational or object database, for example.

It should be understood that it is not practical, or even possible in most cases, for an unaided human being to perform the computational operations of the methods disclosed herein. For example, mapping a single 30 bp read from a sample to any one of the human chromosomes might require years of effort without the assistance of a computational apparatus. Of course, the problem is compounded because reliable aneuploidy calls generally require mapping thousands (*e.g.*, at least about 10,000) or even millions of reads to one or more chromosomes.

The methods disclosed herein can be performed using a system for evaluation of copy number of a genetic sequence of interest in a test sample. The system comprising: (a) a sequencer for receiving nucleic acids from the test sample providing nucleic acid sequence information from the sample; (b) a processor; and (c) one or more computer-readable storage media having stored thereon instructions for execution on said processor to evaluate the copy number of the Y chromosome in the test sample using a reference sequence of the Y chromosome filtered by a mask. The mask comprises bins of specific size on the reference sequence of the Y chromosome. The bins have more than a threshold number of training sequence tags aligned thereto. The training sequence tags comprise genomic reads from a first plurality of female individuals aligned to the reference sequence of the Y chromosome.

In some embodiments, the methods are instructed by a computer-readable medium having stored thereon computer-readable instructions for carrying out a method for identifying any CNV, *e.g.*, chromosomal or partial aneuploidies. Thus one embodiment provides a computer program product comprising one or more computer-readable non-transitory storage media having stored thereon computer-executable instructions that, when executed by one or more processors of a computer system, cause the computer system to implement a method for evaluation of copy number of the Y chromosome in a test sample comprising fetal and maternal cell-free nucleic acids. The method comprises: (a) providing, on the computer system, a training set comprising genomic reads measured from nucleic acid samples of a first plurality of female individuals; (b) aligning, by the computer system, at least about 100,000 genomic reads per individual of the training set to a reference sequence of the Y-chromosome, thereby providing training sequence tags comprising aligned genomic reads and their locations on the reference sequence of the Y chromosome; (c) dividing, by the computer system, the reference sequence of the Y chromosome into bins of a specific size; (d) determining, by the computer system, counts of training sequence tags located in each bin; (e) masking, by the computer system, bins that exceed a masking threshold, the masking threshold being based on the counts of training sequence tags in each bin, thereby providing a masked reference sequence of the Y chromosome for evaluation of copy number of the Y chromosome in a test sample comprising fetal and maternal cell-free nucleic acids.

In some embodiments, the instructions may further include automatically recording information pertinent to the method such as chromosome doses and the presence or absence of a fetal chromosomal aneuploidy in a patient medical record for a human subject providing the maternal test sample. The patient medical record may be maintained by, for example, a laboratory, physician's office, a hospital, a health maintenance organization, an insurance company, or a personal medical record website. Further, based on the results of the processor-implemented analysis, the method may further involve prescribing, initiating, and/or altering treatment of a human subject from whom the maternal test sample was taken. This may involve performing one or more additional tests or analyses on additional samples taken from the subject.

Disclosed methods can also be performed using a computer processing system which is adapted or configured to perform a method for identifying any CNV, *e.g.,* chromosomal or partial aneuploidies. One embodiment provides a computer processing system which is adapted or configured to perform a method as described herein. In one embodiment, the apparatus comprises a sequencing device adapted or configured for sequencing at least a portion of the nucleic acid molecules in a sample to obtain the type of sequence information described elsewhere herein. The apparatus may also include components for processing the sample. Such components are described elsewhere herein.

Sequence or other data, can be input into a computer or stored on a computer readable medium either directly or indirectly. In one embodiment, a computer system is directly coupled to a sequencing device that reads and/or analyzes sequences of nucleic acids from samples. Sequences or other information from such tools are provided via interface in the computer system. Alternatively, the sequences processed by system are provided from a sequence storage source such as a database or other repository. Once available to the processing apparatus, a memory device or mass storage device buffers or stores, at least temporarily, sequences of the nucleic acids. In addition, the memory device may store tag counts for various chromosomes or genomes, *etc.* The memory may also store various routines and/or programs for analyzing the presenting the sequence or mapped data. Such programs/routines may include programs for performing statistical analyses, *etc.*

In one example, a user provides a sample into a sequencing apparatus. Data is collected and/or analyzed by the sequencing apparatus which is connected to a computer. Software on the computer allows for data collection and/or analysis. Data can be stored, displayed (via a monitor or other similar device), and/or sent to another location. The computer may be connected to the internet which is used to transmit data to a handheld device utilized by a remote user (*e.g.,* a physician, scientist or analyst). It is understood that the data can be stored and/or analyzed prior to transmittal.

In some embodiments, raw data is collected and sent to a remote user or apparatus that will analyze and/or store the data. Transmittal can occur via the internet, but can also occur via satellite or other connection. Alternately, data can be stored on a computer-readable medium and the medium can be shipped to an end user *(e.g.,* via mail). The remote user can be in the same or a different geographical location including, but not limited to a building, city, state, country or continent.

In some embodiments, the methods also include collecting data regarding a plurality of polynucleotide sequences *(e.g.,* reads, tags and/or reference chromosome sequences) and sending the data to a computer or other computational system. For example, the computer can be connected to laboratory equipment, *e.g.,* a sample collection apparatus, a nucleotide amplification apparatus, a nucleotide sequencing apparatus, or a hybridization apparatus. The computer can then collect applicable data gathered by the laboratory device. The data can be stored on a computer at any step, *e.g.,* while collected in real time, prior to the sending, during or in conjunction with the sending, or following the sending. The data can be stored on a computer-readable medium that can be extracted from the computer. The data collected or stored can be transmitted from the computer to a remote location, *e.g.,* via a local network or a wide area network such as the internet. At the remote location various operations can be performed on the transmitted data as described below.

Among the types of electronically formatted data that may be stored, transmitted, analyzed, and/or manipulated in systems, apparatus, and methods disclosed herein are the following:

Reads obtained by sequencing nucleic acids in a test sample

Tags obtained by aligning reads to a reference genome or other reference sequence or sequences

The reference genome or sequence

Sequence tag density - Counts or numbers of tags for each of two or more regions (typically chromosomes or chromosome segments) of a reference genome or other reference sequences

Identities of normalizing chromosomes or chromosome segments for particular chromosomes or chromosome segments of interest

Doses for chromosomes or chromosome segments (or other regions) obtained from chromosomes or segments of interest and corresponding normalizing chromosomes or segments

Thresholds for calling chromosome doses as either affected, non-affected, or no call

The actual calls of chromosome doses

Diagnoses (clinical condition associated with the calls)

Recommendations for further tests derived from the calls and/or diagnoses

Treatment and/or monitoring plans derived from the calls and/or diagnoses

These various types of data may be obtained, stored transmitted, analyzed, and/or manipulated at one or more locations using distinct apparatus. The processing options span a wide spectrum. At one end of the spectrum, all or much of this information is stored and used at the location where the test sample is processed, *e.g.,* a doctor's office or other clinical setting. In other extreme, the sample is obtained at one location, it is processed and optionally sequenced at a different location, reads are aligned and calls are made at one or more different locations, and diagnoses, recommendations, and/or plans are prepared at still another location (which may be a location where the sample was obtained).

In various embodiments, the reads are generated with the sequencing apparatus and then transmitted to a remote site where they are processed to produce aneuploidy calls. At this remote location, as an example, the reads are aligned to a reference sequence to produce tags, which are counted and assigned to chromosomes or segments of interest. Also at the remote location, the counts are converted to doses using associated normalizing chromosomes or segments. Still further, at the remote location, the doses are used to generate aneuploidy calls.

Among the processing operations that may be employed at distinct locations are the following:

Sample collection

Sample processing preliminary to sequencing

Sequencing

Analyzing sequence data and deriving aneuploidy calls

Diagnosis

Reporting a diagnosis and/or a call to patient or health care provider

Developing a plan for further treatment, testing, and/or monitoring

Executing the plan

Counseling

Any one or more of these operations may be automated as described elsewhere herein. Typically, the sequencing and the analyzing of sequence data and deriving aneuploidy calls will be performed computationally. The other operations may be performed manually or automatically.

Examples of locations where sample collection may be performed include health practitioners' offices, clinics, patients' homes (where a sample collection tool or kit is provided), and mobile health care vehicles. Examples of locations where sample processing prior to sequencing may be performed include health practitioners' offices, clinics, patients' homes (where a sample processing apparatus or kit is provided), mobile health care vehicles, and facilities of aneuploidy analysis providers. Examples of locations where sequencing may be performed include health practitioners' offices, clinics, health practitioners' offices, clinics, patients' homes (where a sample sequencing apparatus and/or kit is provided), mobile health care vehicles, and facilities of aneuploidy analysis providers. The location where the sequencing takes place may be provided with a dedicated network connection for transmitting sequence data (typically reads) in an electronic format. Such connection may be wired or wireless and have and may be configured to send the data to a site where the data can be processed and/or aggregated prior to transmission to a processing site. Data aggregators can be maintained by health organizations such as Health Maintenance Organizations (HMOs).

The analyzing and/or deriving operations may be performed at any of the foregoing locations or alternatively at a further remote site dedicated to computation and/or the service of analyzing nucleic acid sequence data. Such locations include for example, clusters such as general purpose server farms, the facilities of an aneuploidy analysis service business, and the like. In some embodiments, the computational apparatus employed to perform the analysis is leased or rented. The computational resources may be part of an internet accessible collection of processors such as processing resources colloquially known as the cloud. In some cases, the computations are performed by a parallel or massively parallel group of processors that are affiliated or unaffiliated with one another. The processing may be accomplished using distributed processing such as cluster computing, grid computing, and the like. In such embodiments, a cluster or grid of computational resources collective form a super virtual computer composed of multiple processors or computers acting together to perform the analysis and/or derivation described herein. These technologies as well as more conventional supercomputers may be employed to process sequence data as described herein. Each is a form of parallel computing that relies on processors or computers. In the case of grid computing these processors (often whole computers) are connected by a network (private, public, or the Internet) by a conventional network protocol such as Ethernet. By contrast, a supercomputer has many processors connected by a local high-speed computer bus.

In certain embodiments, the diagnosis (*e.g.*, the fetus has Downs syndrome or the patient has a particular type of cancer) is generated at the same location as the analyzing operation. In other embodiments, it is performed at a different location. In some examples, reporting the diagnosis is performed at the location where the sample was taken, although this need not be the case. Examples of locations where the diagnosis can be generated or reported and/or where developing a plan is performed include health practitioners' offices, clinics, internet sites accessible by computers, and handheld devices such as cell phones, tablets, smart phones, *etc.* having a wired or wireless connection to a network. Examples of locations where counseling is performed include health practitioners' offices, clinics, internet sites accessible by computers, handheld devices, *etc.*

In some embodiments, the sample collection, sample processing, and sequencing operations are performed at a first location and the analyzing and deriving operation is performed at a second location. However, in some cases, the sample collection is collected at one location (*e.g.,* a health practitioner's office or clinic) and the sample processing and sequencing is performed at a different location that is optionally the same location where the analyzing and deriving take place.

In various embodiments, a sequence of the above-listed operations may be triggered by a user or entity initiating sample collection, sample processing and/or sequencing. After one or more these operations have begun execution the other operations may naturally follow. For example, the sequencing operation may cause reads to be automatically collected and sent to a processing apparatus which then conducts, often automatically and possibly without further user intervention, the sequence analysis and derivation of aneuploidy operation. In some implementations, the result of this processing operation is then automatically delivered, possibly with reformatting as a diagnosis, to a system component or entity that processes reports the information to a health professional and/or patient. As explained such information can also be automatically processed to produce a treatment, testing, and/or monitoring plan, possibly along with counseling information. Thus, initiating an early stage operation can trigger an end to end sequence in which the health professional, patient or other concerned party is provided with a diagnosis, a plan, counseling and/or other information useful for acting on a physical condition. This is accomplished even though parts of the overall system are physically separated and possibly remote from the location of, *e.g.,* the sample and sequence apparatus.

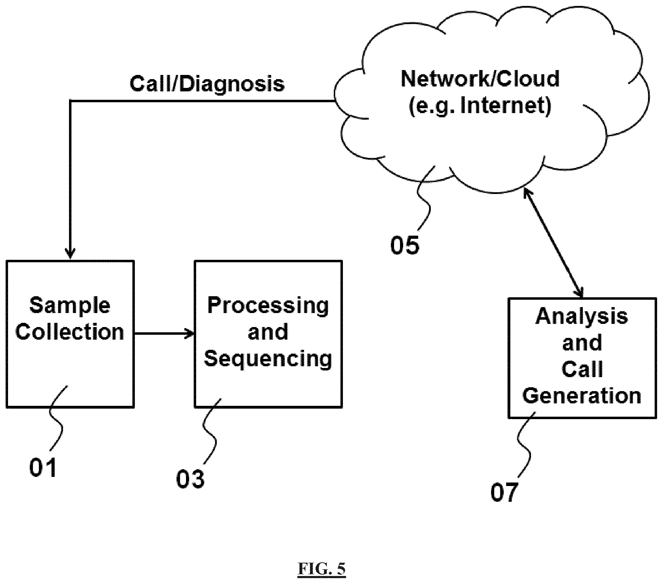

Figure 5 shows one implementation of a dispersed system for producing a call or diagnosis from a test sample. A sample collection location 01 is used for obtaining a test sample from a patient such as a pregnant female or a putative cancer patient. The samples then provided to a processing and sequencing location 03 where the test sample may be processed and sequenced as described above. Location 03 includes apparatus for processing the sample as well as apparatus for sequencing the processed sample. The result of the sequencing, as described elsewhere herein, is a collection of reads which are typically provided in an electronic format and provided to a network such as the Internet, which is indicated by reference number 05 in Figure 5.

The sequence data is provided to a remote location 07 where analysis and call generation are performed. This location may include one or more powerful computational devices such as computers or processors. After the computational resources at location 07 have completed their analysis and generated a call from the sequence information received, the call is relayed back to the network 05. In some implementations, not only is a call generated at location 07 but an associated diagnosis is also generated. The call and or diagnosis are then transmitted across the network and back to the sample collection location 01 as illustrated in Figure 5. As explained, this is simply one of many variations on how the various operations associated with generating a call or diagnosis may be divided among various locations. One common variant involves providing sample collection and processing and sequencing in a single location. Another variation involves providing processing and sequencing at the same location as analysis and call generation.

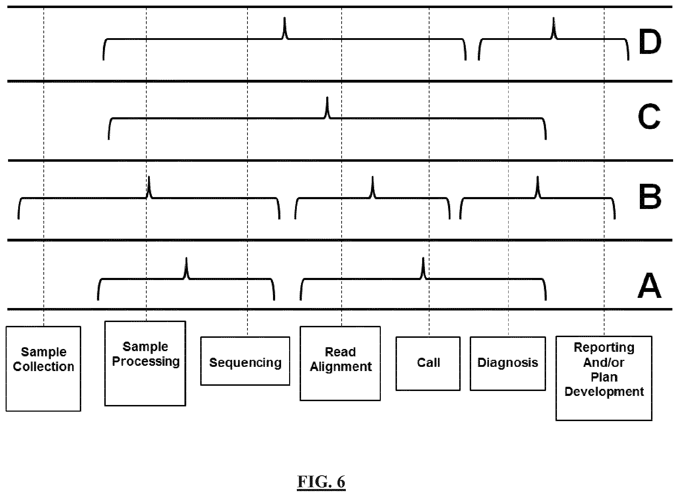

Figure 6 elaborates on the options for performing various operations at distinct locations. In the most granular sense depicted in Figure 6, each of the following operations is performed at a separate location: sample collection, sample processing, sequencing, read alignment, calling, diagnosis, and reporting and/or plan development.

In one embodiment that aggregates some of these operations, sample processing and sequencing are performed in one location and read alignment, calling, and diagnosis are performed at a separate location. See the portion of Figure 6 identified by reference character A. In another implementation, which is identified by character B in Figure 6, sample collection, sample processing, and sequencing are all performed at the same location. In this implementation, read alignment and calling are performed in a second location. Finally, diagnosis and reporting and/or plan development are performed in a third location. In the implementation depicted by character C in Figure 6, sample collection is performed at a first location, sample processing, sequencing, read alignment, calling,, and diagnosis are all performed together at a second location, and reporting and/or plan development are performed at a third location. Finally, in the implementation labeled D in Figure 6, sample collection is performed at a first location, sample processing, sequencing, read alignment, and calling are all performed at a second location, and diagnosis and reporting and/or plan management are performed at a third location.

One embodiment provides a system for use in determining the presence or absence of any one or more different complete fetal chromosomal aneuploidies in a maternal test sample comprising fetal and maternal nucleic acids, the system including a sequencer for receiving a nucleic acid sample and providing fetal and maternal nucleic acid sequence information from the sample; a processor; and a machine readable storage medium comprising instructions for execution on said processor, the instructions comprising:

(a) code for obtaining sequence information for said fetal and maternal nucleic acids in the sample;
(b) code for using said sequence information to computationally identify a number of sequence tags from the fetal and maternal nucleic acids for each of any one or more chromosomes of interest selected from chromosomes 1-22, X, and Y and to identify a number of sequence tags for at least one normalizing chromosome sequence or normalizing chromosome segment sequence for each of said any one or more chromosomes of interest;
(c) code for using said number of sequence tags identified for each of said any one or more chromosomes of interest and said number of sequence tags identified for each normalizing chromosome sequence or normalizing chromosome segment sequence to calculate a single chromosome dose for each of the any one or more chromosomes of interest; and
(d) code for comparing each of the single chromosome doses for each of the any one or more chromosomes of interest to a corresponding threshold value for each of the one or more chromosomes of interest, and thereby determining the presence or absence of any one or more complete different fetal chromosomal aneuploidies in the sample.

In some embodiments, the code for calculating a single chromosome dose for each of the any one or more chromosomes of interest comprises code for calculating a chromosome dose for a selected one of the chromosomes of interest as the ratio of the number of sequence tags identified for the selected chromosome of interest and the number of sequence tags identified for a corresponding at least one normalizing chromosome sequence or normalizing chromosome segment sequence for the selected chromosome of interest.

In some embodiments, the system further comprises code for repeating the calculating of a chromosome dose for each of any remaining chromosome segments of the any one or more segments of any one or more chromosomes of interest.

In some embodiments, the one or more chromosomes of interest selected from chromosomes 1-22, X, and Y comprise at least twenty chromosomes selected from chromosomes 1-22, X, and Y, and wherein the instructions comprise instructions for determining the presence or absence of at least twenty different complete fetal chromosomal aneuploidies is determined.

In some embodiments, the at least one normalizing chromosome sequence is a group of chromosomes selected from chromosomes 1-22, X, and Y. In other embodiments, the at least one normalizing chromosome sequence is a single chromosome selected from chromosomes 1-22, X, and Y.

Another embodiment provides a system for use in determining the presence or absence of any one or more different partial fetal chromosomal aneuploidies in a maternal test sample comprising fetal and maternal nucleic acids, the system comprising: a sequencer for receiving a nucleic acid sample and providing fetal and maternal nucleic acid sequence information from the sample; a processor; and a machine readable storage medium comprising instructions for execution on said processor, the instructions comprising:

(a) code for obtaining sequence information for said fetal and maternal nucleic acids in said sample;
(b) code for using said sequence information to computationally identify a number of sequence tags from the fetal and maternal nucleic acids for each of any one or more segments of any one or more chromosomes of interest selected from chromosomes 1-22, X, and Y and to identify a number of sequence tags for at least one normalizing segment sequence for each of said any one or more segments of any one or more chromosomes of interest;
(c) code using said number of sequence tags identified for each of said any one or more segments of any one or more chromosomes of interest and said number of sequence tags identified for said normalizing segment sequence to calculate a single chromosome segment dose for each of said any one or more segments of any one or more chromosomes of interest; and
(d) code for comparing each of said single chromosome segment doses for each of said any one or more segments of any one or more chromosomes of interest to a corresponding threshold value for each of said any one or more chromosome segments of any one or more chromosome of interest, and thereby determining the presence or absence of one or more different partial fetal chromosomal aneuploidies in said sample.

In some embodiments, the code for calculating a single chromosome segment dose comprises code for calculating a chromosome segment dose for a selected one of the chromosome segments as the ratio of the number of sequence tags identified for the selected chromosome segment and the number of sequence tags identified for a corresponding normalizing segment sequence for the selected chromosome segment.

In some embodiments, the system further comprises code for repeating the calculating of a chromosome segment dose for each of any remaining chromosome segments of the any one or more segments of any one or more chromosomes of interest.

In some embodiments, the system further comprises (i) code for repeating (a)-(d) for test samples from different maternal subjects, and (ii) code for determining the presence or absence of any one or more different partial fetal chromosomal aneuploidies in each of said samples.

In other embodiments of any of the systems provided herein, the code further comprises code for automatically recording the presence or absence of a fetal chromosomal aneuploidy as determined in (d) in a patient medical record for a human subject providing the maternal test sample, wherein the recording is performed using the processor.

In some embodiments of any of the systems provided herein, the sequencer is configured to perform next generation sequencing (NGS). In some embodiments, the sequencer is configured to perform massively parallel sequencing using sequencing-by-synthesis with reversible dye terminators. In other embodiments, the sequencer is configured to perform sequencing-by-ligation. In yet other embodiments, the sequencer is configured to perform single molecule sequencing.

## EXPERIMENTAL

### Example 1

### Preparation and sequencing of primary and enriched sequencing libraries

### a. Preparation of sequencing libraries - abbreviated protocol (ABB)

All sequencing libraries, *i.e.,* primary and enriched libraries, were prepared from approximately 2 ng of purified cfDNA that was extracted from maternal plasma. Library preparation was performed using reagents of the NEBNext™ DNA Sample Prep DNA Reagent Set 1 (Part No. E6000L; New England Biolabs, Ipswich, MA), for Illumina® as follows. Because cell-free plasma DNA is fragmented in nature, no further fragmentation by nebulization or sonication was done on the plasma DNA samples. The overhangs of approximately 2 ng purified cfDNA fragments contained in 40 $\mu$l were converted into phosphorylated blunt ends according to the NEBNext® End Repair Module by incubating in a 1.5 ml microfuge tube the cfDNA with 5$\mu$l 10X phosphorylation buffer, 2 $\mu$l deoxynucleotide solution mix (10 mM each dNTP), 1$\mu$l of a 1:5 dilution of DNA Polymerase I, 1 $\mu$l T4 DNA Polymerase and 1 $\mu$l T4 Polynucleotide Kinase provided in the NEBNext™ DNA Sample Prep DNA Reagent Set 1 for 15 minutes at 20°C. The enzymes were then heat inactivated by incubating the reaction mixture at 75°C for 5 minutes. The mixture was cooled to 4°C, and dA tailing of the blunt-ended DNA was accomplished using 10$\mu$l of the dA-tailing master mix containing the Klenow fragment (3' to 5' exo minus) (NEBNext™ DNA Sample Prep DNA Reagent Set 1), and incubating for 15 minutes at 37°C. Subsequently, the Klenow fragment was heat inactivated by incubating the reaction mixture at 75°C for 5 minutes. Following the inactivation of the Klenow fragment, 1 μl of a 1:5 dilution of Illumina Genomic Adaptor Oligo Mix (Part No. 1000521; Illumina Inc., Hayward, CA) was used to ligate the Illumina adaptors (Non-Index Y-Adaptors) to the dA-tailed DNA using 4 μl of the T4 DNA ligase provided in the NEBNext™ DNA Sample Prep DNA Reagent Set 1, by incubating the reaction mixture for 15 minutes at 25°C. The mixture was cooled to 4°C, and the adaptor-ligated cfDNA was purified from unligated adaptors, adaptor dimers, and other reagents using magnetic beads provided in the Agencourt AMPure XP PCR purification system (Part No. A63881; Beckman Coulter Genomics, Danvers, MA). Eighteen cycles of PCR were performed to selectively enrich adaptor-ligated cfDNA (25 μl) using Phusion ® High-Fidelity Master Mix (25μl; Finnzymes, Woburn, MA) and Illumina's PCR primers (0.5 μM each) complementary to the adaptors (Part No. 1000537 and 1000537). The adaptor-ligated DNA was subjected to PCR (98°C for 30 seconds; 18 cycles of 98°C for 10 seconds, 65°C for 30 seconds, and 72°C for 30; final extension at 72°C for 5 minutes, and hold at 4°C) using Illumina Genomic PCR Primers (Part Nos. 100537 and 1000538) and the Phusion HF PCR Master Mix provided in the NEBNext™ DNA Sample Prep DNA Reagent Set 1, according to the manufacturer's instructions. The amplified product was purified using the Agencourt AMPure XP PCR purification system (Agencourt Bioscience Corporation, Beverly, MA) according to the manufacturer's instructions available at www.beckmangenomics.com/products/AMPureXPProtocol_000387v001.pdf. The purified amplified product was eluted in 40 μl of Qiagen EB Buffer, and the concentration and size distribution of the amplified libraries was analyzed using the Agilent DNA 1000 Kit for the 2100 Bioanalyzer (Agilent technologies Inc., Santa Clara, CA).

### b. Preparation of sequencing libraries -full-length protocol

The full-length protocol described here is essentially the standard protocol provided by Illumina, and only differs from the Illumina protocol in the purification of the amplified library. The Illumina protocol instructs that the amplified library be purified using gel electrophoresis, while the protocol described herein uses magnetic beads for the same purification step. Approximately 2 ng of purified cfDNA extracted from maternal plasma was used to prepare a primary sequencing library using NEBNext™ DNA Sample Prep DNA Reagent Set 1 (Part No. E6000L; New England Biolabs, Ipswich, MA) for Illumina® essentially according to the manufacturer's instructions. All steps except for the final purification of the adaptor-ligated products, which was performed using Agencourt magnetic beads and reagents instead of the purification column, were performed according to the protocol accompanying the NEBNext™ Reagents for Sample Preparation for a genomic DNA library that is sequenced using the Illumina® GAII. The NEBNext™ protocol essentially follows that provided by Illumina, which is available at grcf.jhml.edu/hts/protocols/11257047_ChIP_Sample_Prep.pdf.

The overhangs of approximately 2 ng purified cfDNA fragments contained in 40 μl were converted into phosphorylated blunt ends according to the NEBNext® End Repair Module by incubating the 40μl cfDNA with 5μl 10X phosphorylation buffer, 2 μl deoxynucleotide solution mix (10 mM each dNTP), 1 μl of a 1:5 dilution of DNA Polymerase I, 1 μl T4 DNA Polymerase and 1 μl T4 Polynucleotide Kinase provided in the NEBNext™ DNA Sample Prep DNA Reagent Set 1 in a 200 μl microfuge tube in a thermal cycler for 30 minutes at 20°C. The sample was cooled to 4°C, and purified using a QIAQuick column provided in the QIAQuick PCR Purification Kit (QIAGEN Inc., Valencia, CA) as follows. The 50 μl reaction was transferred to 1.5 ml microfuge tube, and 250 μl of Qiagen Buffer PB were added. The resulting 300 μl were transferred to a QIAquick column, which was centrifuged at 13,000 RPM for 1 minute in a microfuge. The column was washed with 750 μl Qiagen Buffer PE, and re-centrifuged. Residual ethanol was removed by an additional centrifugation for 5 minutes at 13,000 RPM. The DNA was eluted in 39 μl Qiagen Buffer EB by centrifugation. dA tailing of 34 μl of the blunt-ended DNA was accomplished using 16 μl of the dA-tailing master mix containing the Klenow fragment (3' to 5' exo minus) (NEBNext™ DNA Sample Prep DNA Reagent Set 1), and incubating for 30 minutes at 37°C according to the manufacturer's NEBNext® dA-Tailing Module. The sample was cooled to 4°C, and purified using a column provided in the MinElute PCR Purification Kit (QIAGEN Inc., Valencia, CA) as follows. The 50 μl reaction was transferred to 1.5 ml microfuge tube, and 250 μl of Qiagen Buffer PB were added. The 300 μl were transferred to the MinElute column, which was centrifuged at 13,OOORPM for 1 minute in a microfuge. The column was washed with 750 μl Qiagen Buffer PE, and re-centrifuged. Residual ethanol was removed by an additional centrifugation for 5 minutes at 13,000 RPM. The DNA was eluted in 15 μl Qiagen Buffer EB by centrifugation. Ten microliters of the DNA eluate were incubated with 1 μl of a 1:5 dilution of the Illumina Genomic Adapter Oligo Mix (Part No. 1000521), 15 μl of 2X Quick Ligation Reaction Buffer, and 4 μl Quick T4 DNA Ligase, for 15 minutes at 25°C according to the NEBNext® Quick Ligation Module. The sample was cooled to 4°C, and purified using a MinElute column as follows. One hundred and fifty microliters of Qiagen Buffer PE were added to the 30 μl reaction, and the entire volume was transferred to a MinElute column were transferred to a MinElute column, which was centrifuged at 13,OOORPM for 1 minute in a microfuge. The column was washed with 750 μl Qiagen Buffer PE, and re-centrifuged. Residual ethanol was removed by an additional centrifugation for 5 minutes at 13,000 RPM. The DNA was eluted in 28 μl Qiagen Buffer EB by centrifugation. Twenty three microliters of the adaptor-ligated DNA eluate were subjected to 18 cycles of PCR (98°C for 30 seconds; 18 cycles of 98°C for 10 seconds, 65°C for 30 seconds, and 72°C for 30; final extension at 72°C for 5 minutes, and hold at 4°C) using Illumina Genomic PCR Primers (Part Nos. 100537 and 1000538) and the Phusion HF PCR Master Mix provided in the NEBNext™ DNA Sample Prep DNA Reagent Set 1, according to the manufacturer's instructions. The amplified product was purified using the Agencourt AMPure XP PCR purification system (Agencourt Bioscience Corporation, Beverly, MA) according to the manufacturer's instructions available at www.beckmangenomics.com/products/AMPureXPProtocol_000387v001.pdf. The Agencourt AMPure XP PCR purification system removes unincorporated dNTPs, primers, primer dimers, salts and other contaminates, and recovers amplicons greater than 100 bp. The purified amplified product was eluted from the Agencourt beads in 40 μl of Qiagen EB Buffer and the size distribution of the libraries was analyzed using the Agilent DNA 1000 Kit for the 2100 Bioanalyzer (Agilent technologies Inc., Santa Clara, CA).

### c. Analysis of sequencing libraries prepared according to the abbreviated (a) and the full-length (b) protocols

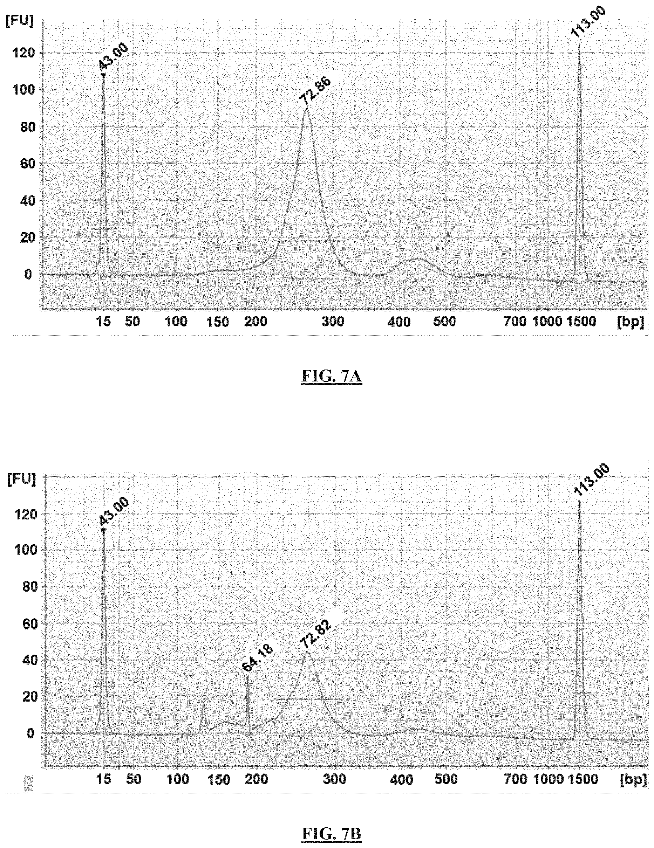

The electropherograms generated by the Bioanalyzer are shown in Figures 7A and 7B. Figure 7A shows the electropherogram of library DNA prepared from cfDNA purified from plasma sample M24228 using the full-length protocol described in (a), and Figure 7B shows the electropherogram of library DNA prepared from cfDNA purified from plasma sample M24228 using the full-length protocol described in (b). In both figures, peaks 1 and 4 represent the 15 bp Lower Marker, and the 1,500 Upper Marker, respectively; the numbers above the peaks indicate the migration times for the library fragments; and the horizontal lines indicate the set threshold for integration. The electropherogram in Figure 7A shows a minor peak of fragments of 187 bp and a major peak of fragments of 263 bp, while the electropherogram in Figure 7B shows only one peak at 265 bp. Integration of the peak areas resulted in a calculated concentration of 0.40 ng/μl for the DNA of the 187 bp peak in Figure 7A, a concentration of 7.34 ng/μl for the DNA of the 263bp peak in Figure 7A, and a concentration of 14.72 ng/μl for the DNA of the 265 bp peak in Figure 7B. The Illumina adaptors that were ligated to the cfDNA are known to be 92 bp, which when subtracted from the 265 bp, indicate that the peak size of the cfDNA is 173 bp. It is possible that the minor peak at 187 bp represents fragments of two primers that were ligated end-to-end. The linear two-primer fragments are eliminated from the final library product when the abbreviated protocol is used. The abbreviated protocol also eliminates other smaller fragments of less than 187 bp. In this example, the concentration of purified adaptor-ligated cfDNA is double that of the adaptor-ligated cfDNA produced using the full-length protocol. It has been noted that the concentration of the adaptor-ligated cfDNA fragments was always greater than that obtained using the full-length protocol (data not shown).

Thus, an advantage of preparing the sequencing library using the abbreviated protocol is that the library obtained consistently comprises only one major peak in the 262-267 bp range while the quality of the library prepared using the full-length protocol varies as reflected by the number and mobility of peaks other than that representing the cfDNA. Non-cfDNA products would occupy space on the flow cell and diminish the quality of the cluster amplification and subsequent imaging of the sequencing reactions, which underlies the overall assignment of the aneuploidy status. The abbreviated protocol was shown not to affect the sequencing of the library.

Another advantage of preparing the sequencing library using the abbreviated protocol is that the three enzymatic steps of blunt-ending, d-A tailing, and adaptor-ligation, take less than an hour to complete to support the validation and implementation of a rapid aneuploid diagnostic service.

Another advantage is that the three enzymatic steps of blunt-ending, d-A tailing, and adaptor ligation, are performed in the same reaction tube, thus avoiding multiple sample transfers that would potentially lead to loss of material, and more importantly to possible sample mix-up and sample contamination.

### Example 2

### Selecting a training set for the Y chromosome using HOPACH clustering

Data reduction has a wide variety of applications, and there exist a variety of suggested approaches. This example used a hybrid clustering method to select a representative training set of female samples for calculation of a mask for the Y chromosome. The derived mask filters out gender non-discriminatory segments of the Y chromosome, providing a useful tool for non-invasive fetal gender discrimination. The clustering method, Hierarchical Ordered Partitioning And Collapsing Hybrid (HOPACH), is a hierarchical tree of clusters. HOPACH methodology combines the strengths of both partitioning and agglomerative clustering methods and allows a research to review clusters at increasing levels of detail. The example involved analyzing samples of 475 normal females known to have no Y chromosomes. A subset of the 475 samples are selected as the training set that is representative of females in the population to be test. Building a representative training set as performed by the example involves the following steps:

1. Providing genomic reads (e.g., 25mer reads) of all available female samples for training purposes (**N**);
2. Aligning genomic reads of all available female samples to a reference genome, thereby providing sequence tags relating to sequence reads and their aligned locations;

3. dividing sequence tag counts in contiguous genomic regions of bins of pre-defined size ( e.g. **M** 1kb bins);

4. Calculating a per-sample within-bin coverage as the total count of non-duplicated sequence tags that have been aligned uniquely to a given region on chromosome Y;

5. Performing HOPACH on a **NxM** matrix and optimizing the number of clusters when Partitioning Around Medoids (PAM) by maximizing average silhouette over a range of possible values;

6. Selecting samples for training sets, e.g., by randomly selecting an equal number of samples for each cluster as described above.

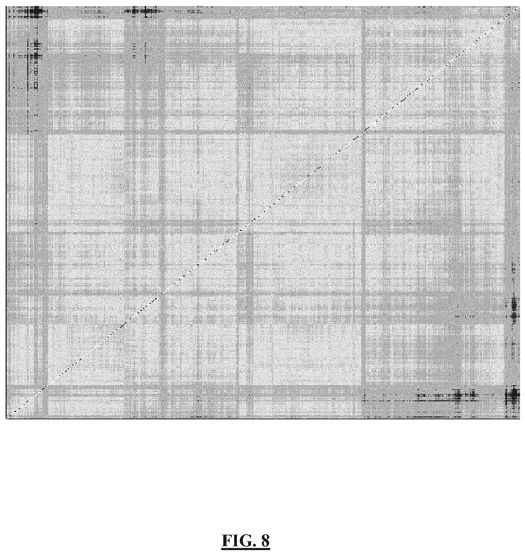

Figure 8 illustrates a correlation heatmap of pairwise chrY 1kb coverage across 475 females. The heatmap shows pairwise coverage correlations across samples in the training set. Both X- and Y- axis are samples sorted by HOPACH results, with each cell representing the degree of correlation of chrY hit coverage for two given training set samples in 1 kb bin. The visible pattern of the correlation map indicates that the samples underlying the obtained clusters have diverse distribution profiles on the Y chromosome.

For validation of diagnostic efficacy of the masked reference, an independent set of female samples and a cohort of low fetal fraction males are used to assess male/female discrimination of chromosome Y counts obtained using a reference sequence filtered by a mask obtained using a training described above.

## Example 3

### Obtaining a mask for the Y chromosome

In calculation to obtain a mask for the Y chromosome, bin size selection should be driven by the most frequent size of the repeat seen in human genome. Studies of various classes of repeat in the human genome and their pattern of occurrence suggest that a 500-1000 bp range as the most optimal for initial binning that can later be coupled with bin merging to produce a final set of masking intervals. However, other technical restrictions may require an analysis to increase bin size, *e.g.,* an upper limit on total count of masking segments, *etc.*

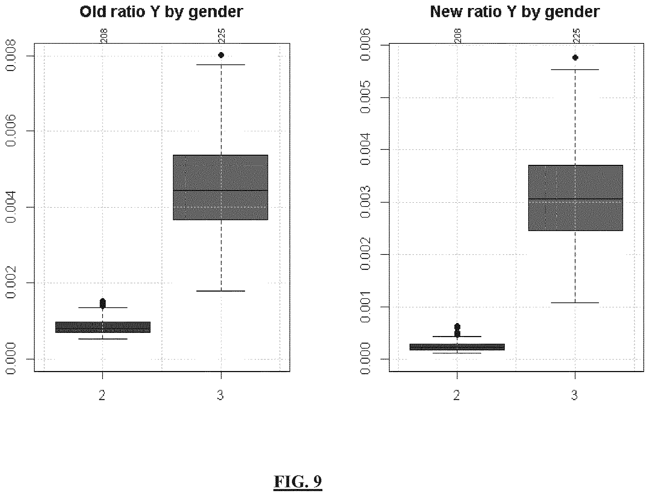

In this example, a 1kb bin size was used to obtain a mask using the training set obtained in Example 2. The mask obtained is used to perform initial chrY filtering, resulting in significant improvement of chrY performance (SNR 20 vs. 35) compared to masking that was based on similar filtering approach with a bin size of 1 Mb, see Figure 9. Figure 9 shows the chrY count/chr4 count using the mask Y chromosome obtained with the method obtained by the following greedy approach:

1. Calculate total of non-duplicated 25mer read counts for every non-overlapping genomic bin of pre-defined size across all female samples in training dataset.

2. Genomic bins are then sorted by absolute counts in decreasing order with most overrepresented bins that correspond to chromosome Y regions being the top candidates for removal/masking.

3. Next, masking threshold is varied from low (e.g. 10% of the bins being masked) to high (e.g. 100% of the bins being masked) and male/female discrimination metric (e.g. a signal to noise ratio, or SNR, calculated by the difference between the samples divided by the standard deviation of the samples) is calculated in an independent validation set. The validation set includes female samples not in the training set and male samples having low fetal fraction.

4. Masking threshold is then established at highest SNR achieved.

Figure 9 shows box-whisker charts of the chrY count/chr4 count for 1 Mb bin size on the left panel and for 1 kb bin size on the right panel. The box on the left labeled by the number "2" shows data obtained from validating female samples that are independent from the female samples in the training set. The box on the right labeled by the number "3" shows data from validating "male samples," which are maternal samples comprising low fraction of male fetal DNA. The line in the middle of a box indicates the mean of the chrY ratio, the upper and lower sides of the box indicate the standard deviation around means. The whiskers indicate the 95% confidence interval. The large SD in males is explained by underlying low fetal fraction. As apparent from the difference between the left panel (1 Mb in size) and the right panel (one Kb bin size), the Y chromosome mask obtained using smaller bin size provides results that further separate male samples from female samples.

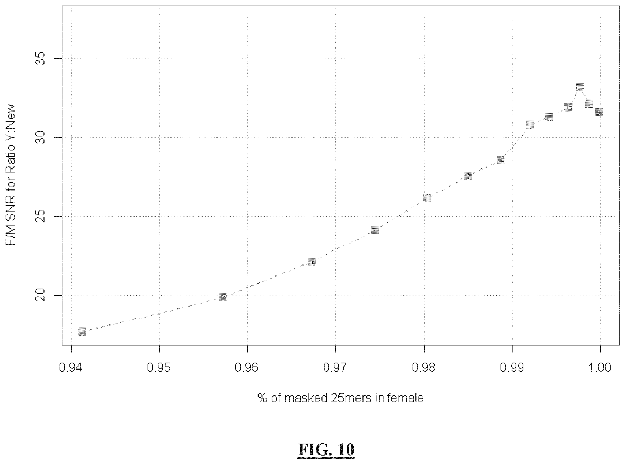

Regarding masking threshold, empirical analyses can assist identification of the most effective threshold value. Figure 10 shows Male/Female discrimination signal to noise ratio as a function of fraction of bins masked. Consistent with theoretical expectations, examination of various thresholds shows that aggressive removal of bins with non-zero representation in females leads to highest SNR The discrimination signal increases continuously up to more than 99%. The signal only starts to drop when very close to 100% of bins having 1 sequence tag count from the female samples were removed. The more aggressive threshold values reduce observed coverage estate observed in fetal male by about 68%.

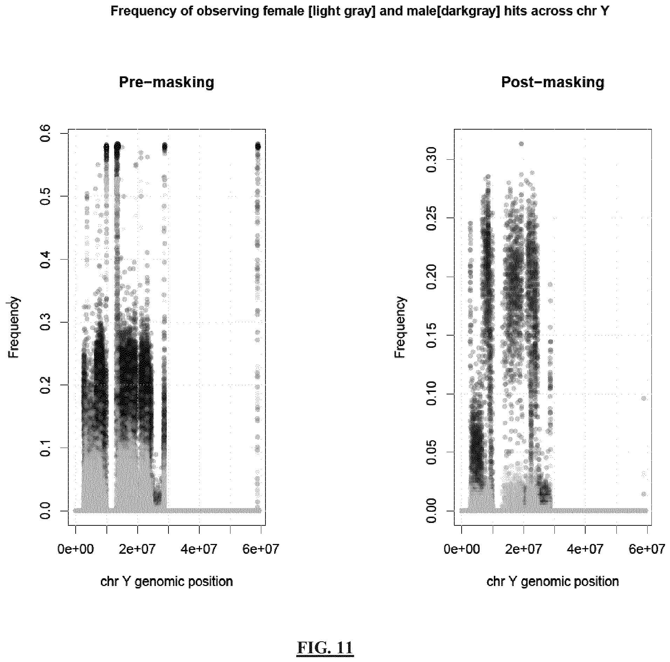

Masks of the Y chromosome and other chromosomes may then be used to calculate the sequence tags that fall on the sequences of interest (including chromosomes and sub-chromosome regions). Using a masked Y chromosome, some embodiments can more efficiently differentiate gender of fetus using cfDNA compared to using an unmasked Y chromosome. Figure 11 shows the frequency distribution of sequence tags mapped to the Y chromosome for samples including female (light gray) vs. male (dark gray) fetal cfDNAs. The left panel shows the distribution of sequence tags mapped to an unmasked Y chromosome. The right panel shows the distribution mapped to a masked Y chromosome according to methods described above. The difference between female (light gray) vs. male (dark gray) samples is significantly and obviously larger for the masked Y chromosome (right panel) relative to the unmasked Y chromosome (left panel).

The following examples illustrate how one may use masked reference sequences such as those described above to evaluate copy number and CNVs of allosomes and autosomes. At least some of the data presented in the examples below were obtained without using masked reference sequences obtained as described above. Nevertheless, the examples provide technical guidance to enable one skilled in the art to use in reference sequence in practicing CNV evaluation and genetic diagnoses.

### Example 4

### Dose and variance for chromosomes 13, 18, 21, X, and Y

To examine the extent of inter-chromosomal and inter-sequencing variation in the number of mapped sequence tags for all chromosomes, plasma cfDNA obtained from peripheral blood of 48 volunteer pregnant subjects was extracted, sequenced and analyzed as follows.

The total number of sequence tags that were mapped to each chromosome (sequence tag density) was determined. Alternatively, the number of mapped sequence tags may be normalized to the length of the chromosome to generate a sequence tag density ratio. The normalization to chromosome length is not a required step, and can be performed solely to reduce the number of digits in a number to simplify it for human interpretation. Chromosome lengths that can be used to normalize the sequence tags counts can be the lengths provided on the world wide web at genome.ucsc.edu/goldenPath/stats.html#hg18.

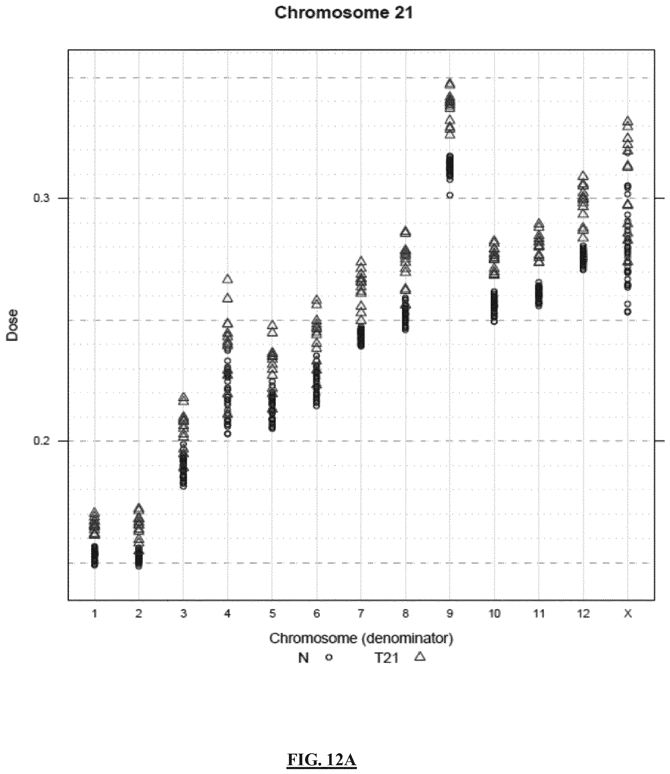

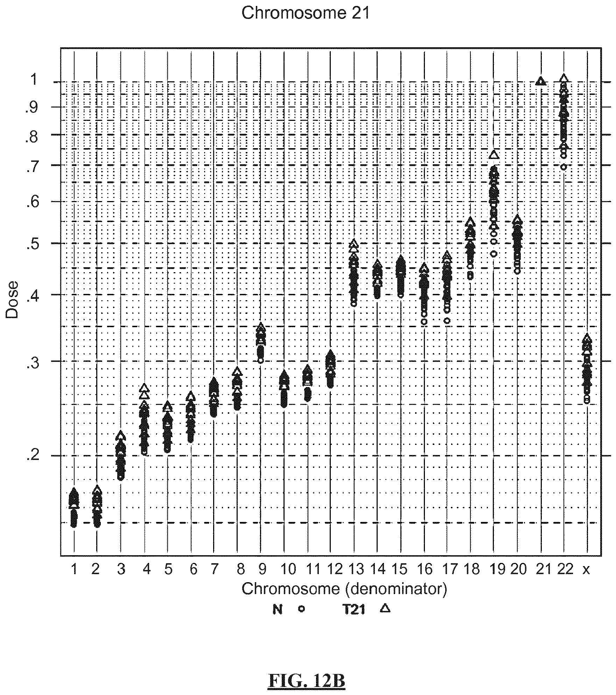

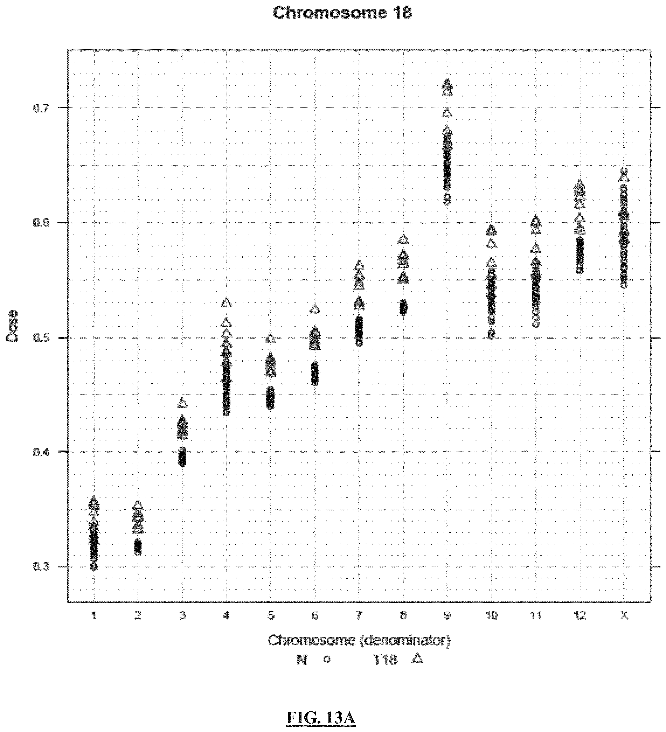

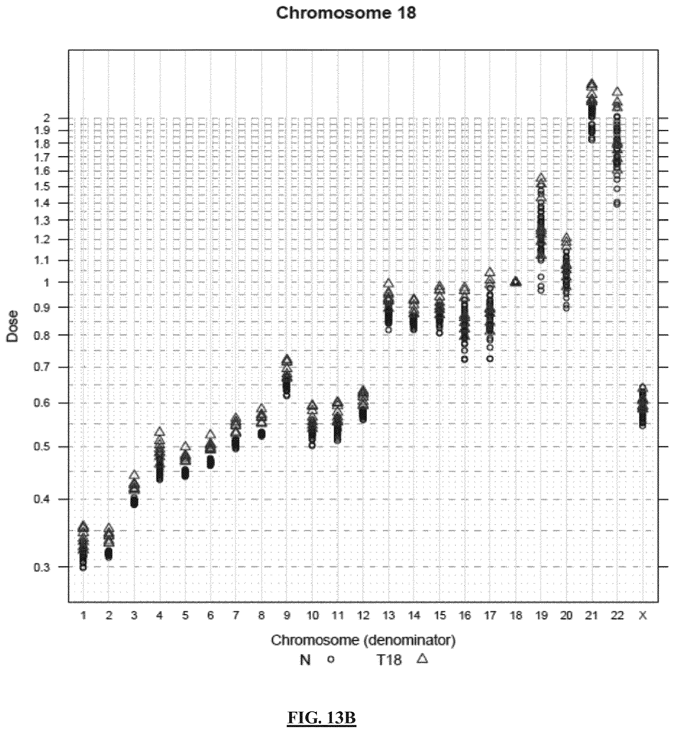

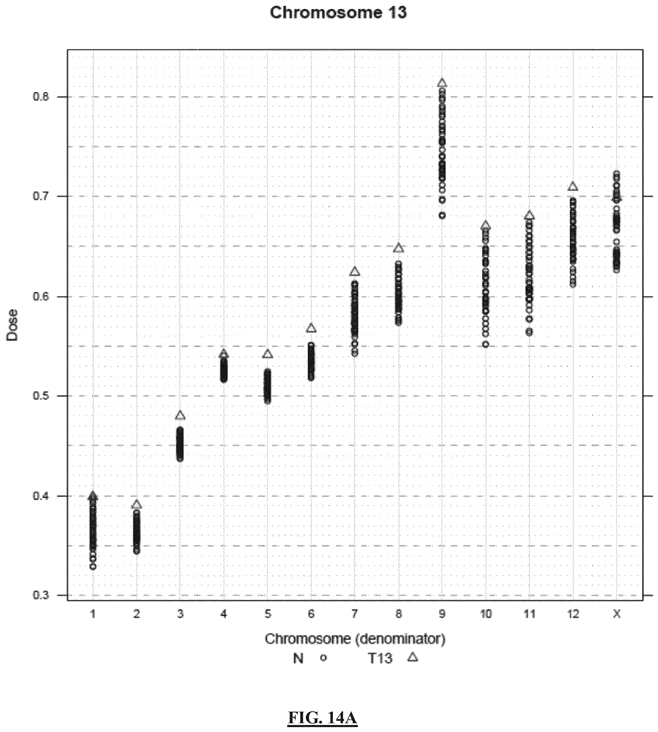

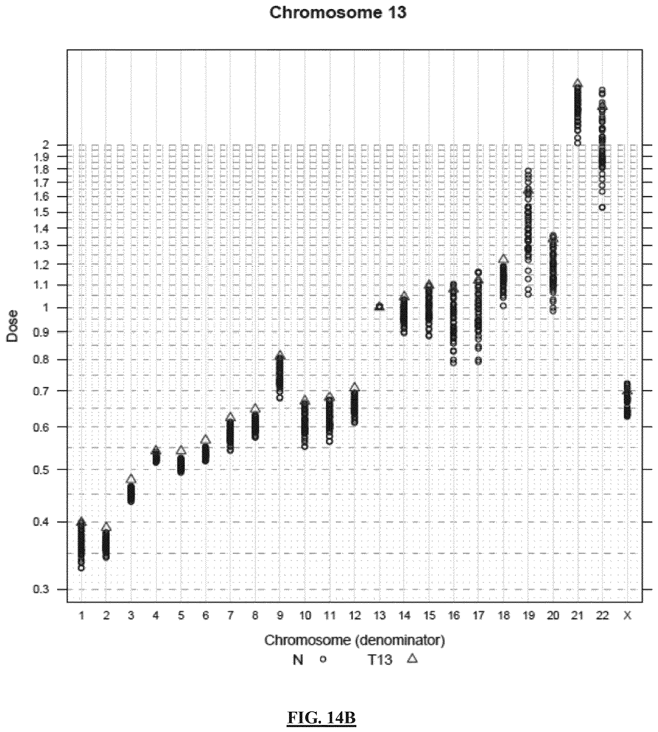

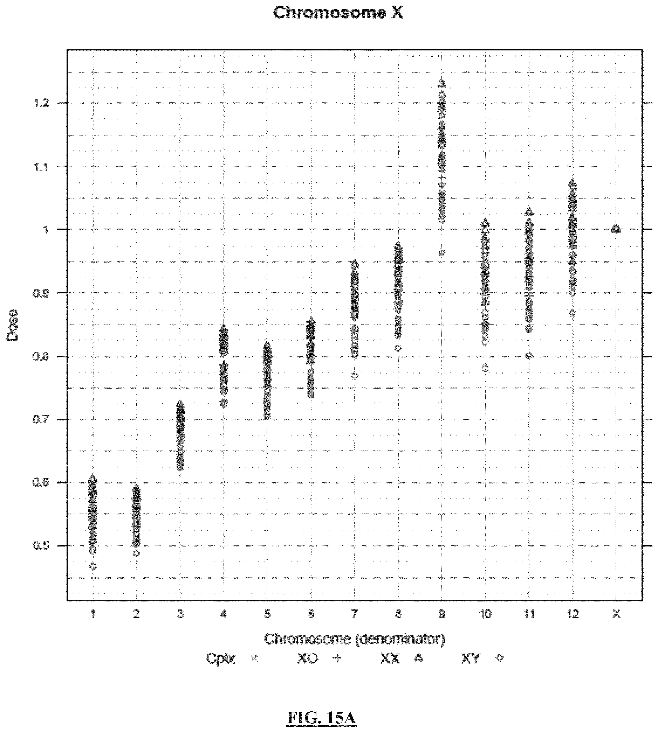

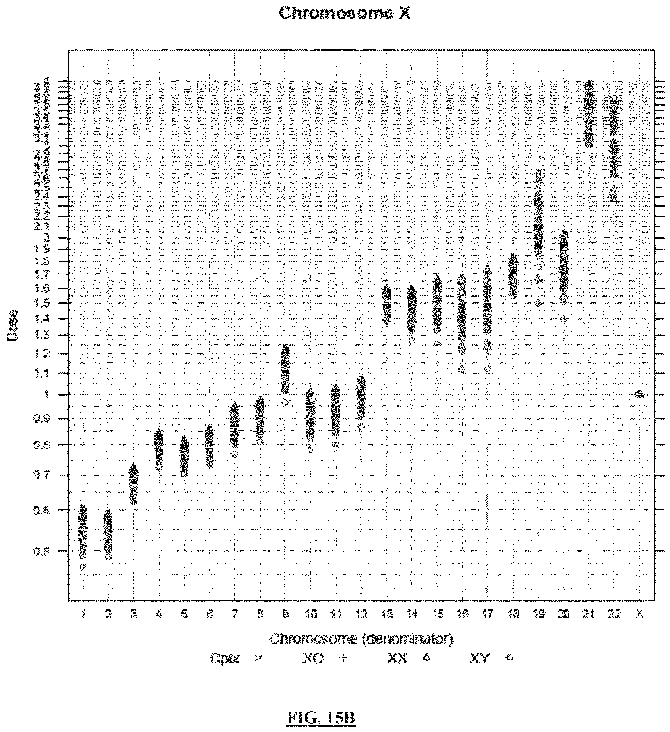

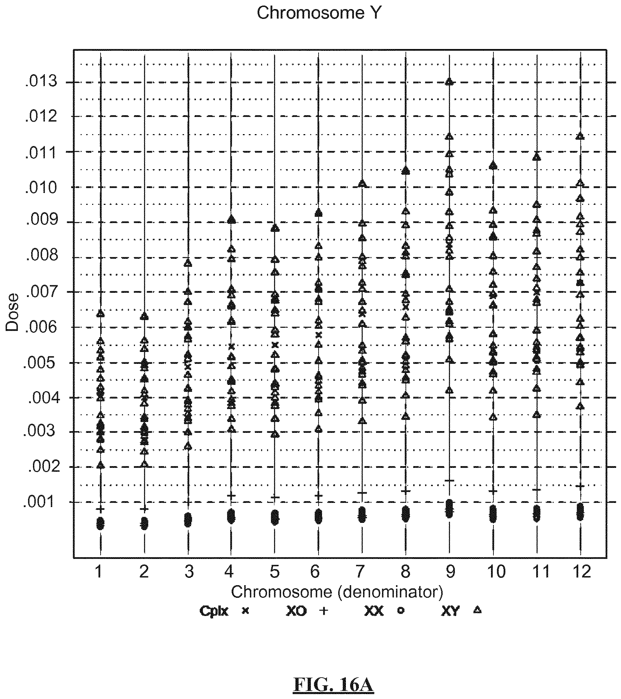

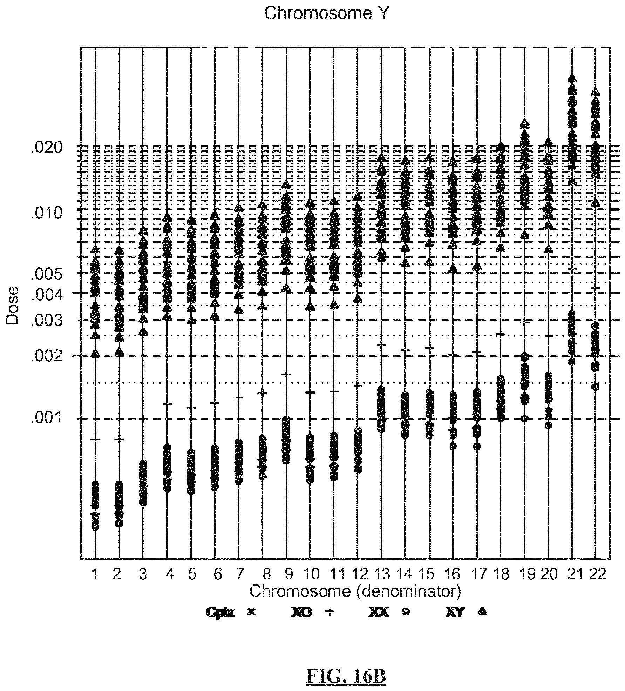

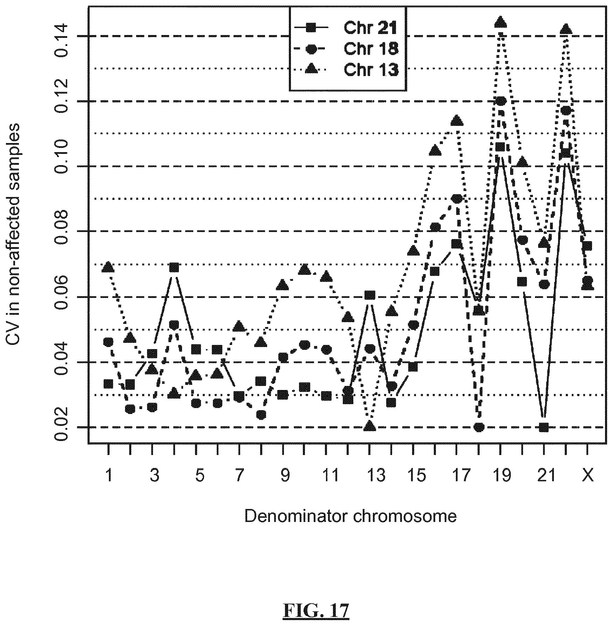

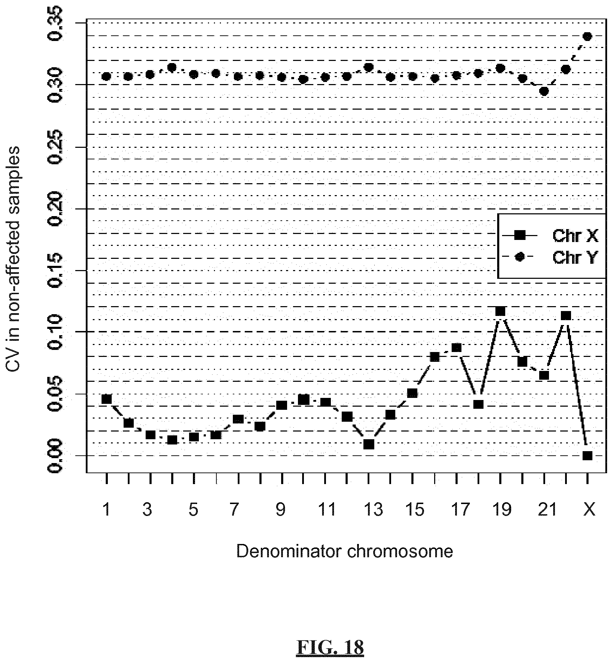

The resulting sequence tag density for each chromosome was related to the sequence tag density of each of the remaining chromosomes to derive a qualified chromosome dose, which was calculated as the ratio of the sequence tag density for the chromosome of interest, e.g., chromosome 21, and the sequence tag density of each of the remaining chromosomes, *i.e.,* chromosomes 1-20, 22 and X. Table 1 provides an example of the calculated qualified chromosome dose for chromosomes of interest 13, 18, 21, X, and Y, determined in one of the qualified samples. Chromosomes doses were determined for all chromosomes in all samples, and the average doses for chromosomes of interest 13, 18, 21, X and Y in the qualified samples are provided in Tables 2 and 3, and depicted in Figures 12-16. Figures 12-16 also depict the chromosome doses for the test samples. The chromosome doses for each of the chromosomes of interest in the qualified samples provides a measure of the variation in the total number of mapped sequence tags for each chromosome of interest relative to that of each of the remaining chromosomes. Thus, qualified chromosome doses can identify the chromosome or a group of chromosomes, i.e., normalizing chromosome that has a variation among samples that is closest to the variation of the chromosome of interest, and that would serve as ideal sequences for normalizing values for further statistical evaluation. Figures 17 and 18 depict the calculated average chromosome doses determined in a population of qualified samples for chromosomes 13, 18, and 21, and chromosomes X and Y.

In some instances, the best normalizing chromosome may not have the least variation, but may have a distribution of qualified doses that best distinguishes a test sample or samples from the qualified samples, *i.e.,* the best normalizing chromosome may not have the lowest variation, but may have the greatest differentiability. Thus, differentiability accounts for the variation in chromosome dose and the distribution of the doses in the qualified samples.

Tables 2 and 3 provide the coefficient of variation as the measure of variability, and p values of Student's *t*-test as a measure of differentiability for chromosomes 18, 21, X and Y, wherein the smaller the *t*-test p value, the greater the differentiability. The differentiability for chromosome 13 was determined as the ratio of difference between the mean chromosome dose in the qualified samples and the dose for chromosome 13 in the only T13 test sample, and the standard deviation of mean of the qualified dose.

The qualified chromosome doses also serve as the basis for determining threshold values when identifying aneuploidies in test samples as described in the following.

**TABLE 1. Qualified Chromosome Dose for Chromosomes 13, 18, 21, X and Y (n=1; sample #11342, 46 XY)**

| Chromosome | chr 21 | chr 18 | chr 13 | chr X | chrY |
|---|---|---|---|---|---|
| chr1 | 0.149901 | 0.306798 | 0.341832 | 0.490969 | 0.003958 |

(continued)

| Chromosome | chr 21 | chr 18 | chr 13 | chr X | chrY |
|---|---|---|---|---|---|
| chr2 | 0.15413 | 0.315452 | 0.351475 | 0.504819 | 0.004069 |
| chr3 | 0.193331 | 0.395685 | 0.44087 | 0.633214 | 0.005104 |
| chr4 | 0.233056 | 0.476988 | 0.531457 | 0.763324 | 0.006153 |
| chr5 | 0.219209 | 0.448649 | 0.499882 | 0.717973 | 0.005787 |
| chr6 | 0.228548 | 0.467763 | 0.521179 | 0.748561 | 0.006034 |
| chr7 | 0.245124 | 0.501688 | 0.558978 | 0.802851 | 0.006472 |
| chr8 | 0.256279 | 0.524519 | 0.584416 | 0.839388 | 0.006766 |
| chr9 | 0.309871 | 0.634203 | 0.706625 | 1.014915 | 0.008181 |
| chr10 | 0.25122 | 0.514164 | 0.572879 | 0.822817 | 0.006633 |
| chr11 | 0.257168 | 0.526338 | 0.586443 | 0.8423 | 0.00679 |
| chr12 | 0.275192 | 0.563227 | 0.627544 | 0.901332 | 0.007265 |
| chr13 | 0.438522 | 0.897509 | 1 | 1.436285 | 0.011578 |
| chr14 | 0.405957 | 0.830858 | 0.925738 | 1.329624 | 0.010718 |
| chr15 | 0.406855 | 0.832697 | 0.927786 | 1.332566 | 0.010742 |
| chr16 | 0.376148 | 0.769849 | 0.857762 | 1.231991 | 0.009931 |
| chr17 | 0.383027 | 0.783928 | 0.873448 | 1.254521 | 0.010112 |
| chr18 | 0.488599 | 1 | 1.114194 | 1.600301 | 0.0129 |
| chr19 | 0.535867 | 1.096742 | 1.221984 | 1.755118 | 0.014148 |
| chr20 | 0.467308 | 0.956424 | 1.065642 | 1.530566 | 0.012338 |
| chr21 | 1 | 2.046668 | 2.280386 | 3.275285 | 0.026401 |
| chr22 | 0.756263 | 1.547819 | 1.724572 | 2.476977 | 0.019966 |
| chrX | 0.305317 | 0.624882 | 0.696241 | 1 | 0.008061 |
| chrY | 37.87675 | 77.52114 | 86.37362 | 124.0572 | 1 |

**TABLE 2. Qualified Chromosome Dose, Variance and Differentiability for chromosomes 21, 18 and 13**

| | 21 (n=35) | | | | 18 (n=40) | | | |
|---|---|---|---|---|---|---|---|---|
| | Avg | Stdev | CV | p value of t-test | Avg | Stdev | CV | p value of t-test |
| chr1 | 0.15335 | 0.001997 | 1.30 | 3.18E-10 | 0.31941 | 0.008384 | 2.62 | 0.001675 |
| chr2 | 0.15267 | 0.001966 | 1.29 | 9.87E-07 | 0.31807 | 0.001756 | 0.55 | 4.39E-05 |
| chr3 | 0.18936 | 0.004233 | 2.24 | 1.04E-05 | 0.39475 | 0.002406 | 0.61 | 3.39E-05 |
| chr4 | 0.21998 | 0.010668 | 4.85 | 0.000501 | 0.45873 | 0.014292 | 3.12 | 0.001349 |
| chr5 | 0.21383 | 0.005058 | 2.37 | 1.43E-05 | 0.44582 | 0.003288 | 0.74 | 3.09E-05 |
| chr6 | 0.22435 | 0.005258 | 2.34 | 1.48E-05 | 0.46761 | 0.003481 | 0.74 | 2.32E-05 |
| chr7 | 0.24348 | 0.002298 | 0.94 | 2.05E-07 | 0.50765 | 0.004669 | 0.92 | 9.07E-05 |
| chr8 | 0.25269 | 0.003497 | 1.38 | 1.52E-06 | 0.52677 | 0.002046 | 0.39 | 4.89E-05 |
| chr9 | 0.31276 | 0.003095 | 0.99 | 3.83E-09 | 0.65165 | 0.013851 | 2.13 | 0.000559 |
| chr10 | 0.25618 | 0.003112 | 1.21 | 2.28E-10 | 0.53354 | 0.013431 | 2.52 | 0.002137 |
| chr11 | 0.26075 | 0.00247 | 0.95 | 1.08E-09 | 0.54324 | 0.012859 | 2.37 | 0.000998 |
| chr12 | 0.27563 | 0.002316 | 0.84 | 2.04E-07 | 0.57445 | 0.006495 | 1.13 | 0.000125 |
| chr13 | 0.41828 | 0.016782 | 4.01 | 0.000123 | 0.87245 | 0.020942 | 2.40 | 0.000164 |
| chr14 | 0.40671 | 0.002994 | 0.74 | 7.33E-08 | 0.84731 | 0.010864 | 1.28 | 0.000149 |
| chr15 | 0.41861 | 0.007686 | 1.84 | 1.85E-10 | 0.87164 | 0.027373 | 3.14 | 0.003862 |
| chr16 | 0.39977 | 0.018882 | 4.72 | 7.33E-06 | 0.83313 | 0.050781 | 6.10 | 0.075458 |
| chr17 | 0.41394 | 0.02313 | 5.59 | 0.000248 | 0.86165 | 0.060048 | 6.97 | 0.088579 |
| chr18 | 0.47236 | 0.016627 | 3.52 | 1.3E-07 | | | | |
| chr19 | 0.59435 | 0.05064 | 8.52 | 0.01494 | 1.23932 | 0.12315 | 9.94 | 0.231139 |
| chr20 | 0.49464 | 0.021839 | 4.42 | 2.16E-06 | 1.03023 | 0.058995 | 5.73 | 0.061101 |
| chr21 | | | | | 2.03419 | 0.08841 | 4.35 | 2.81E-05 |

(continued)

| 21 (n=35) 18 (n=40) | | | | | | | | |
|---|---|---|---|---|---|---|---|---|
| | Avg | Stdev | CV | p value of t-test | Avg | Stdev | CV | p value of t-test |
| chr22 | 0.84824 | 0.070613 | 8.32 | 0.02209 | 1.76258 | 0.169864 | 9.64 | 0.181808 |
| chrX | 0.27846 | 0.015546 | 5.58 | 0.000213 | 0.58691 | 0.026637 | 4.54 | 0.064883 |

**TABLE 3. Qualified Chromosome Dose, Variance and Differentiability for chromosomes 13, X, and Y**

| 13 (n=47) | | | | | X (n=19) | | | |
|---|---|---|---|---|---|---|---|---|
| | **Avg** | **Stdev** | **CV** | **Diff** | **Avg** | **Stdev** | **CV** | **t-test** |
| chr1 | 0.36536 | 0.01775 | 4.86 | 1.904 | 0.56717 | 0.025988 | 4.58 | 0.001013 |
| chr2 | 0.36400 | 0.009817 | 2.70 | 2.704 | 0.56753 | 0.014871 | 2.62 | |
| chr3 | 0.45168 | 0.007809 | 1.73 | 3.592 | 0.70524 | 0.011932 | 1.69 | |
| chr4 | 0.52541 | 0.005264 | 1.00 | 3.083 | 0.82491 | 0.010537 | 1.28 | |
| chr5 | 0.51010 | 0.007922 | 1.55 | 3.944 | 0.79690 | 0.012227 | 1.53 | 1.29E-11 |
| chr6 | 0.53516 | 0.008575 | 1.60 | 3.758 | 0.83594 | 0.013719 | 1.64 | 2.79E-11 |
| chr7 | 0.58081 | 0.017692 | 3.05 | 2.445 | 0.90507 | 0.026437 | 2.92 | 7.41E-07 |
| chr8 | 0.60261 | 0.015434 | 2.56 | 2.917 | 0.93990 | 0.022506 | 2.39 | 2.11E-08 |
| chr9 | 0.74559 | 0.032065 | 4.30 | 2.102 | 1.15822 | 0.047092 | 4.07 | 0.000228 |
| chr10 | 0.61018 | 0.029139 | 4.78 | 2.060 | 0.94713 | 0.042866 | 4.53 | 0.000964 |
| chr11 | 0.62133 | 0.028323 | 4.56 | 2.081 | 0.96544 | 0.041782 | 4.33 | 0.000419 |
| chr12 | 0.65712 | 0.021853 | 3.33 | 2.380 | 1.02296 | 0.032276 | 3.16 | 3.95E-06 |
| chr13 | | | | | 1.56771 | 0.014258 | 0.91 | 2.47E-15 |
| chr14 | 0.96966 | 0.034017 | 3.51 | 2.233 | 1.50951 | 0.05009 | 3.32 | 8.24E-06 |
| chr15 | 0.99673 | 0.053512 | 5.37 | 1.888 | 1.54618 | 0.077547 | 5.02 | 0.002925 |
| chr16 | 0.95169 | 0.080007 | 8.41 | 1.613 | 1.46673 | 0.117073 | 7.98 | 0.114232 |
| chr17 | 0.98547 | 0.091918 | 9.33 | 1.484 | 1.51571 | 0.132775 | 8.76 | 0.188271 |
| chr18 | 1.13124 | 0.040032 | 3.54 | 2.312 | 1.74146 | 0.072447 | 4.16 | 0.001674 |
| chr19 | 1.41624 | 0.174476 | 12.32 | 1.306 | 2.16586 | 0.252888 | 11.68 | 0.460752 |
| chr20 | 1.17705 | 0.094807 | 8.05 | 1.695 | 1.81576 | 0.137494 | 7.57 | 0.08801 |
| chr21 | 2.33660 | 0.131317 | 5.62 | 1.927 | 3.63243 | 0.235392 | 6.48 | 0.00675 |
| chr22 | 2.01678 | 0.243883 | 12.09 | 1.364 | 3.08943 | 0.34981 | 11.32 | 0.409449 |
| chrX | 0.66679 | 0.028788 | 4.32 | 1.114 | | | | |
| chr2-6 | 0.46751 | 0.006762 | 1.45 | 4.066 | | | | |
| chr3-6 | 0.50332 | 0.005161 | 1.03 | 5.260 | | | | |

Examples of diagnoses of T21, T13, T18 and a case of Turner syndrome obtained using the normalizing chromosomes, chromosome doses and differentiability for each of the chromosomes of interest are described in Example 5. Note that although Example 5 shows that the average of the tags on the normalizing chromosome is used for analysis of aneuploidy, the sum of the tags for the normalizing chromosome can be used instead in other embodiments.

## Example 5

### Diagnosis of Fetal Aneuploidy Using Normalizing Chromosomes

To apply the use of chromosome doses for assessing aneuploidy in a biological test sample, maternal blood test samples were obtained from pregnant volunteers and cfDNA was prepared, sequenced and analyzed using method described above.

*Trisomy 21*

Table 4 provides the calculated dose for chromosome 21 in an exemplary test sample (#11403). The calculated threshold for the positive diagnosis of T21 aneuploidy was set at > 2 standard deviations from the mean of the qualified (normal) samples. A diagnosis for T21 was given based on the chromosome dose in the test sample being greater than the set threshold. Chromosomes 14 and 15 were used as normalizing chromosomes in separate calculations to show that either a chromosome having the lowest variability, e.g., chromosome 14, or a chromosome having the greatest differentiability, e.g., chromosome 15, can be used to identify the aneuploidy. Thirteen T21 samples were identified using the calculated chromosome doses, and the aneuploidy samples were confirmed to be T21 by karyotype.

**TABLE 4. Chromosome Dose for a T21 aneuploidy (sample #11403, 47 XY +21)**

| Chromosome | Sequence Tag Density | Chromosome Dose for Chr 21 | Threshold |
|---|---|---|---|
| Chr21 | 333,660 | 0.419672 | 0.412696 |
| Chr14 | 795,050 | | |
| Chr21 | 333,660 | 0.441038 | 0.433978 |
| Chr15 | 756,533 | | |

*Trisomy 18*

Table 5 provides the calculated dose for chromosome 18 in a test sample (#11390). The calculated threshold for the positive diagnosis of T18 aneuploidy was set at 2 standard deviations from the mean of the qualified (normal) samples. A diagnosis for T18 was given based on the chromosome dose in the test sample being greater than the set threshold. Chromosome 8 was used as the normalizing chromosome. In this instance chromosome 8 had the lowest variability and the greatest differentiability. Eight T18 samples were identified using chromosome doses, and were confirmed to be T18 by karyotype.

These data show that a normalizing chromosome can have both the lowest variability and the greatest differentiability.

**TABLE 5. Chromosome Dose for a T18 aneuploidy (sample #11390, 47 XY +18)**

| Chromosome | Sequence Tag Density | Chromosome Dose for Chr 18 | Threshold |
|---|---|---|---|
| Chr18 | 602,506 | 0.585069 | 0.530867 |
| Chr8 | 1,029,803 | | |

*Trisomy 13*

Table 6 provides the calculated dose for chromosome 13 in a test sample (#51236). The calculated threshold for the positive diagnosis of T13 aneuploidy was set at 2 standard deviations from the mean of the qualified samples. A diagnosis for T13 was given based on the chromosome dose in the test sample being greater than the set threshold. The chromosome dose for chromosome 13 was calculated using either chromosome 5 or the group of chromosomes 3, 4, 5, and 6 as the normalizing chromosome. One T13 sample was identified.

**TABLE 6. Chromosome Dose for a T13 aneuploidy (sample #51236, 47 XY +13)**

| Chromosome | Sequence Tag Density | Chromosome Dose for Chr 13 | Threshold |
|---|---|---|---|
| Chr13 | 692,242 | 0.541343 | 0.52594 |
| Chr5 | 1,278,749 | | |
| Chr13 | 692,242 | | |
| Chr3-6 [average] | 1,304,954 | 0.530472 | 0.513647 |

The sequence tag density for chromosomes 3-6 is the average tag counts for chromosomes 3-6.

The data show that the combination of chromosomes 3, 4, 5 and 6 provide a variability that is lower than that of chromosome 5, and the greatest differentiability than any of the other chromosomes.

Thus, a group of chromosomes can be used as the normalizing chromosome to determine chromosome doses and identify aneuploidies.

### Turner Syndrome (monosomy X)

Table 7 provides the calculated dose for chromosomes X and Y in a test sample (#51238). The calculated threshold for the positive diagnosis of Turner Syndrome (monosomy X) was set for the X chromosome at < -2 standard deviations from the mean, and for the absence of the Y chromosome at < -2 standard deviations from the mean for qualified (normal) samples.

**TABLE 7. Chromosome Dose for a Turners (XO) aneuploidy (sample #51238, 45 X)**

| Chromosome | Sequence Tag Density | Chromosome Dose for Chr X and Chr Y | Threshold |
|---|---|---|---|
| ChrX | 873,631 | 0.786642 | 0.803832 |
| Chr4 | 1,110,582 | | |
| ChrY | 1,321 | | |
| Chr_Total (1-22, X) (Average) | 856,623.6 | 0.001542101 | 0.00211208 |

A sample having an X chromosome dose less than that of the set threshold was identified as having less than one X chromosome. The same sample was determined to have a Y chromosome dose that was less than the set threshold, indicating that the sample did not have a Y chromosome. Thus, the combination of chromosome doses for X and Y were used to identify the Turner Syndrome (monosomy X) samples.

Thus, the method provided enables for the determination of CNV of chromosomes. In particular, the method enables for the determination of over- and under-representation chromosomal aneuploidies by massively parallel sequencing of maternal plasma cfDNA and identification of normalizing chromosomes for the statistical analysis of the sequencing data. The sensitivity and reliability of the method allow for accurate first and second trimester aneuploidy testing.

### Example 6

### Demonstration of Detection of Aneuploidy

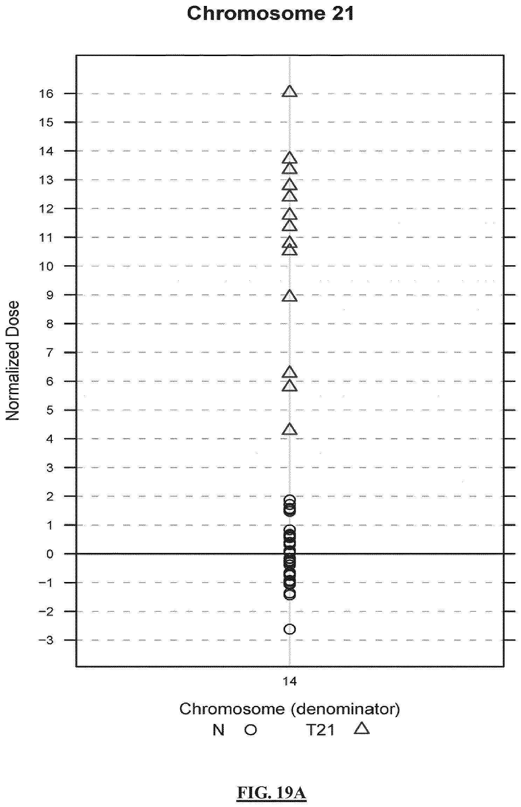

Sequencing data obtained for the samples described in Examples 2 and 3, and shown in Figures 12-16 were further analyzed to illustrate the sensitivity of the method in successfully identifying aneuploidies in maternal samples. Normalized chromosome doses for chromosomes 21, 18, 13 X and Y were analyzed as a distribution relative to the standard deviation of the mean (Y-axis) and shown in Figures 19A-19E. The normalizing chromosome used is shown as the denominator (X-axis).

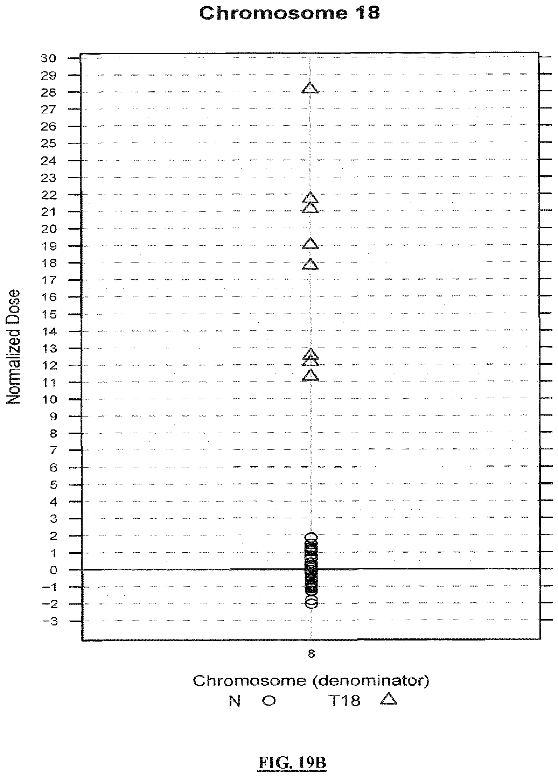

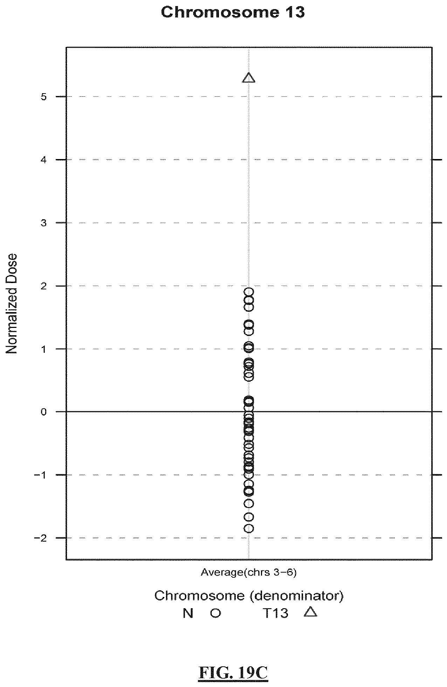

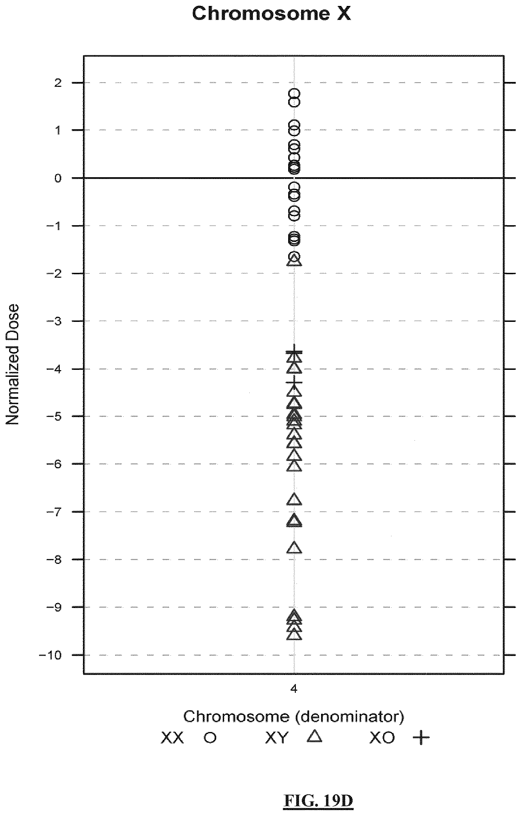

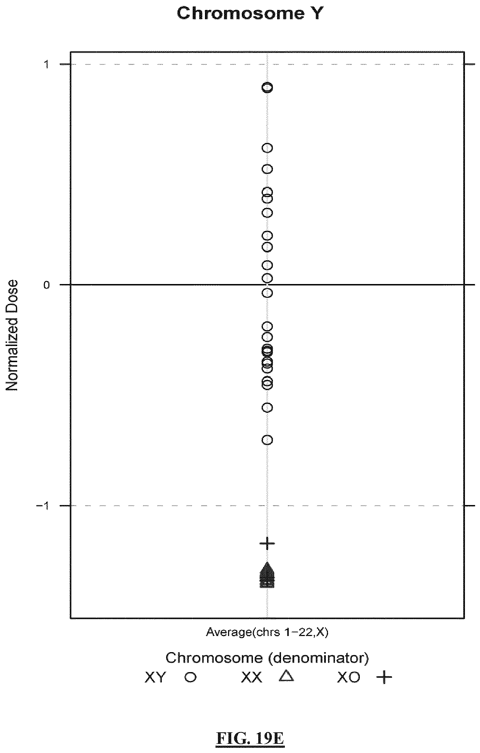

Figure 19A shows the distribution of chromosome doses relative to the standard deviation from the mean for chromosome 21 dose in the unaffected samples (o) and the trisomy 21 samples (T21; Δ) when using chromosome 14 as the normalizing chromosome for chromosome 21. Figure 19B shows the distribution of chromosome doses relative to the standard deviation from the mean for chromosome 18 dose in the unaffected samples (o) and the trisomy 18 samples (T18; Δ) when using chromosome 8 as the normalizing chromosome for chromosome 18. Figure 19C shows the distribution of chromosome doses relative to the standard deviation from the mean for chromosome 13 dose in the unaffected samples (o) and the trisomy 13 samples (T13; Δ), using the average sequence tag density of the group of chromosomes 3, 4, 5, and 6 as the normalizing chromosome to determine the chromosome dose for chromosome 13. Figure 19D shows the distribution of chromosome doses relative to the standard deviation from the mean for chromosome X dose in the unaffected female samples (o), the unaffected male samples (Δ), and the monosomy X samples (XO; +) when using chromosome 4 as the normalizing chromosome for chromosome X. Figure 19E shows the distribution of chromosome doses relative to the standard deviation from the mean for chromosome Y dose in the unaffected male samples (o the unaffected female sample s (Δ), and the monosomy X samples (+), when using the average sequence tag density of the group of chromosomes 1-22 and X as the normalizing chromosome to determine the chromosome dose for chromosome Y.

The data show that trisomy 21, trisomy 18, trisomy 13 were clearly distinguishable from the unaffected (normal) samples. The monosomy X samples were easily identifiable as having chromosome X dose that were clearly lower than those of unaffected female samples (Figure 19D), and as having chromosome Y doses that were clearly lower than that of the unaffected male samples (Figure 19E).

Therefore the method provided is sensitive and specific for determining the presence or absence of chromosomal aneuploidies in a maternal blood sample.

**Example 7**

**Genome Wide Fetal Aneuploidy Detection by Sequencing of Maternal Plasma DNA: Diagnostic Accuracy in a Prospective, Blinded, Multicenter Study**

The method for determining the presence or absence of aneuploidies in maternal test samples was used in a prospective study, and its diagnostic accuracy was shown as described below. The prospective study further demonstrates the efficacy of the method to detect fetal aneuploidy for multiple chromosomes across the genome. The blinded study emulates an actual population of pregnant women in which the fetal karyotype is unknown, and all samples with any abnormal karyotypes were selected for sequencing. Determinations of the classifications made according to the method of the disclosure were compared to fetal karyotypes from invasive procedures to determine the diagnostic performance of the method for multiple chromosomal aneuploidies.

*Summary of this example.*

Blood samples were collected in a prospective, blinded study from 2,882 women undergoing prenatal diagnostic procedures at 60 United States sites (clinicaltrials.gov NCT01122524).

An independent biostatistician selected all singleton pregnancies with any abnormal karyotype, and a balanced number of randomly selected pregnancies with euploid karyotypes. Chromosome classifications were made for each sample according the method disclosed herein and compared to fetal karyotype.

Within an analysis cohort of 532 samples, 89/89 trisomy 21 cases, (sensitivity 100% (95% CI 95·9 - 100)), 35/36 trisomy 18 cases (sensitivity 97.2%, (95% CI 85.5 - 99.9)), 11/14 trisomy 13 cases (sensitivity 78.6%, (95% CI 49.2 - 99.9)), 232/233 females (sensitivity 99.6%, (95% CI 97.6 - >99.9)), 184/184 males (sensitivity 100%, 95% CI 98.0 - 100)), and 15/16 monosomy X cases (sensitivity 93.8%, 95% CI 69.8 - 99.8)) were classified. There were no false positives for autosomal aneuploidies in unaffected subjects (100% specificity, (95% CI >98.5 - 100)). In addition, fetuses with mosaicism for trisomy 21 (3/3), trisomy 18 (1/1), and monosomy X (2/7), three cases of translocation trisomy, two cases of other autosomal trisomies (20 and 16) and other sex chromosome aneuploidies (XXX, XXY and XYY) were correctly classified.

The results further demonstrate the efficacy of the present method to detect fetal aneuploidy for multiple chromosomes across the genome using maternal plasma DNA. The high sensitivity and specificity for the detection of trisomies 21, 18, 13 and monosomy X suggest that the present method can be incorporated into existing aneuploidy screening algorithms to reduce unnecessary invasive procedures.

*Materials and Methods*

The MELISSA (MatErnal BLood IS Source to Accurately diagnose fetal aneuploidy) study was conducted as a prospective, multi-center observational study with blinded nested case: control analyses. Pregnant women, 18 years and older undergoing an invasive prenatal procedure to determine fetal karyotype were recruited (Clinicaltrials.gov NCT01122524). Eligibility criteria included pregnant women between 8 weeks, 0 days and 22 weeks, 0 days gestation who met at least one of the following additional criteria: age ≥ 38 years, positive screening test result (serum analytes and/or nuchal translucency (NT) measurement), presence of ultrasound markers associated with increased risk for fetal aneuploidy, or prior aneuploid fetus. Written informed consent was obtained from all women who agreed to participate.

Enrollment occurred at 60 geographically dispersed medical centers in 25 states per protocol approved by institutional review boards (IRB) at each institution. Two clinical research organizations (CROs) (Quintiles, Durham, NC and Emphusion, San Francisco, CA) were retained to maintain study blinding and provide clinical data management, data monitoring, biostatistics, and data analysis services.

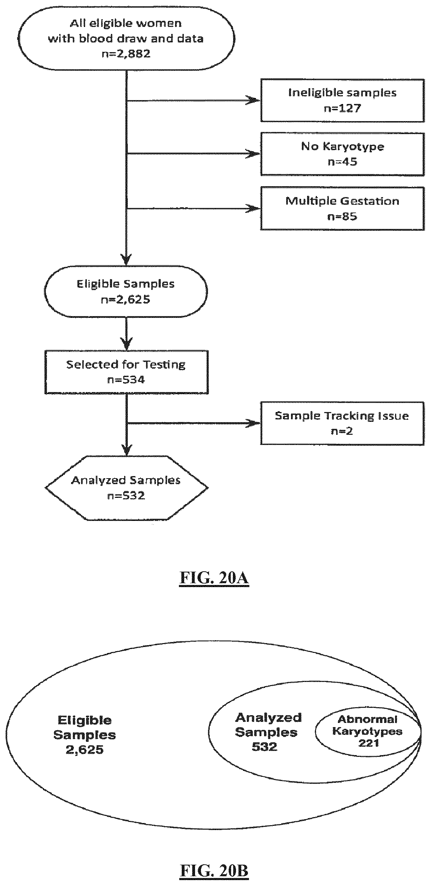

Before any invasive procedure, a peripheral venous blood sample (17 mL) was collected in two acid citrate dextrose (ACD) tubes (Becton Dickinson) that were de-identified and labeled with a unique study number. Site research personnel entered study number, date, and time of blood draw into a secure electronic case report form (eCRF). Whole blood samples were shipped overnight in temperature-controlled containers from sites to the laboratory (Verinata Health, Inc., CA). Upon receipt and sample inspection, cell-free plasma was prepared and stored frozen at -80°C in 2 to 4 aliquots until time of sequencing. Date and time of sample receipt at the laboratory were recorded. A sample was determined to be eligible for analysis if it was received overnight, was cool to touch, and contained at least 7 mL blood. Samples that were eligible at receipt were reported to the CRO weekly and used for selection on a random sampling list (see below and Figure 20). Clinical data from the woman's current pregnancy and fetal karyotype were entered into the eCRF by site research personnel and verified by CRO monitors through source document review.

Sample size determination was based on the precision of the estimates for a targeted range of performance characteristics (sensitivity and specificity) for the index test. Specifically, the number of affected (T21, T18, T13, male, female, or monosomy X) cases and unaffected (non-T21, non-T18, non-T13, not male, not female, or not monosomy X) controls were determined to estimate the sensitivity and specificity, respectively, to within a pre-specified small margin of error based on the normal approximation (N=(1.96 $\sqrt{}$(1-p)/margin of error)$^2$, where p=the estimate of the sensitivity or specificity). Assuming a true sensitivity of 95% or greater, a sample size between 73 to 114 cases ensured that the precision of the estimate of sensitivity would be such that the lower bound of the 95% confidence interval (CI) would be 90% or greater (margin of error $\leq$5%). For smaller sample sizes, a larger estimated margin of error of the 95% CI for sensitivity was projected (from 6% to 13.5%). To estimate the specificity with greater precision a larger number of unaffected controls (~4:1 ratio to cases) were planned at the sampling stage. This ensured the precision of the estimate of specificity to at least 3%. Accordingly, as the sensitivity and/or specificity increased, the precision of the confidence interval would also increase.

Based on sample size determination, a random sampling plan was devised for the CRO to generate lists of selected samples to sequence (minimum of 110 cases affected by T21, T18, or T13 and 400 non-affected for trisomy, allowing up to half of these to have karyotypes other than 46,XX or 46,XY). Subjects with a singleton pregnancy and an eligible blood sample were eligible for selection. Subjects with ineligible samples, no karyotype recorded, or a multiple gestation were excluded (Figure 20). Lists were generated on a regular basis throughout the study and sent to the Verinata Health laboratory.

Each eligible blood sample was analyzed for six independent categories. The categories were aneuploidy status for chromosomes 21, 18 and 13, and gender status for male, female and monosomy X. While still blinded, one of three classifications (affected, unaffected, or unclassified) were generated prospectively for each of the six independent categories for each plasma DNA sample. Using this scenario, the same sample could be classified as affected in one analysis (e.g., aneuploidy for chromosome 21) and unaffected for another analysis (e.g., euploid for chromosome 18).

Conventional metaphase cytogenetic analysis of cells obtained by chorionic villus sampling (CVS) or amniocentesis was used as the reference standard in this study. Fetal karyotyping was performed in diagnostic laboratories routinely used by the participating sites. If after enrollment a patient underwent both CVS and amniocentesis, karyotype results from amniocentesis were used for study analysis. Fluorescence *in situ* hybridization (FISH) results for targeting chromosomes 21, 18, 13, X, and Y was allowed if a metaphase karyotype was not available (Table 9). All abnormal karyotype reports (*i.e.,* other than 46, XX and 46, XY) were reviewed by a board-certified cytogeneticist and classified as affected or unaffected with respect to chromosomes 21, 18, and 13 and gender status for XX, XY and monosomy X.

Pre-specified protocol conventions defined the following abnormal karyotypes to be assigned a status of 'censored' for karyotype by the cytogeneticist: triploidy, tetraploidy, complex karyotypes other than trisomy (e.g., mosaicism) that involved chromosomes 21, 18, or 13, mosaics with mixed sex chromosomes, sex chromosome aneuploidy or karyotypes that could not be fully interpreted by the source document (e.g. marker chromosomes of unknown origin). Since the cytogenetic diagnosis was not known to the sequencing laboratory, all cytogenetically censored samples were independently analyzed and assigned a classification determined using sequencing information according to the method disclosure herein (Sequencing Classification), but were not included in the statistical analysis. Censored status pertained only to the relevant one or more of the six analyses (e.g., a mosaic T18 would be censored from chromosome 18 analysis, but considered 'unaffected' for other analyses, such as chromosomes 21, 13, X, and Y) (Table 10). Other abnormal and rare complex karyotypes, which could not be fully anticipated at the time of protocol design, were not censored from analysis (Table 11).

The data contained in the eCRF and clinical database were restricted to authorized users only (at the study sites, CROs, and contract clinical personnel). It was not accessible to any employees at Verinata Health until the time of unblinding.

After receiving random sample lists from the CRO, total cell-free DNA (a mixture of maternal and fetal) was extracted from thawed selected plasma samples. Sequencing libraries were prepared utilizing the Illumina TruSeq kit v2.5. Sequencing was carried out (6-plex -, *i.e.,* 6 samples/lane) was performed on an Illumina HiSeq 2000 instrument in the Verinata Health laboratory.-Single-end reads of 36 base pairs were obtained. The reads were mapped across the genome, and the sequence tags on each chromosome of interest were counted and used to classify the sample for independent categories as described above.

The clinical protocol required evidence of fetal DNA presence in order to report a classification result. A classification of male or aneuploid was considered sufficient evidence of fetal DNA. In addition, each sample was also tested for the presence of fetal DNA using two allele specific methods. In the first method, the AmpflSTR Minifiler kit (Life Technologies, San Diego, CA) was used to interrogate the presence of a fetal component in the cell free DNA. Electrophoresis of short tandem repeat (STR) amplicons was carried out on the ABI 3130 Genetic Analyzer following manufacturer's protocols. All nine STR loci in this kit were analyzed by comparing the intensity of each peak reported as a percentage of the sum of the intensities of all peaks, and the presence of minor peaks was used to provide evidence of fetal DNA. In cases in which no minor STR could be identified, an aliquot of the sample was examined with a single nucleotide polymorphism (SNP) panel of 15 SNPs with average heterozygosity ≥ 0.4 selected from the Kidd *et al.* panel (Kidd et al., Forensic Sci Int 164(1):20-32 [2006]). Allele specific methods that can be used to detect and/or quantify fetal DNA in maternal samples are described in U.S. Patent Publications 20120010085, 20110224087, and 20110201507.

Normalized chromosome values (NCVs) were determined by calculating all possible permutations of denominators for all autosomes and sex chromosomes as described above, however, because the sequencing is this study was carried out on a different instrument than our previous work with multiple samples/lane, new normalizing chromosome denominators had to be determined. The normalizing chromosome denominators in the current study were determined based on a training set of 110 independent (*i.e.,* not from MELISSA eligible samples) unaffected samples (*i.e.,* qualified samples) sequenced prior to analysis of the study samples. The new normalizing chromosomes denominators were determined by calculating all possible permutations of denominators for all autosomes and sex chromosomes that minimized the variation for the unaffected training set for all chromosomes across the genome (Table 8).

The NCV rules that were applied to provide the autosome classification of each test sample were those described above. For classification of aneuploidies of autosomes, a NCV > 4.0 was required to classify the chromosome as affected (*i.e.,* aneuploid for that chromosome) and a NCV < 2.5 to classify a chromosome as unaffected. Samples with autosomes that have an NCV between 2.5 and 4.0 were named "unclassified".

Sex chromosome classification in the present test was performed by sequential application of NCVs for both X and Y as follows:

1. If NCV X < -4.0 AND NCV Y < 2.5, then the sample was classified as monosomy X.

2. If NCV X > -2.5 AND NCV X < 2.5 AND NCV Y < 2.5, then the sample was classified as female (XX).

3. If NCV X > 4.0 AND NCV Y < 2.5, then the sample was classified as XXX.

4. If NCV X > -2.5 AND NCV X < 2.5 AND NCV Y > 33, then the sample was classified as XXY.

5. If NCV X < -4.0 AND NCV Y > 4.0, then the sample was classified as male (XY).

6. If condition 5 was met, but NCV Y was approximately 2 times greater than expected for the measured NCV X value, then the sample was classified as XYY.

7. If the chromosome X and Y NCVs did not fit into any of the above criteria, then the sample was classified as unclassified for sex.

Because the laboratory was blinded to the clinical information, the sequencing results were not adjusted for any of the following demographic variables: maternal body mass index, smoking status, presence of diabetes, types of conception (spontaneous or assisted), prior pregnancies, prior aneuploidy, or gestational age. Neither maternal nor paternal samples were utilized for classification, and the classifications according to the present method did not depend on the measurement of specific loci or alleles.

The sequencing results were returned to an independent contract biostatistician prior to unblinding and analysis. Personnel at the study sites, CROs (including the biostatistician generating random sampling lists) and the contract cytogeneticist were blinded to sequencing results.

TABLE 8. **Systematically Determined Normalizing Chromosome Sequences for All Chromosomes**

| Chromosome of Interest | Systematically Determined Normalizing Sequence | Chromosome of Interest | Systematically determined Normalizing Sequence |
|---|---|---|---|
| 1 | 6+10+14+15+17+22 | 13 | 4+6 |
| 2 | 1+3+4+6+8+9+10 | 14 | 1+3+4+5+9+11+15 +17 |
| 3 | +5+6+10+12 | 15 | 1+10+20 |
| 4 | 5 | 16 | 20 |
| 5 | 3+4+8+12 | 17 | 15+19+22 |
| 6 | 2+3+4+14 | 18 | 5+8 |
| 7 | 3+4+6+8+14+16+19 | 19 | 22 |

(continued)

| Chromosome of Interest | Systematically Determined Normalizing Sequence | Chromosome of Interest | Systematically determined Normalizing Sequence |
|---|---|---|---|
| 8 | 5+6+10 | 20 | 15+16+17+22 |
| 9 | 1+2+5+7+8+11+14+15 +16+17+22 | 21 | 4+17+22 |
| 10 | 2+9+15+16+20 | 22 | 19 |
| 11 | 2+8+9+14+16+19+20 | X | 4+5+8 |
| 12 | 1+3+5+6+8+15+19 | Y | 4 |

Statistical methods were documented in a detailed statistical analysis plan for the study. Point estimates for sensitivity and specificity along with exact 95% confidence intervals using the Clopper-Pearson method were computed for each of the six analysis categories. For all statistical estimation procedures performed, samples with no fetal DNA detected, 'censored' for complex karyotype (per protocol-defined conventions), or 'unclassified' by the sequencing test were removed.

## *Results*

Between June 2010 and August 2011, 2,882 pregnant women were enrolled in the study. The characteristics of the eligible subjects and the selected cohort are given in Table 9. Subjects that enrolled and provided blood, but were later found during data monitoring to exceed inclusion criteria and have an actual gestational age at enrollment beyond 22 weeks, 0 days were allowed to remain in the study (n=22) Three of these samples were in the selected set. Figure 20 shows the flow of samples between enrollment and analysis. There were 2,625 samples eligible for selection.

**TABLE 9. Patient Demographics**

| | Eligible Patients ( n=2882) | Analyzed Patients (n=534) | Affected Patients (n=221) |
|---|---|---|---|
| **Maternal Age, yrs** Mean (SD) Min/Max | 35.8 (5.93) 18/49 | 35.2 (6.40) 18/46 | 34.4 (6.73) 18/46 |
| **Multiparous, N (%)** | 2348 (81.5) | 425 (79.5) | 176 (79.6) |
| **Pregnancy by Assisted Reproductive Techniques, N (%)** | 247 (8.6) | 38 (7.1) | 17 (7.7) |
| **Race, N (%)** White African American Asian American Indian or Alaska Native Multi-racial | 2078 (72.1) 338 (11.7) 271 (9.4) 22 (0.8) 173 (6.0) | 388 (72.7) 58 (10.9) 53 (9.9) 5 (0.9) 30 (5.6) | 161 (72.9) 28 (12.7) 18 (8.1) 2 (0.9) 12 (5.4) |
| **BMI**(kg/m$^2$) Mean (SD) Min/Max | 26.6 (5.89) 15/76 | 26.2 (5.73) 17/59 | 26.2 (5.64) 18/56 |
| **Current Smoker, N (%)** | 165 (5.7) | 29 (5.4) | 6 (2.7) |
| **Maternal Diabetes Mellitus, N (%)** | 61 (2.1) | 11 (2.1) | 6(2.7) |
| **Trimester** | | | |

(continued)

| | Eligible Patients ( n=2882) | Analyzed Patients (n=534) | Affected Patients (n=221) |
|---|---|---|---|
| First | 832 (28.9) | 165 (30.9) | 126 (57.0) |
| Second | 2050 (71.1) | 369 (69.1) | 95 (43.0) |
| **Gestational Age (GA)*, wks, days** | 15.5 (3.27) | 15.1 (3.16) | 14.8 (3.18) |
| Mean | 8/31 | 10/23 | 10/23 |
| Min/Max | | | |
| **Karyotype Source, N (%)** | | | |
| CVS | 1044 (36.8) | 228 (42.7) | 121 (54.8) |
| Amniocentesis | 1783 (62.8) | 301 (56.4) | 95 (43.0) |
| Products of Conception | 10 (0.4) | 5 (0.9) | 5 (2.2) |
| **Amniocentesis after CVS, N (%)** | 7 (0.2) | 1 (0.2) | 0(0.0) |
| **Karyotype by FISH-only, N (%)** | 105 (3.6) | 18 (3.4) | 13 (5.9) |
| **Number of Fetuses** 1 | 2797 (97.1) | 534 (100.0) | 221 (100.0) |
| 2 | 76(2.6) | 0 (0.0) | 0(0.0) |
| 3 | 7 (0.2) | 0 (0.0) | 0(0.0) |
| 4 | 2 (0.2) | 0 (0.0) | 0(0.0) |
| **Prenatal Risk, N (%)** | | | |
| AMA only (≥38 years) | 1061 (36.8) | 152 (28.5) | 21 (9.5) |
| Positive screen risk | 622 (21.6) | 91 (17.0) | 14 (6.3) |
| Ultrasound abnormality | 477(6.6) | 122 (22.8) | 81 (36.7)** |
| Prior aneuploidy pregnancy | 82 (2.8) | 15 (2.8) | 4(1.8) |
| More than 1 risk | 640 (22.2) | 154 (28.9) | 101 (45.7)** |
| **Screening Risk Estimated** | 1749 | 310 | 125 |
| **By, N (%)** | 179 (10.2) | 53 (17.1) | 36 (28.8) |
| Nuchal Translucency | 677 (38.7) | 117 (37.7) | 47 (37.6) |
| measure alone | 414 (23.7) | 72 (23.3) | 16 (12.8) |
| First Trimester Combined | 137 (7.8) | 14(4.5) | 3 (2.4) |
| Second Trimester Triple or | 218 (12.5) | 32 (10.3) | 15 (12.0) |
| Quadruple Fully Integrated (1st and 2nd Trimester) Sequential Other | 124 (7.1) | 22 (7.1) | 8 (6.4) |
| **Abnormal Fetal** | 837 (29.0) | 242 (45.3) | 166 (75.1)** |
| **Ultrasound, N (%)** | 719 (24.9) | 212 (39.7) | 143 (64.7) |
| One or more Soft Marker | 228 (7.9) | 79 (15.8) | 65 (29.4) |
| One or more Major Marker | 26 (0.9) | 11 (2.1) | 11 (5.0) |
| IUGR (<10th percentile) Amniotic Fluid Volume Abnormality | 24 (0.8) | 7 (1.3) | 4(1.8) |

*GA at time of invasive procedure.

**Higher penetrance of ultrasound abnormalities in fetuses with abnormal karyotypes Abbreviations: BMI - Body Mass Index, IUGR - Intrauterine growth retardation

Per the random sampling plan, all eligible subjects with an abnormal karyotype were selected for analysis (Figure 20B) as well as a set of subjects carrying euploid fetuses so that the total sequenced study population resulted in an approximately 4:1 ratio of unaffected to affected subjects for trisomies 21. From this process, 534 subjects were selected. Two samples were subsequently removed from analysis due to sample tracking issues in which a full chain of custody between sample tube and data acquisition did not pass quality audit (Figure 20). This resulted in 532 subjects for analysis contributed by 53 of the 60 study sites. The demographics of the selected cohort were similar to the overall cohort.

*Test Performance*

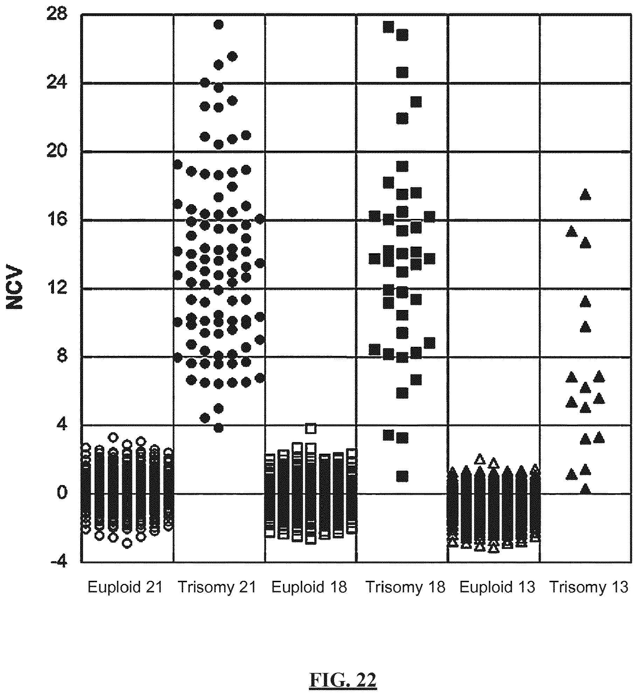

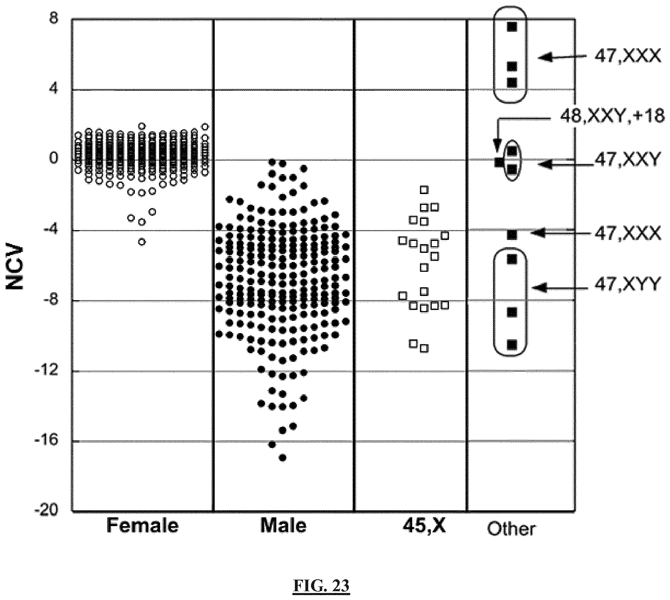

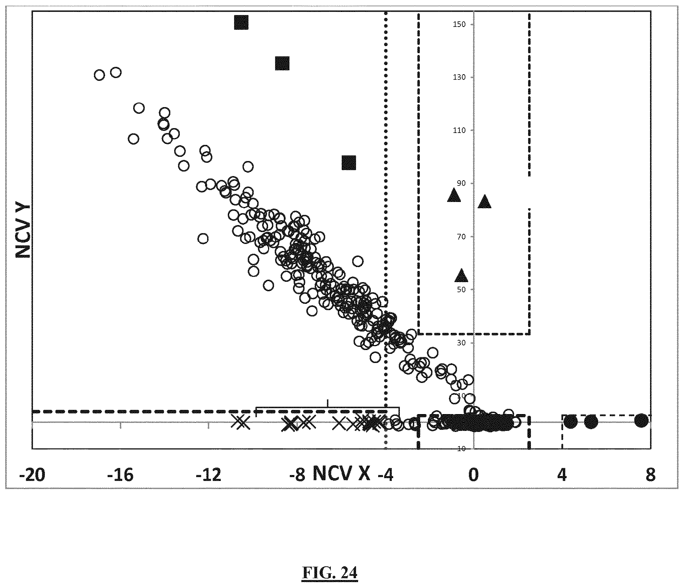

Figures 21A-21C show the flow diagram for aneuploidy analysis of chromosomes 21, 18 and 13 and Figures 21D-21F show gender analysis flow. Table 12 shows the sensitivity, specificity and confidence interval for each of the six analyses, and Figures 22, 23, and 24, show the graphical distribution of samples according to the NCVs following sequencing. In all 6 categories of analysis, 16 samples (3.0%) were removed due to no fetal DNA detected. After unblinding, there were no distinguishing clinical features for these samples. The number of censored karyotypes for each category was dependent on the condition being analyzed (fully detailed in Figure 22).

Sensitivity and specificity of the method to detect T21 in the analysis population (n=493) were 100% (95% CI = 95.9, 100.0) and 100% (95% CI = 99.1, 100.0), respectively (Table 12 and Figure 21A). This included correct classification for one complex T21 karyotype, 47, XX, inv(7)(p22q32),+21, and two translocation T21 arising from Robertsonian translocations one of which was also mosaic for monosomy X (45, X,+21,der(14;21)q10;q10)[4]/46, XY,+21,der(14;21)q10;q10)[17] and 46, XY,÷21,der(21;21)q10;q10).

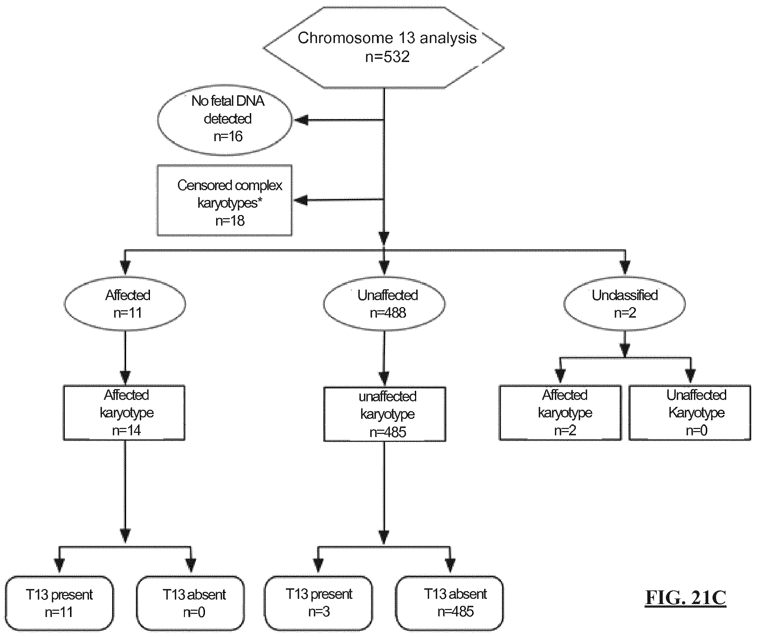

Sensitivity and specificity to detect T18 in the analysis population (n=496) were 97.2% (85.5, 99.9) and 100% (99.2, 100.0) (Table 12 and Figure 21B). Although censored (as per protocol) from the primary analysis, four samples with mosaic karyotype for T21 and T18 were all correctly classified by the method disclosure here as 'affected' for aneuploidy (Table 10). Because they were correctly detected they are indicated on the left side of Figures 21A and 21B. All remaining censored samples were correctly classified as unaffected for trisomies 21, 18, and 13 (Table 10). Sensitivity and specificity to detect T13 in the analysis population were 78.6% (49.2, 99.9) and 100% (99.2, 100.0) (Figure 21C). One T13 case detected arose from a Robertsonian translocation (46, XY,+13,der(13;13)q10;q10). There were seven unclassified samples in the chromosome 21 analysis (1.4%), five in the chromosome 18 analysis (1.0%), and two in the chromosome 13 analysis (0.4%) (Figure 21A-21C). In all categories there was an overlap of three samples that had both a censored karyotype (69,XXX) and no fetal DNA detected. One unclassified sample in the chromosome 21 analysis was correctly identified as T13 in the chromosome 13 analysis and one unclassified sample in the chromosome 18 analysis was correctly identified as T21 in the chromosome 21 analysis.

### TABLE 10. Censored Karyotypes

| Karyotype | Censored Category | Sequencing Classification | Sequencing Classification |
|---|---|---|---|
| | | Aneuploidy | Gender |
| **Mosaic Trisomy 21 and 18 (n=4)** | | | |
| 47,XY,+21[5]/46,XY[12] | 21 | Affected (T21) | Male |
| 47,XX,+21[4]/46,XX [5] | 21 | Affected (T21) | Unclassified |
| 47,XY,+21[21]/48,XY,+21+mar [4]* | 21, 18, 13, gender | Affected (T21) | Male |
| 47,XX,+18 [42] /46, XX [8] | 18 | Affected (T18) | Female |
| Other Complex Mosaicism (n=2) | | | |
| 45,XY,-13[5]/46, XY,r(13) (p11.1q22)[15] | 13 | Unaffected (21,18,13) | Male |
| 92,XXXX[20]/46,XX[61] | 21, 18, 13, gender | Unaffected (21,18, 13) | Unclassified |
| **Added material of uncertain origin (n=5)** | | | |
| 46,XX, add (X)(p22.1) | 21, 18, 13, gender | Unaffected (21,18, 13) | Female |
| 46,XY, add(10)(q26) | 21, 18, 13, gender | Unaffected (21,18, 13) | Male |
| 46,XY,add(15)(p11.2) | 21, 18, 13, gender | Unaffected (21,18, 13) | Male |
| 47,XY,+mar/46,XY | 21, 18, 13, gender | Unaffected (21,18, 13) | Male |
| 47,XX+mar [12]/46,XX[8] | 21, 18, 13, gender | Unaffected (21,18, 13) | Female |
| **Triploidy (n=10)** | | | |

(continued)

| Karyotype | Censored Category | Sequencing Classification Aneuploidy | Sequencing Classification Gender |
|---|---|---|---|
| 69,XXY | 21, 18, 13, gender | Unaffected (21,18, 13) | Unclassified sex |
| 69,XXX (n=9) | 21, 18, 13, gender | Unaffected (21,18, 13) (n=6) Unclassified (n=3) | Female (n=5) Unclassified (n=4) |
| Sex **Chromosome Aneuploidy** **(n=10)** | | | |
| 47,XXX (n=4) | gender | Unaffected (21,18, 13) (n=4) | XXX (n=3) Monosomy X (n=1) |
| 47,XXY (n=3) | gender * | Unaffected (21, 18, 13) (n=2) Unclassified (18) ** and Unaffected (21, 13)(n=1) | XXY (n=2) Unclassified (n=1) |
| 47,XYY (n=3) | gender | Unaffected (21,18, 13) (n=3) | XYY (n=3) |
| **Mosaic Monosomy** X (n=7) | | | |
| 45,X/46,XX (n=3) | gender | Unaffected (21,18, 13) (n=3) | Female (n=2) Monosomy X (n=1) |
| 45,X/47,XXX | gender | Unaffected (21,18, 13) | Monosomy X |
| 45,X/46,XY (n=2) | gender | Unaffected (21,18, 13) (n=2) | Male (n=2) |
| 45,X,+21,der(14;21)(q10;q10)[4]/ 46,XY +21, der(14;21)(q10;q10) [17] | gender | Affected (T21) and Unaffected (18, 13) | Male |
| **Other Reasons (n=3)** | | | |
| Gender not disclosed in report (n=2) | gender | Unaffected (21,18, 13) | Female (n=2) |
| 46,XY with maternal cell contamination (n=1) | gender | Unaffected (21,18, 13) | Male |

*Subject excluded from all analysis categories due to marker chromosome in one cell line.
**Subject with karyotype 48,XXY,+18 was unclassified in chromosome 18 analysis and sex aneuploidy was not detected.

### TABLE 11. Abnormal and complex karyotypes that were not censored

| Karyotype | Sequencing Classification Aneuploidy | Sequencing Classification Gender |
|---|---|---|
| **Monosomy X (n=20)** | | |
| 45,X (n=15) | Unaffected (21, 18, 13) | Monosomy X |
| 45,X (n=4) | Unaffected (21, 18, 13) | Unclassified |
| 45,X (n=1) | Unaffected (21, 18, 13) | Female |
| **Other Autosomal Trisomy or Partial Trisomy (n=5)** | | |
| 47,XX,+16 | Chromosome 16 aneuploidy | Unclassified |
| 47,XX,+20 | Chromosome 20 aneuploidy | Unclassified |
| Partial trisomy 6q12q16.3 and 6q16.3, no gender | Unaffected (21, 18, 13)* | Female |
| 47,XY,+22 | Unaffected (21, 18, 13) | Male |
| 47,XX,+22 | Unclassified (21, 18, 13) | Unclassified |
| **Translocations (n=7)** | | |
| Balanced (n=6) | Unaffected (21, 18, 13) | correct class (Male or Female) |
| Unbalanced (n=1) | Unaffected (21, 18, 13) | Female |

(continued)

| Karyotype | Sequencing Classification Aneuploidy | Sequencing Classification Gender |
|---|---|---|
| **Other Complex Mosaicism (n=4)** | Unaffected (21, 18, 13) | correct class (Male or Female) |
| **Other Complex Variants (n=4)** | Unaffected (21, 18, 13) | correct class (Male or Female) |

*An increased normalized chromosome value (NCV) of 3.6 was noticed from sequencing tags in chromosome 6 after unblinding.

The sex chromosome analysis population for determining performance of the method (female, male, or monosomy X) was 433. Our refined algorithm for classifying the gender status, which allowed for accurate determination of sex chromosome aneuploidies, resulted in a higher number of unclassified results. Sensitivity and specificity for detecting diploid female state (XX) were 99.6% (95% CI = 97.6, >99.9) and 99.5% (95% CI = 97.2, >99.9), respectively; sensitivity and specificity to detect male (XY) were both 100% (95% CI = 98.0, 100.0); and sensitivity and specificity for detecting monosomy X (45,X) were 93.8% (95% CI = 69.8, 99.8) and 99.8% (95% CI = 98.7, >99.9). Although censored from the analysis (as per protocol), the sequencing classifications of mosaic monosomy X karyotypes were as follows (Table 10): 2/7 classified as monosomy X, 3/7 classified with a Y chromosome component classified as XY and 2/7 with XX chromosome component classified as female. Two samples that were classified as monosomy X had karyotypes of 47, XXX and 46, XX. Eight of ten sex chromosome aneuploidies for karyotypes 47, XXX, 47,XXY and 47,XYY were correctly classified (Table 10). If the sex chromosome classifications had been limited to monosomy X, XY and XX, most of the unclassified samples would have been correctly classified as male, but the XXY and XYY sex aneuploidies would not have been identified.

In addition to accurately classifying trisomies 21, 18, 13 and gender, the sequencing results also correctly classified aneuploidy for chromosomes 16 and 20 in two samples (47,XX,+16 and 47,XX,+20) (Table 11). Interestingly, one sample with a clinically complex alteration of the long arm of chromosome 6 (6q) and two duplications, one of which was 37.5Mb in size, showed an increased NCV from sequencing tags in chromosome 6 (NCV=3.6). In another sample, aneuploidy of chromosome 2 was detected according to the method disclosed herein but not observed in the fetal karyotype at amniocentesis (46,XX). Other complex karyotype variants shown in Tables 10 and 11 include samples from fetuses with chromosome inversions, deletions, translocations, triploidy and other abnormalities that were not detected here, but could potentially be classified at higher sequencing density and/or with further algorithm optimization using the method of the disclosure. In these cases, the method correctly classified the samples as unaffected for trisomy 21, 18, or 13 and as male or female.

In this study, 38/532 analyzed samples were from women who underwent assisted reproduction. Of these, 17/38 samples had chromosomal abnormalities; no false positives or false negatives were detected in this sub-population.

**TABLE 12. Sensitivity and Specificity of the Method**

| Performance | Sensitivity (%) | 95% CI | Specificity (%) | 95% CI |
|---|---|---|---|---|
| Trisomy 21 (n=493) | 100.0 (89/89) | 95.9 - 100.0 | 100.0 (404/404) | 99.1 - 100.0 |
| Trisomy 18 (n=496) | 97.2 (35/36) | 85.5 -99.9 | 100 (460/460) | 99.2 - 100.0 |
| Trisomy 13 (n=499) | 78.6 (11/14) | 49.2-99.9 | 100.0 (485/485) | 99.2 - 100.0 |
| Female (n=433) | 99.6 (232/233) | 97.6 - >99.9 | 99.5 (199/200) | 97.2 - >99.9 |
| Male (n=433) | 100.0 (184/184) | 98.0 - 100.0 | 100.0 (249/249) | 98.5 - 100.0 |
| Monosomy X (n=433) | 93.8 (15/16) | 69.8-99.8 | 99.8 (416/417) | 98.7 - >99.9 |

## *Discussion*

This prospective study to determine whole chromosome fetal aneuploidy from maternal plasma was designed to emulate the real world scenario of sample collection, processing and analysis. Whole blood samples were obtained at the enrollment sites, did not require immediate processing, and were shipped overnight to the sequencing laboratory. In contrast to a prior prospective study that only involved chromosome 21 (Palomaki et al., Genetics in Medicine 2011:1), in this study, *all* eligible samples with *any* abnormal karyotype were sequenced and analyzed. The sequencing laboratory did not have prior knowledge of which fetal chromosomes might be affected nor the ratio of aneuploid to euploid samples. The study design recruited a high-risk study population of pregnant women to assure a statistically significant prevalence of aneuploidy, and Tables 10 and 11 indicate the complexity of the karyotypes that were analyzed. The results demonstrate that: i) fetal aneuploidies (including those resulting from translocation trisomy, mosaicism, and complex variations) can be detected with high sensitivity and specificity and ii) aneuploidy in one chromosome does not affect the ability of the method disclosed herein to correctly identify the euploid status of other chromosomes. The algorithms utilized in the previous studies appear to be unable to effectively determine other aneuploidies that inevitably would be present in a general clinical population (Erich et al., Am J Obstet Gynecol 2011 Mar;204(3):205 e1-11, Chiu et al., BMJ 2011;342:c7401).

With regard to mosaicism, the analysis of sequencing information in this study was able to correctly classify samples that had mosaic karyotypes for chromosomes 21 and 18 in 4/4 affected samples. These results demonstrate the sensitivity of the analysis for detecting specific characteristics of cell free DNA in a complex mixture. In one case, the sequencing data for chromosome 2 indicated a whole or partial chromosome aneuploidy while the amniocentesis karyotype result for chromosome 2 was diploid. In two other examples, one sample with 47,XXX karyotype and another with a 46,XX karyotype, the method classified these samples as monosomy X. It is possible these are mosaic cases, or that the pregnant woman herself is mosaic. (It is important to remember that the sequencing is performed on total DNA, which is a combination of maternal and fetal DNA.) While cytogenetic analysis of amniocytes or villi from invasive procedures is currently the reference standard for aneuploidy classification, a karyotype performed on a limited number of cells cannot rule out low-level mosaicism. The current clinical study design did not include long term infant follow-up or access to placental tissue at delivery, so we are unable to determine if these were true or false positive results. We speculate that the specificity of the sequencing process, coupled with optimized algorithms according to the method to detect genome wide variation, may ultimately provide more sensitive identification of fetal DNA abnormalities, particularly in cases of mosaicism, than standard karyotyping.

The International Society for Prenatal Diagnosis has issued a Rapid Response Statement commenting on the commercial availability of massively parallel sequencing (MPS) for prenatal detection of Down syndrome (Benn et al., Prenat Diagn 2012 doi:10.1002/pd.2919). They state that before routine MPS-based population screening for fetal Down syndrome is introduced, evidence is needed that the test performs in some sub-populations, such as in women who conceive by *in vitro* fertilization. The results reported here suggest that the present method is accurate in this group of pregnant women, many of whom are at high risk for aneuploidy.

Although these results demonstrate the excellent performance of the present method with optimized algorithms for aneuploidy detection across the genome in singleton pregnancies from women at increased risk for aneuploidy, more experience, particularly in low-risk populations, is needed to build confidence in the diagnostic performance of the method when the prevalence is low and in multiple gestation. In the early stages of clinical implementation, classification of chromosomes 21, 18 and 13 using sequencing information according to the present method should be utilized after a positive first or second trimester screening result. This will reduce unnecessary invasive procedures caused by the false positive screening results, with a concomitant reduction in procedure related adverse events. Invasive procedures could be limited to confirmation of a positive result from sequencing. However, that there are clinical scenarios (e.g., advanced maternal age and infertility) in which pregnant women will want to avoid an invasive procedure; they may request this test as an alternative to the primary screen and/or invasive procedure. All patients should receive thorough pre-test counseling to ensure that they understand the limitations of the test and the implications of the results. As experience accumulates with more samples, it is possible that this test will replace current screening protocols and become a primary screening and ultimately a noninvasive diagnostic test for fetal aneuploidy.

## Claims

1. A method, implemented at a computer system that includes one or more processors and system memory, for generating a masked reference sequence of the Y chromosome for evaluating copy number of the Y chromosome, the method comprising:

   (a) providing, on the computer system, a training set of genomic reads measured from nucleic acid samples of a first plurality of female individuals;
   (b) aligning, by the computer system, genomic reads of the training set to a reference genome comprising a reference sequence of the Y-chromosome, thereby providing training sequence tags comprising aligned genomic reads and their locations on the reference sequence of the Y chromosome;

(c) dividing, by the computer system, the reference sequence of the Y chromosome into a plurality of bins;
(d) determining, by the computer system, counts of training sequence tags located in each bin; and
(e) masking, by the computer system, bins that exceed a masking threshold, the masking threshold being based on the counts of training sequence tags in each bin, thereby generating the masked reference sequence of the Y chromosome.

2. A method, implemented at a computer system that includes one or more processors and system memory, for evaluation of copy number of the Y chromosome in a test sample, the method comprising:

(a) providing, on the computer system, a training set comprising genomic reads measured from nucleic acid samples of a first plurality of female individuals;
(b) aligning, by the computer system, genomic reads of the training set to a reference genome comprising a reference sequence of the Y-chromosome, thereby providing training sequence tags comprising aligned genomic reads and their locations on the reference sequence of the Y chromosome;
(c) dividing, by the computer system, the reference sequence of the Y chromosome into a plurality of bins;
(d) determining, by the computer system, counts of training sequence tags located in each bin;
(e) masking, by the computer system, bins that exceed a masking threshold, the masking threshold being based on the counts of training sequence tags in each bin, thereby providing a masked reference sequence of the Y chromosome; and
(f) evaluating, by the computer system, copy number of the Y chromosome of the fetus in the test sample, comprising:

(i) sequencing cell-free nucleic acids from a test sample comprising fetal and maternal cell-free nucleic acids obtained from a pregnant female carrying a fetus, thereby generating genomic reads of the test sample;
(ii) aligning, by the computer system, the genomic reads of the test sample to an unmasked reference sequence or to the masked reference sequence, thereby providing testing sequence tags comprising aligned genomic reads and locations thereof;
(iii) measuring, by the computer system, counts of the testing sequence tags having locations on unmasked segments of the masked reference sequence of the Y chromosome; and
(iv) evaluating, by the computer system, copy number of the Y chromosome of the fetus in the test sample based on the counts of the testing sequence tags having locations on unmasked segments of the masked reference sequence of the Y chromosome.

3. The method of claim 1 or claim 2, wherein the masking threshold is determined by:

(a) providing, on the computer system, two or more masking threshold candidates;
(b) masking, by the computer system, bins that exceed the masking threshold candidates, thereby providing two or more masked reference sequences;
(c) calculating, by the computer system, a threshold evaluation index for evaluation of copy number of a genetic sequence of interest based on each of the two or more masked reference sequences; and
(d) selecting, on the computer system, the candidate having the highest threshold evaluation index as the masking threshold.

4. The method of claim 3, wherein calculating the threshold evaluation index comprises: evaluating copy number of the Y chromosome for nucleic acid samples of (a) female individuals different from the first plurality of female individuals, and (b) male individuals known to have a Y chromosome; and optionally calculating the threshold evaluation index as the difference between the means of (a) and (b), divided by the standard deviation of (a).

5. The method of claim 1 or claim 2, wherein:

(i) a size of each of said plurality of bins is determined by:

dividing, by the computer system, the reference sequence of the Y chromosome into bins of a candidate bin size;
calculating, by the computer system, a bin evaluation index based on the candidate bin size;
iteratively repeating the preceding steps of this claim on the computer system using different candidate bin sizes, thereby yielding two or more different evaluation indices; and
selecting, on the computer system, the candidate bin size yielding the highest bin evaluation index as the size of the bins;

(ii) the first plurality of female individuals have diverse alignment profiles **characterized by** different distributions of the genomic reads on the reference sequence of the Y chromosome; or
(iii) the method comprises in step (b) aligning, by the computer system, at least about 100,000 genomic reads per individual of the training set to a reference genome comprising a reference sequence of the Y-chromosome.

6. The method of claim 5(ii), wherein the providing a training set comprises dividing a second plurality of female individuals into two or more clusters and selecting a number of individuals in each of the two or more clusters to form the first plurality of female individuals, and wherein optionally:

(i) selecting a number of individuals in each of the two or more clusters comprises selecting an equal number of individuals in each of the two or more clusters; or
(ii) the dividing said second plurality of female individuals into two or more clusters comprises hierarchical ordered partitioning and collapsing hybrid (HOPACH) clustering.

7. The method of claim 1 or claim 2, wherein:

(i) the genomic reads comprise sequences of about 20 to 50-bp from anywhere in the entire genome of an individual;
(ii) the bin size is smaller than about 2000 bp; or
(iii) the masking threshold is at least about 90th percentile of sequence tag counts.

8. The method of claim 1, further comprising:

(i) sequencing cell-free nucleic acids from a test sample comprising fetal and maternal cell-free nucleic acids obtained from a pregnant female carrying a fetus, thereby generating genomic reads of the test sample;
(ii) aligning, by the computer system, the genomic reads of the test sample to an unmasked reference sequence or to the masked reference sequence, thereby providing testing sequence tags comprising aligned genomic reads and locations thereof;
(iii) measuring, by the computer system, counts of the testing sequence tags having locations on unmasked segments of the masked reference sequence of the Y chromosome; and
(iv) evaluating, by the computer system, copy number of the Y chromosome of the fetus in the test sample based on the counts of the testing sequence tags having locations on unmasked segments of the masked reference sequence of the Y chromosome.

9. The method of claim 2 or 8, wherein step (iv) comprises:

calculating a chromosome dose from the counts of the testing sequence tags on the masked reference sequence of the Y chromosome; and
evaluating copy number of the Y chromosome in the test sample based on the chromosome dose and data from control samples.

10. The method of claim 9, wherein the chromosome dose is calculated as a ratio between (a) coverage of the testing sequence tags on the masked reference sequence of the Y chromosome, and (b) coverage of one or more normalizing sequences.

11. The method of claim 9, further comprising:

calculating a normalized chromosome value from the chromosome dose and data from control samples; and
evaluating copy number of the Y chromosome in the test sample based on the normalized chromosome value.

12. The method of claim 2 or 8, wherein step (iv) comprises:

(b) determining the presence or absence of Y chromosome in the genome of the fetal cell-free nucleic acids; or
(c) determining the presence or absence of at least one fetal aneuploidy.

13. A system for evaluation of copy number of a genetic sequence of interest in a test sample comprising fetal and maternal cell-free nucleic acids obtained from a pregnant female, the system comprising:

> a sequencer for receiving nucleic acids from the test sample providing nucleic acid sequence information from the sample;
> a processor; and
> one or more computer-readable storage media having stored thereon instructions for execution on said processor to perform the method of any of claims 1-12.

14. A computer program product comprising one or more computer-readable non-transitory storage media having stored thereon computer-executable instructions that, when executed by one or more processors of a computer system, cause the computer system to implement the method of any of claims 1-12.

**Patentansprüche**

1. Verfahren, das auf einem Computersystem implementiert wird, das einen oder mehrere Prozessoren und Systemspeicher umfasst, zum Erzeugen einer maskierten Referenzsequenz des Y-Chromosoms zum Auswerten einer Kopienzahl des Y-Chromosoms, wobei das Verfahren umfasst:

> (a) Bereitstellen, auf dem Computersystem, einer Trainingsgruppe von Genomabschnitten, die anhand von Nukleinsäureproben einer ersten Vielzahl an weiblichen Individuen gemessen werden;
> (b) Abgleichen, durch das Computersystem, von Genomabschnitten der Trainingsgruppe mit einem Referenzgenom, das eine Referenzsequenz des Y-Chromosoms umfasst, wodurch Trainingssequenzmarkierungen bereitgestellt werden, die abgeglichene Genomabschnitte und deren Orte in der Referenzsequenz des Y-Chromosoms umfassen;
> (c) Aufteilen, durch das Computersystem, der Referenzsequenz des Y-Chromosoms in mehrere Behälter;
> (d) Bestimmen, durch das Computersystem, von Zahlen von Trainingssequenzmarkierungen, die in jedem Behälter liegen; und
> (e) Maskieren, durch das Computersystem, von Behältern, die einen Maskierungsschwellenwert überschreiten, wobei der Maskierungsschwellenwert auf den Zahlen von Trainingssequenzmarkierungen in jedem Behälter basiert, wodurch die maskierte Referenzsequenz des Y-Chromosoms erzeugt wird.

2. Verfahren, das auf einem Computersystem implementiert wird, das einen oder mehrere Prozessoren und Systemspeicher umfasst, zum Auswerten einer Kopienzahl des Y-Chromosoms in einer Testprobe, wobei das Verfahren umfasst:

> (a) Bereitstellen, auf dem Computersystem, einer Trainingsgruppe, die Genomabschnitte umfasst, die anhand von Nukleinsäureproben einer ersten Vielzahl an weiblichen Individuen gemessen werden;
> (b) Abgleichen, durch das Computersystem, von Genomabschnitten der Trainingsgruppe mit einem Referenzgenom, das eine Referenzsequenz des Y-Chromosoms umfasst, wodurch Trainingssequenzmarkierungen bereitgestellt werden, die abgeglichene Genomabschnitte und deren Orte in der Referenzsequenz des Y-Chromosoms umfassen;
> (c) Aufteilen, durch das Computersystem, der Referenzsequenz des Y-Chromosoms in mehrere Behälter;
> (d) Bestimmen, durch das Computersystem, von Zahlen von Trainingssequenzmarkierungen, die in jedem Behälter liegen;
> (e) Maskieren, durch das Computersystem, von Behältern, die einen Maskierungsschwellenwert überschreiten, wobei der Maskierungsschwellenwert auf den Zahlen von Trainingssequenzmarkierungen in jedem Behälter basiert, wodurch eine maskierte Referenzsequenz des Y-Chromosoms bereitgestellt wird; und
> (f) Auswerten, durch das Computersystem, der Kopienzahl des Y-Chromosoms des Fötus in der Testprobe, umfassend:
>
>> (i) Sequenzieren von zellfreien Nukleinsäuren aus einer Testprobe, die fetale und mütterliche zellfreie Nukleinsäuren umfasst, die von einer schwangeren Frau erhalten werden, die einen Fötus trägt, wodurch Genomabschnitte der Testprobe erzeugt werden;
>> (ii) Abgleichen, durch das Computersystem, der Genomabschnitte der Testprobe mit einer unmaskierten Referenzsequenz oder mit der maskierten Referenzsequenz, wodurch Testsequenzmarkierungen bereitgestellt werden, die abgeglichene Genomabschnitte und Orte davon umfassen;
>> (iii) Messen, durch das Computersystem, von Zahlen der Testsequenzmarkierungen, die Orte auf unmaskierten Segmenten der maskierten Referenzsequenz des Y-Chromosoms aufweisen; und

(iv) Auswerten, durch das Computersystem, der Kopienzahl des Y-Chromosoms des Fötus in der Testprobe basierend auf den Zahlen der Testsequenzmarkierungen, die Orte auf unmaskierten Segmenten der maskierten Referenzsequenz des Y-Chromosoms aufweisen.

3. Verfahren nach Anspruch 1 oder Anspruch 2, wobei der Maskierungsschwellenwert bestimmt wird durch:

(a) Bereitstellen, auf dem Computersystem, von zwei oder mehr Maskierungsschwellenwertkandidaten;
(b) Maskieren, durch das Computersystem, von Behältern, die die Maskierungsschwellenwertkandidaten überschreiten, wodurch zwei oder mehr maskierte Referenzsequenzen bereitgestellt werden;
(c) Berechnen, durch das Computersystem, eines Schwellenwertauswertungsindexes zur Auswertung der Kopienzahl einer genetischen Sequenz von Interesse, basierend auf jeder der zwei oder mehr maskierten Referenzsequenzen; und
(d) Auswählen, auf dem Computersystem, des Kandidaten, der den höchsten Schwellenwertauswertungsindex aufweist, als Maskierungsschwellenwert.

4. Verfahren nach Anspruch 3, wobei das Berechnen des Schwellenwertauswertungsindexes umfasst: Auswerten der Kopienzahl des Y-Chromosoms für Nukleinsäureproben von (a) weiblichen Individuen, die von der ersten Vielzahl an weiblichen Individuen verschieden sind, und (b) männlichen Individuen, in Bezug auf welche bekannt ist, dass sie ein Y-Chromosom aufweisen; und wahlweise Berechnen des Schwellenwertauswertungsindexes als Differenz zwischen dem Mittelwert von (a) und (b) geteilt durch die Standardabweichung von (a).

5. Verfahren nach Anspruch 1 oder Anspruch 2, wobei:

(i) eine Größe jedes der mehreren Behälter bestimmt wird durch:

Aufteilen, durch das Computersystem, der Referenzsequenz des Y-Chromosoms in Behälter einer Kandidatenbehältergröße;
Berechnen, durch das Computersystem, eines Behälterauswertungsindexes basierend auf der Kandidatenbehältergröße;
iteratives Wiederholen der vorherigen Schritte dieses Anspruchs auf dem Computersystem unter Verwendung verschiedener Kandidatenbehältergrößen, wodurch zwei oder mehr verschiedene Auswertungsindizes geliefert werden; und
Auswählen, auf dem Computersystem, der Kandidatenbehältergröße, die den höchsten Behälterauswertungsindex liefert, als Größe der Behälter;

(ii) die erste Vielzahl an weiblichen Individuen verschiedene Abgleichungsprofile aufweisen, die durch verschiedene Verteilungen der Genomabschnitte auf der Referenzsequenz des Y-Chromosoms gekennzeichnet sind; oder

(iii) das Verfahren in Schritt (b) umfasst: Abgleichen, durch das Computersystem, von wenigstens ungefähr 100.000 Genomabschnitten pro Individuum der Trainingsgruppe mit einem Referenzgenom, das eine Referenzsequenz des Y-Chromosoms umfasst.

6. Verfahren nach Anspruch 5 (ii), wobei das Bereitstellen einer Trainingsgruppe umfasst, eine zweite Vielzahl an weiblichen Individuen in zwei oder mehr Cluster aufzuteilen und eine Anzahl an Individuen in jedem der zwei oder mehr Cluster auszuwählen, um die erste Vielzahl an weiblichen Individuen zu bilden, und wobei wahlweise:

(i) das Auswählen einer Anzahl an Individuen in jedem der zwei oder mehr Cluster das Auswählen einer gleichen Anzahl an Individuen in jedem der zwei oder mehr Cluster umfasst; oder
(ii) das Aufteilen der zweiten Vielzahl an weiblichen Individuen in zwei oder mehr Cluster hierarchisches (HOPACH, Hierarchichal Ordered Partitioning and Collapsing Hybrid) Clustering umfasst.

7. Verfahren nach Anspruch 1 oder Anspruch 2, wobei:

(i) die Genomabschnitte Sequenzen von ungefähr 20 bis 50 bp von einer beliebigen Stelle im gesamten Genom eines Individuums umfassen;
(ii) die Behältergröße kleiner als ungefähr 2000 bp ist; oder
(iii) der Maskierungsschwellenwert wenigstens ungefähr das 90ste Perzentil der Sequenzmarkierungszahlen ist.

**8.** Verfahren nach Anspruch 1, ferner umfassend:

(i) Sequenzieren von zellfreien Nukleinsäuren aus einer Testprobe, die fetale und mütterliche zellfreie Nukleinsäuren umfasst, die von einer schwangeren Frau erhalten werden, die einen Fötus trägt, wodurch Genomabschnitte der Testprobe erzeugt werden;
(ii) Abgleichen, durch das Computersystem, der Genomabschnitte der Testprobe mit einer unmaskierten Referenzsequenz oder mit der maskierten Referenzsequenz, wodurch Testsequenzmarkierungen bereitgestellt werden, die abgeglichene Genomabschnitte und Orte davon umfassen;
(iii) Messen, durch das Computersystem, von Zahlen der Testsequenzmarkierungen, die Orte auf unmaskierten Segmenten der maskierten Referenzsequenz des Y-Chromosoms aufweisen; und
(iv) Auswerten, durch das Computersystem, der Kopienzahl des Y-Chromosoms des Fötus in der Testprobe basierend auf den Zahlen der Testsequenzmarkierungen, die Orte auf unmaskierten Segmenten der maskierten Referenzsequenz des Y-Chromosoms aufweisen.

**9.** Verfahren nach Anspruch 2 oder 8, wobei Schritt (iv) umfasst:

Berechnen einer Chromosomendosis anhand der Zahlen der Testsequenzmarkierungen auf der maskierten Referenzsequenz des Y-Chromosoms; und
Auswerten der Kopienzahl des Y-Chromosoms in der Testprobe basierend auf der Chromosomendosis und Daten von Kontrollproben.

**10.** Verfahren nach Anspruch 9, wobei die Chromosomendosis als ein Verhältnis zwischen (a) der Abdeckung der Testsequenzmarkierungen auf der maskierten Referenzsequenz des Y-Chromosoms und (b) der Abdeckung einer oder mehrerer Normalisierungssequenzen berechnet wird.

**11.** Verfahren nach Anspruch 9, ferner umfassend:

Berechnen eines normalisierten Chromosomenwerts anhand der Chromosomendosis und von Daten von Kontrollproben; und
Auswerten der Kopienzahl des Y-Chromosoms in der Testprobe basierend auf dem normalisierten Chromosomenwert.

**12.** Verfahren nach Anspruch 2 oder 8, wobei Schritt (iv) umfasst:

(b) Bestimmen des Vorhandenseins oder Nichtvorhandenseins des Y-Chromosoms im Genom der fetalen zellfreien Nukleinsäuren; oder
(c) Bestimmen des Vorhandenseins oder Nichtvorhandenseins von wenigstens einer fetalen Aneuploidie.

**13.** System zur Auswertung der Kopienzahl einer genetischen Sequenz von Interesse in einer Testprobe, die fetale und mütterliche zellfreie Nukleinsäuren umfasst, die von einer schwangeren Frau erhalten werden, wobei das System umfasst:

einen Sequenzierer zum Erhalten von Nukleinsäuren aus der Testprobe unter Bereitstellung von Nukleinsäuresequenzinformationen anhand der Probe;
einen Prozessor; und
ein oder mehrere computerlesbare Speichermedien, auf denen Anweisungen zur Ausführung auf dem Prozessor gespeichert sind, um das Verfahren nach einem der Ansprüche 1-12 durchführen.

**14.** Computerprogrammprodukt, das ein oder mehrere computerlesbare, nichttransitorische Speichermedien umfasst, auf denen von einem Computer ausführbare Befehle gespeichert sind, die, wenn sie von einem oder mehreren Prozessoren eines Computersystems ausgeführt werden, bewirken, dass das Computersystem das Verfahren nach einem der Ansprüche 1-12 implementiert.

**Revendications**

**1.** Procédé, mis en œuvre sur un système informatique qui comprend un ou plusieurs processeurs et une mémoire de système, pour générer une séquence de référence masquée du chromosome Y pour évaluer le nombre d'exemplaires du chromosome Y, le procédé comprenant :

(a) la fourniture, sur le système informatique, d'un ensemble d'apprentissage de lectures génomiques mesurées à partir d'échantillons d'acide nucléique d'une première pluralité d'individus de sexe féminin ;
(b) l'alignement, par le système informatique, de lectures génomiques de l'ensemble d'apprentissage sur un génome de référence comprenant une séquence de référence du chromosome Y, fournissant ainsi des marqueurs de séquence d'apprentissage comprenant des lectures génomiques alignées et leurs emplacements sur la séquence de référence du chromosome Y ;
(c) la division, par le système informatique, de la séquence de référence du chromosome Y en une pluralité de cases ;
(d) la détermination, par le système informatique, de nombres de marqueurs de séquence d'apprentissage situés dans chaque case; et
(e) le masquage, par le système informatique, de cases qui dépassent un seuil de masquage, le seuil de masquage étant basé sur les nombres de marqueurs de séquence d'apprentissage dans chaque case, générant ainsi la séquence de référence masquée du chromosome Y.

2. Procédé, mis en œuvre sur un système informatique qui comprend un ou plusieurs processeurs et une mémoire de système, pour l'évaluation du nombre d'exemplaires du chromosome Y dans un échantillon de test, le procédé comprenant :

(a) la fourniture, sur le système informatique, d'un ensemble d'apprentissage comprenant des lectures génomiques mesurées à partir d'échantillons d'acide nucléique d'une première pluralité d'individus de sexe féminin ;
(b) l'alignement, par le système informatique, de lectures génomiques de l'ensemble d'apprentissage sur un génome de référence comprenant une séquence de référence du chromosome Y, fournissant ainsi des marqueurs de séquence d'apprentissage comprenant des lectures génomiques alignées et leurs emplacements sur la séquence de référence du chromosome Y ;
(c) la division, par le système informatique, de la séquence de référence du chromosome Y en une pluralité de cases ;
(d) la détermination, par le système informatique, de nombres de marqueurs de séquence d'apprentissage situés dans chaque case ;
(e) le masquage, par le système informatique, de cases qui dépassent un seuil de masquage, le seuil de masquage étant basé sur les nombres de marqueurs de séquence d'apprentissage dans chaque case, fournissant ainsi une séquence de référence masquée du chromosome Y ; et
(f) l'évaluation, par le système informatique, du nombre d'exemplaires du chromosome Y du fœtus dans l'échantillon de test, comprenant :

(i) le séquençage d'acides nucléiques dépourvus de cellules provenant d'un échantillon de test comprenant des acides nucléiques dépourvus de cellules fœtales et maternelles obtenus d'une femme enceinte portant un foetus, générant ainsi des lectures génomiques de l'échantillon de test ;
(ii) l'alignement, par le système informatique, des lectures génomiques de l'échantillon de test sur une séquence de référence non masquée ou sur la séquence de référence masquée, fournissant ainsi des marqueurs de séquence de test comprenant des lectures génomiques alignées et leurs emplacements ;
(iii) la mesure, par le système informatique, de nombres des marqueurs de séquence de test ayant des emplacements sur des segments non masqués de la séquence de référence masquée du chromosome Y ; et
(iv) l'évaluation, par le système informatique, du nombre d'exemplaires du chromosome Y du fœtus dans l'échantillon de test sur la base des comptes des marqueurs de séquence de test ayant des emplacements sur des segments non masqués de la séquence de référence masquée du chromosome Y.

3. Procédé selon la revendication 1 ou selon la revendication 2, le seuil de masquage étant déterminé par :

(a) la fourniture, sur le système informatique, de deux ou plus de deux candidats de seuil de masquage ;
(b) le masquage, par le système informatique, des cases qui dépassent les candidats de seuil de masquage, fournissant ainsi deux ou plus de deux séquences de référence masquées ;
(c) le calcul, par le système informatique, d'un indice d'évaluation de seuil pour l'évaluation du nombre d'exemplaires d'une séquence génétique d'intérêt sur la base de chacune des deux ou plus de deux séquences de référence masquées ; et
(d) la sélection, sur le système informatique, du candidat ayant l'indice d'évaluation de seuil le plus élevé comme seuil de masquage.

**4.** Procédé selon la revendication 3, le calcul de l'indice d'évaluation de seuil comprenant : l'évaluation du nombre d'exemplaires du chromosome Y pour des échantillons d'acide nucléique (a) d'individus de sexe féminin différents de la première pluralité d'individus de sexe féminin, et (b) d'individus de sexe masculin connus pour avoir un chromosome Y ; et éventuellement le calcul de l'indice d'évaluation de seuil en tant que différence entre les moyennes de (a) et (b), divisée par l'écart type de (a).

**5.** Procédé selon la revendication 1 ou selon la revendication 2,

(i) une taille de chacune de ladite pluralité de cases étant déterminée par :

la division, par le système informatique, de la séquence de référence du chromosome Y en cases d'une taille de case candidate ;
le calcul, par le système informatique, d'un indice d'évaluation de case basé sur la taille de la case candidate ;
la répétition de manière itérative des étapes précédentes de cette revendication sur le système informatique à l'aide de différentes tailles de case candidates, produisant ainsi deux ou plus de deux indices d'évaluation différents ; et
la sélection, sur le système informatique, de la taille de case candidate produisant l'indice d'évaluation de case le plus élevé en tant que taille des cases ;

(ii) la première pluralité d'individus de sexe féminin ayant divers profils d'alignement **caractérisés par** différentes distributions des lectures génomiques sur la séquence de référence du chromosome Y ; ou
(iii) le procédé comprenant à l'étape (b) l'alignement, par le système informatique, d'au moins environ 100 000 lectures génomiques par individu de l'ensemble d'apprentissage sur un génome de référence comprenant une séquence de référence du chromosome Y.

**6.** Procédé selon la revendication 5(ii), la fourniture d'un ensemble d'apprentissage comprenant la division d'une seconde pluralité d'individus de sexe féminin en deux ou plus de deux groupes et la sélection d'un nombre d'individus dans chacun des deux ou plus de deux groupes pour former la première pluralité d'individus de sexe féminin, et, de manière facultative :

(i) la sélection d'un nombre d'individus dans chacun des deux ou plus de deux groupes comprenant la sélection d'un nombre égal d'individus dans chacun des deux ou plus de deux groupes ; ou
(ii) la division de ladite seconde pluralité d'individus de sexe féminin en deux ou plus de deux groupes comprenant le regroupement des subdivisions hiérarchiques ordonnées et la disparition des hybrides (HOPACH - *hierarchical ordered partitioning and collapsing hybrid*).

**7.** Procédé selon la revendication 1 ou selon la revendication 2,

(i) les lectures génomiques comprenant des séquences d'environ 20 à 50 pb provenant de n'importe quelle partie de l'ensemble du génome entier d'un individu ;
(ii) la taille de case étant inférieure à environ 2 000 pb ; ou
(iii) le seuil de masquage étant au moins égal au 90e percentile de nombres de marqueurs de séquence.

**8.** Procédé selon la revendication 1, comprenant en outre :

(i) le séquençage d'acides nucléiques dépourvus de cellules provenant d'un échantillon de test comprenant des acides nucléiques dépourvus de cellules fœtales et maternelles obtenus d'une femme enceinte portant un fœtus, générant ainsi des lectures génomiques de l'échantillon de test ;
(ii) l'alignement, par le système informatique, des lectures génomiques de l'échantillon de test sur une séquence de référence non masquée ou sur la séquence de référence masquée, fournissant ainsi des marqueurs de séquence de test comprenant des lectures génomiques alignées et leurs emplacements ;
(iii) la mesure, par le système informatique, de nombres des marqueurs de séquence de test ayant des emplacements sur des segments non masqués de la séquence de référence masquée du chromosome Y ; et
(iv) l'évaluation, par le système informatique, du nombre d'exemplaires du chromosome Y du fœtus dans l'échantillon de test sur la base des nombres des marqueurs de séquence de test ayant des emplacements sur des segments non masqués de la séquence de référence masquée du chromosome Y.

**9.** Procédé selon la revendication 2 ou 8, l'étape (iv) comprenant :

le calcul d'une dose de chromosome à partir des nombres des marqueurs de séquence de test sur la séquence de référence masquée du chromosome Y ; et

l'évaluation du nombre d'exemplaires du chromosome Y dans l'échantillon de test sur la base de la dose de chromosome et des données provenant des échantillons de contrôle.

10. Procédé selon la revendication 9, la dose de chromosome étant calculée en tant que rapport entre (a) la couverture des marqueurs de séquence de test sur la séquence de référence masquée du chromosome Y, et (b) la couverture d'une ou plusieurs séquences de normalisation.

11. Procédé selon la revendication 9, comprenant en outre :

le calcul d'une valeur chromosomique normalisée à partir de la dose de chromosome et des données provenant des échantillons de contrôle ; et

l'évaluation du nombre d'exemplaires du chromosome Y dans l'échantillon de test sur la base de la valeur chromosomique normalisée.

12. Procédé selon la revendication 2 ou 8, l'étape (iv) comprenant :

(b) la détermination de la présence ou de l'absence du chromosome Y dans le génome des acides nucléiques dépourvus de cellules fœtales ; ou

(c) la détermination de la présence ou de l'absence d'au moins une aneuploïdie fœtale.

13. Système d'évaluation du nombre d'exemplaires d'une séquence génétique d'intérêt dans un échantillon de test comprenant des acides nucléiques dépourvus de cellules fœtales et maternelles obtenus d'une femme enceinte, le système comprenant :

un séquenceur pour recevoir des acides nucléiques provenant de l'échantillon de test fournissant des informations de séquence d'acide nucléique à partir de l'échantillon ;

un processeur ; et

un ou plusieurs supports de stockage lisibles par ordinateur sur lesquels sont stockées des instructions destinées à être exécutées sur ledit processeur pour réaliser le procédé selon l'une quelconque des revendications 1 à 12.

14. Produit de programme informatique comprenant un ou plusieurs supports de stockage non-transitoires lisibles par ordinateur sur lesquels sont stockées des instructions exécutables par ordinateur qui, lorsqu'elles sont exécutées par un ou plusieurs processeurs d'un système informatique, amènent le système informatique à mettre en œuvre le procédé selon l'une quelconque des revendications 1 à 12.

**FIG. 1**

**Geography of masked chrY bins**

**FIG. 2**

200

**Evaluation of copy number of the Y chromosome in a test sample comprising fetal and maternal cell-free nucleic acids**

210

Provide a training set comprising genomic reads measured from nucleic acid samples of a first plurality of female individuals

220

Align genomic reads of the training set to a reference sequence of the Y-chromosome

230

Provide training sequence tags comprising aligned genomic reads and their locations on the reference sequence of the Y chromosome

240

Divide the reference sequence into bins of a specific size

250

Determine counts of training sequence tags located in each bin

260

Mask bins that exceed a filtering threshold, thereby providing a masked reference sequence of the Y chromosome

**FIG. 3A**

200

**Evaluation of copy number of the Y chromosome in a test sample comprising fetal and maternal cell-free nucleic acids**

260

Provide a masked reference sequence of the Y chromosome

262

Sequence the cell free nucleic acids from the test sample using a sequencer, thereby generating genomic reads of the test sample

264

Align the genomic reads of the test sample to the reference sequence

266

Provide testing sequence tags comprising aligned genomic reads and locations thereof

268

Measure counts of the testing sequence tags on the masked reference sequence of the Y chromosome;

270

Evaluate copy number of the Y chromosome in the test sample based on the counts of the testing sequence tags on the masked reference sequence

**FIG. 3B**

100

110
Obtained qualified samples comprising nucleic acids

115
Obtained test sample comprising nucleic acid

120
Sequence at least a portion of the qualified nucleic acids

125
Sequence at least a portion of test nucleic acids

130
Determine qualified sequence tag densities

135
Determine test sequence tag densities

145
Identify qualified normalizing sequence

150
Determine a test sequence dose based on test sequence tag densities of a sequence of interest and the corresponding normalizing sequence

155
Determine Thresholds

160
Compare test sequence dose to threshold value

165
Determine presence or absence of copy number variation

**FIG. 4**

Call/Diagnosis

Network/Cloud
(e.g. Internet)

05

Sample
Collection

Processing
and
Sequencing

Analysis
and
Call
Generation

01

03

07

FIG. 5

**FIG. 6**

**FIG. 7A**

**FIG. 7B**

**FIG. 8**

**FIG. 9**

**FIG. 10**

Frequency of observing female [light gray] and male[darkgray] hits across chr Y

FIG. 11

**FIG. 12A**

FIG. 12B

**Chromosome 18**

**FIG. 13A**

## Chromosome 18

**FIG. 13B**

**Chromosome 13**

**FIG. 14A**

**Chromosome 13**

FIG. 14B

**Chromosome X**

**FIG. 15A**

**Chromosome X**

**FIG. 15B**

Chromosome Y

**FIG. 16A**

Chromosome Y

**FIG. 16B**

**FIG. 17**

FIG. 18

**Chromosome 21**

FIG. 19A

## Chromosome 18

**FIG. 19B**

FIG. 19C

# Chromosome X

**FIG. 19D**

**Chromosome Y**

Average(chrs 1–22,X)

Chromosome (denominator)

XY  ○        XX  △        XO  +

**FIG. 19E**

**FIG. 20A**

**FIG. 20B**

FIG. 21A

FIG. 21B

Chromosome 13 analysis
n=532

No fetal DNA detected
n=16

Censored complex karyotypes*
n=18

Unclassified
n=2

Unaffected Karyotype
n=0

Affected karyotype
n=2

Unaffected
n=488

unaffected karyotype
n=485

T13 absent
n=485

T13 present
n=3

Affected
n=11

Affected karyotype
n=14

T13 absent
n=0

T13 present
n=11

**FIG. 21C**

**FIG. 21D**

Female analysis n=532

No fetal DNA detected n=16

Censored complex karyotypes* n=37

Unclassified n=49 → Not female Karyotype n=43; Female karyotype n=6

Not female n=200 → Not female karyotype n=200 → Not female n=199; Female n=1

Female n=233 → Female karyotype n=233 → Not female n=1; Female n=232

FIG. 21E

FIG. 21F

**FIG. 22**

FIG. 23

FIG. 24

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2013103320 A
- US 61552374
- WO 2013062856 A1
- US 20130029852
- US 13555037 B
- US 20130029852 A1
- US 20080139801
- US 20090026082
- WO 2009046445 A
- US 20120010085 A
- US 20110224087 A
- US 20110201507 A

### Non-patent literature cited in the description

- **MAGI et al.** Read count approach for DNA copy number variants detection. *Bioinformatics,* 15 February 2012, vol. 28 (4), 470-8
- **SAMBROOK et al.** Molecular Cloning: A Laboratory Manual. Cold Spring Harbor, 2001
- **AUSUBEL et al.** *Current Protocols in Molecular Biology,* 1987
- **REDON et al.** *Nature,* 2006, vol. 23, 444-454
- **SHAIKH et al.** *Genome Res,* 2009, vol. 19, 1682-1690
- **WALSH et al.** *Science,* 2008, vol. 320, 539-543
- **VOLKERDING et al.** *Clin Chem,* 2009, vol. 55, 641-658
- **METZKER M.** *Nature Rev,* 2010, vol. 11, 31-46
- **M. VAN DER LAAN ; K. POLLARD.** A new algorithm for hybrid hierarchical clustering with visualization and the bootstrap. *Journal of Statistical Planning and Inference,* 2003, vol. 117, 275-303
- **TREANGEN TJ ; SALZBERG SL.** Repetitive DNA and next-generation sequencing: computational challenges and solutions. *Nat Rev Genet,* 29 November 2011, vol. 13 (1), 36-46
- **FAN et al.** *Proc Natl Acad Sci,* 2008, vol. 105, 16266-16271
- **KOIDE et al.** *Prenatal Diagnosis,* 2005, vol. 25, 604-607
- **CHEN.** *Nature Med,* 1996, vol. 2, 1033-1035
- **LO et al.** *Lancet,* 1997, vol. 350, 485-487
- **BOTEZATU et al.** *Clin Chem,* 2000, vol. 46, 1078-1084
- **SU et al.** *J Mol. Diagn.,* 2004, vol. 6, 101-107
- **ALNEMRI ; LIWACK.** *J Biol. Chem,* 1990, vol. 265, 17323-17333
- **RICHARDS ; BOYER.** *J Mol Biol,* 1965, vol. 11, 327-240
- *Ann Rev Biophys Biomol Struct,* 1995, vol. 24, 167-183
- *Mol Biotechnol,* 2004, vol. 26, 233-248
- **CHAN et al.** *Clin Chem,* 2004, vol. 50, 8892
- **LI et al.** *Clin Chem,* 2004, vol. 50, 1002-1011
- **FAN et al.** *Clin Chem,* 2010, vol. 56, 1279-1286
- **HARRIS T.D. et al.** *Science,* 2008, vol. 320, 106-109
- **MARGULIES, M. et al.** *Nature,* 2005, vol. 437, 376-380
- **SONI GV ; MELLER A.** *Clin Chem,* 2007, vol. 53, 1996-2001
- **BENTLEY et al.** *Nature,* 2009, vol. 6, 53-59
- **KOZAREWA et al.** *Nature Methods,* 2009, vol. 6, 291-295
- **LANGMEAD et al.** *Genome Biology,* 2009, vol. 10, R25.1-R25.10
- **CHAN et al.** *Clin Chem,* 2004, vol. 50, 88-92
- **ILLANES et al.** *Early Human Dev,* 2007, vol. 83, 563-566
- **LO et al.** *Am J Hum Genet,* 1999, vol. 64, 218-224
- **VOSRANOVA L et al.** *Molecular Cytogen,* 2008, vol. 1, 13
- **JOOSTEN et al.** *Prenatal Diagn,* 1997, vol. 17, 271-5
- **HARRIS et al.** *Prenatal Diagn,* 2011, vol. 31, 932-944
- **KIDD et al.** *Forensic Sci Int,* 2006, vol. 164 (1), 20-32
- **PALOMAKI et al.** *Genetics in Medicine,* 2011, 1
- **ERICH et al.** *Am J Obstet Gynecol,* March 2011, vol. 204 (3), 205
- **CHIU et al.** *BMJ,* 2011, vol. 342, c7401
- **BENN et al.** *Prenat Diagn,* 2012